# EUROPEAN PATENT APPLICATION

(11) **EP 4 721 736 A1**
(43) Date of publication of application: **08.04.2026**
(21) Application number: 24810453.1
(22) Date of filing: 23.05.2024
(51) Int. Cl.: A61K 31/385, A61P 27/10

(54) **PHARMACEUTICAL COMPOSITION FOR EFFECTIVELY DELAYING AND TREATING MYOPIA**

(30) Priority: 24.05.2023 CN 202310597594
(71) Applicant: The Eye Hospital of Wenzhou Medical University, Wenzhou, Zhejiang 325000 (CN)
(72) Inventor: ZHOU, Xiangtian, Wenzhou, Zhejiang 325027 (CN); ZHAO, Fei, Wenzhou, Zhejiang 325027 (CN); PAN, Miaozhen, Wenzhou, Zhejiang 325027 (CN); ZHENG, Qinyuan, Wenzhou, Zhejiang 325027 (CN); QU, Jia, Wenzhou, Zhejiang 325027 (CN)
(74) Representative: Huang, Liwei
(86) International application number: PCT/CN2024/094904
(87) International publication number: WO 2024/240216

(57) **Abstract**

The present application relates to a pharmaceutical composition for effectively delaying and treating myopia, and in particular, to a method for treating myopia by using lipoic acid and/or lipoic acid choline ester. The pharmaceutical composition may effectively prevent and control myopia and/or delay myopia progression, while maintaining safety and free of obvious side effects, which results in a good clinical application prospect.

## Description

### Technical Field

The present application relates to a pharmaceutical composition for effectively delaying and treating myopia, and in particular, to a method for treating myopia with lipoic acid and/or lipoic acid choline ester, and a method for controlling myopia progression and a use thereof, and provides corresponding drugs or formulations. The present application relates to the field of medicine.

### Background Art

Myopia, a most common type of refractive errors, refers to a refractive state where in relaxed accommodation, incoming parallel light rays are focused in front of the retina after being refracted by the refractive system of the eyes. Myopia has highly complex pathogenesis that involves several factors, including genetics, environment, age, intensity of eye use, and even dietary habits, etc. Among the massive volume of literature on the pathogenesis myopia, many experimental conclusions on the treatment or induction of myopia are questioned by peer experts, and certain cases even have conflicting results under similar experimental conditions, particularly in studies on interventions of myopia molecular targets with compounds. For example, it has been found in a negative lens-induced animal model that a cAMP analog 8-Bromo-cAMP may effectively treat myopia (Rachel Ka Man Chun et al., Cyclic Adenosine Monophosphate Activates Retinal Apolipoprotein A1 Expression and Inhibits Myopic Eye Growth, Invest Ophthalmol Vis Sci., 2015 Dec; 56(13):8151-7.). Conversely, another study using a cAMP analog reported that the cAMP analog not only showed no therapeutic effect on myopia but also may induce myopia (Fuxin Zhao et al., Declines in PDE4B activity promote myopia progression through downregulation of scleral collagen expression, Exp Eye Res., 2021 Nov; 212:108758.). In actual experiments, typical antioxidants such as vitamin C (ascorbic acid) failed to achieve therapeutic effects in wild-type mouse myopia models. In contrast, vitamin C was found to inhibit form-deprivation-induced myopia in mice with pigmentary degeneration of retina caused by a gene mutation (rd10) (Erica Landis et al., Ascorbic acid, and not L-DOPA, protects against form-deprivation myopia in retinal degeneration mouse models., IOVS, ARVO Annual Meeting Abstract, September 2016, Volume 57, Issue 12). As another example, for atropine sulfate that is already approved for marketing in China, even with identical treated groups and active ingredients, while both low and high concentrations of atropine administrated clinically inhibited onset and development of myopia, higher concentration of atropine exhibits a "rebound effect upon discontinuation". The data from animal disease models and clinical studies continually remind researchers that it is exceedingly difficult to predict the final efficacy of myopia treatment by experiments based solely on theoretical hypotheses or speculation. This is primarily because the mechanism of myopia remains incompletely understood, which results in difficult prevention and control.

In addition to insufficient basic research, it has been observed that the cellular biological mechanism involved in myopia differs from that in other tissues or organs. For example, scleral collagen content, a key indicator in myopia treatment research, is upregulated during fibroblast-to-myofibroblast transdifferentiation in other tissues of the body (Shan Jiang et al., Inhibition of TRPC6 suppressed TGFβ-induced fibroblast-myofibroblast transdifferentiation in renal interstitial NRK-49F cells., Exp Cell Res., 2022 Dec 1; 421(1):113374. and Kazuya Kusama et al., PGE2 and Thrombin Induce Myofibroblast Transdifferentiation via Activin A and CTGF in Endometrial Stromal Cells., Endocrinology. 2021 Dec 1; 162(12):bqab207.), whereas during myopia development, the occurrence of this transdifferentiation is accompanied by a decrease of collagen (Hao Wu et al., Scleral hypoxia is a target for myopia control., Proc Natl Acad Sci U S A., 2018 Jul 24; 115(30):E7091-E7100.). Consequently, in attempts to treat myopia, it is difficult to draw upon research findings on other organs or diseases due to significant differences in structure and function among different organs, as well as substantial disparities in cellular signaling pathways across different diseases. In addition, due to the complex structures of mammalian eyes, for example, the presence of biological barriers such as corneae and sclerae, it presents considerable challenges in development of drugs for treating myopia and utilization of drugs in prevention and control of myopia.

Furthermore, there are numerous types of myopia. Complex types of myopia or myopia occurring in different age groups, such as pathological myopia, myopia with an underlying diabetic condition or directly induced by hyperglycemia, myopia in the elderly, high myopia, and myopia caused by lenticular or corneal pathologies, often exhibit distinct pathological changes or are accompanied by other underlying diseases that influence treatment. Consequently, the clinical treatment methods for complex myopia differ from those for ordinary myopia. Currently, no drugs have significant efficacy and high safety for treating myopia, particularly for controlling the progression of myopia in children and adolescents. Thus, there are clinical needs that are not met in this field.

Lipoic acid, commonly referred to as alpha-lipoic acid, is an octanoic acid with a disulfide bond (as shown in Formula I). It exhibits both liposolubility and water solubility and can be reduced to dihydrolipoic acid in vivo. Lipoic acid has as two enantiomers: R-(dextro)-lipoic acid and S-(levo)-lipoic acid. Intermediates for lipoic acid crude drug generally include particulate lipoic acid, R-alpha lipoic acid, lipoic acid tromethamine salt, and ethyl 6,8-dichlorooctanoate, etc, among which, the R-alpha lipoic acid tromethamine salt has strong stability and high bioavailability. Lipoic acid choline ester (LACE) is a chemically synthesized derivative of alpha-lipoic acid and can be further formed into a salt in ocular formulations, as described in patent CN108135842A. It has disclosed in the prior art that several forms of lipoic acid, or optical isomers thereof or racemates, or solvates thereof, or pharmaceutically acceptable salts or esters thereof, prodrugs thereof, metabolites thereof, related compounds or extracts thereof, or crystalline compounds thereof can either enhance the solubility of lipoic acid or improve the stability of lipoic acid, and are all considered to have the functions of lipoic acid. Therefore, all such substances are deemed potentially applicable for the treatment of myopia in the present application.

In the field of medicine, lipoic acid is primarily used for treatment of multiple diseases, such as diabetic peripheral neuropathy and neurological complications. In the field of ophthalmic presbyopia treatment, lipoic acid can restore elasticity of crystalline lens in aging individuals; however, LACE fails to meet the primary endpoint in interim analysis of Novartis Phase IIb dose-ranging study. In addition, with the antioxidant capacity of lipoic acid, oral administration of lipoic acid has historically been investigated in clinical trials for treatment of age-related macular degeneration, although the efficacy thereof also did not reach a clinical endpoint (Benjamin J Kim et al., Orally Administered Alpha Lipoic Acid as a Treatment for Geographic Atrophy: A Randomized Clinical Trial., Clinical Trial Ophthalmol Retina., 2020 Sep; 4(9):889-898.). In the field of food and health supplements, lipoic acid has been publicly reported as a promising anti-obesity drug.

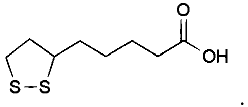

### Summary of the Invention

A novel use of lipoic acid has been surprisingly discovered in the present application, and it has been demonstrated through repeated experiments that lipoic acid and lipoic acid choline ester is capable of treating myopia and controlling onset and progression of myopia. That is, lipoic acid is not used as a supplementary or auxiliary ingredient in a myopia treatment drug to assist, accompany, or aid another active ingredient for treating myopia to enhance (for example, by directing more main ingredients in the drug to target cells) effect of the active ingredient for treating myopia. Instead, lipoic acid is as a direct or main active ingredient for treating myopia.

The present application is the first to demonstrate that lipoic acid alone is capable of significantly inhibit myopia progression, whereby it is believed that lipoic acid may effectively treat various types of myopia, including simple myopia or axial myopia (such as myopia in children and adolescents), and mild or moderate myopia. Compared to the use of treating myopia in the elderly, or high myopia, or high myopia in the elderly, or pathological myopia, lipoic acid exhibits significantly superior effect in treating myopia with continuously decreasing refraction (for example, simple myopia, axial myopia, axial simple myopia, low and moderate myopia, myopia in children and adolescents, or progressive myopia).

Lipoic acid and lipoic acid choline ester may also be used for prevention of various types of myopia. For example, pharmacological intervention with lipoic acid or lipoic acid choline ester during the progression phase of myopia may prevent and inhibit the onset of myopia in the elderly, high myopia, high myopia in the elderly, and pathological myopia.

The present application further investigates concentration, proportion, and formula of lipoic acid or lipoic acid choline ester in a drug or a formulation for treating myopia as well as dosing frequency and an administration method such as combined therapy of the drug, where topical (ocular) administration not only demonstrates favorable safety, but also exhibits markedly superior therapeutic effect compared to systemic administration.

The present application provides a use of lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing, where the use is one or two or more of:
(1) preventing and/or treating myopia and a myopia-related symptom, and/or correcting myopia; or
(2) controlling onset and development of myopia with orthokeratology lenses or another myopia treatment drug; or
(3) performing intervention treatment on myopic eyes or delaying myopia progression; or
(4) inhibiting or retarding axial elongation and/or increase in length (depth) of vitreous chamber in an individual with myopia or an individual with a tendency to develop myopia; or
(5) preventing, retarding, reducing or treating abnormal eyeball development related to vision disorder; or
(6) enabling an individual to obtain clearer distance visual acuity without wearing lenses or relying on visual acuity correction means than distance visual acuity before using the lenses or the means and/or than distance visual acuity when the lenses or the means; or
(7) inhibiting, delaying or retarding progress or rate of negative refraction in individuals with myopia or individuals with a tendency to develop myopia; or
(8) preparing a drug, a formulation, a composition, or a device for achieving at least one of the uses of (1) to (7).

In some embodiments, an R-type optical isomer accounts for 50% and above (for example, 99%) or lower than 50% of all of the lipoic acid, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing.

In some embodiments, an R-type optical isomer accounts for 50% and above (for example, 99%) or lower than 50% of all of the lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing.

In some embodiments, the lipoic acid is a particulate lipoic acid.

In some embodiments, a specific content ratio or molar ratio of two optical isomers influence biological activity of the lipoic acid, or the optical isomer or racemate thereof, or the solvate thereof, or the pharmaceutically acceptable salt or ester thereof (for example, lipoic acid choline ester), or the prodrug thereof, or the metabolite thereof, or the related compound or extract thereof, or the crystalline compound thereof, or the combination of the foregoing, for example, the efficacy for myopia treatment.

In some embodiments, the following administration is adopted: systemic administration such as oral administration, and/or topical administration (eye drop instillation, intraocular injection, intraocular implantation, periocular application of a skin ointment/emulsion, or application of an eye ointment), and/or parenteral administration (for example, mucosal administration, transdermal administration, microneedle administration), and/or non-invasive administration (for example, administration using an ophthalmic spray). Preferably, the systemic administration refers to an improved systemic administration.

In some embodiments, free lipoic acid may be detected in the blood of individuals with myopia or individuals with a tendency to develop myopia after oral administration.

In some embodiments, the drug, the formulation, or the composition may be an injection, a tablet a lyophilized powder for injection, a capsule, an effervescent tablet, a chewable tablet, a buccal tablet, granules, an ointment, a syrup, an oral liquid, an aerosol, a nasal drop, an externally applied formulation, an oral formulation, or the like; preferably, an ophthalmic dosage form, including but not limited to eye drops (eye water), an eye ointment, an ophthalmic spray, an implant tablet, an ophthalmic gel, an eye patch, an ophthalmic microsphere, an ophthalmic sustained-release formulation, a periocular injection, and an intraocular injection; or alternatively, a regular solution, an aqueous solution, an unsaturated solution, an oil-water mixed liquor, a suspension (agent), a liniment, a lotion, a cream, drops, a powder preparation for infusion, a spray, a medicinal extract, a patch, a paste, a pill, a suppository, an emulsion, or a group containing cellulose (for example, methylcellulose), a polyol, cyclodextrin (for example, hydroxypropyl-beta-cyclodextrin), an alkaline solubilizer, a dendrimer, a nanomaterial, a sustained-release material, a liposome, or any combination thereof.

In some embodiments, the drug, the formulation, or the composition contains distearoylphosphatidylcholine, soybean lecithin, octadecylamine, Poloxamer 188, sodium chloride, mannitol, mercaptoethanol, tromethamine, sodium sulfite, ethylenediamine, benzyl alcohol, arginine, or any combination thereof. It may be apparent that the drug, the formulation, or the composition herein may also not contain any of the substances described in this paragraph.

In some embodiments, the individuals with myopia or the individuals with a tendency to develop myopia include children and/or adolescents, preferably aged 2 to 30, and more preferably aged 6 to 18; or minors, preferably individuals whose eyes (eyeballs) are still in growth or development; or school-age individuals, preferably students in grades 1 to 12; or individuals with parents having high myopia; or individuals with insufficient hyperopia reserve.

In some embodiments, the myopia is: refractive myopia or axial myopia; congenital myopia (myopia present at birth or before school age), early-onset myopia (onset before 14 years old), delayed-onset myopia (onset at 16-18 years old), late-onset myopia (onset after adulthood); low myopia (mild myopia), moderate myopia, high myopia (severe myopia); pseudomyopia, true myopia; myopia in children and/or adolescents (preferably aged 2-30, and more preferably aged 6-18), myopia in minors, myopia in adults, myopia in the elderly; simple myopia, pathological myopia; axial simple myopia, simple axial myopia; axial myopia in children and/or adolescents (preferably aged 2-30, and more preferably aged 6-18); axial myopia in school-age and preschool individuals; primary myopia, secondary myopia; primary myopia in children and/or adolescents (preferably aged 2-30, and more preferably aged 6-18); or progressive myopia in children and/or adolescents (preferably aged 2-30, and more preferably aged 6-18); curvature myopia, index myopia, astigmatic myopia, positional myopia, curved myopia; axial myopia with continuously increased negative refraction; myopia caused by prolonged close-up work, myopia and pseudomyopia induced by eye strain, negative refraction caused by an adverse drug reaction, myopic eyes, myopia caused by reading, myopia caused by using an electronic products such as a mobile phone, myopia caused by mismatched refractive media (components), refractive myopia, myopia caused by abnormal refractive development, myopia caused by excessive eyeball growth, myopia caused by unhygienic eye use, myopia characterized by imaging focuses of distant objects falling in front of the retina due to various reasons, myopia demonstrating poor or no response to atropine treatment, non-complex myopia (such as progressive complex myopia not accompanied by disordered lipid peroxide (POL) oxidation, myopia in non-elderly individuals, non-high myopia, or keratoconus-associated myopia unrelated to Nrf2/HO-1 signaling pathway), myopia caused by insufficient outdoor activities, accommodative spastic myopia, myopia in children, myopia in infants, hereditary myopia, and myopia dominated by environmental factors.

In some embodiments, myopia primarily refers to myopia highly prevalent in healthy young individuals or younger individuals (where patients are free from underlying diseases, such as hypertension, hyperlipidemia, and hyperglycemia), rather than high myopia or myopia in the elderly.

In some embodiments, myopia includes or does not include high myopia or myopic complications induced by high myopia, myopia or myopic complications thereof induced by diabetes (hyperglycemia), myopia or myopic complications thereof induced by lenticular pathologies, and/or myopia or myopic complications thereof associated with keratoconus.

In some embodiments, the myopia-related symptom includes a complication induced by myopia (especially high myopia), such as spotted vision, glaucoma, posterior staphyloma, retinopathy, retinal detachment, retinal tear, amblyopia, macular hemorrhage, choroidal neovascularization, choroidal atrophy, macular degeneration or maculopathy, visual field defect, progressive or sudden diminution of visual acuity (especially near visual acuity), sore and swelling eyes and/or eye pain, nyctalopia, astigmatism, loss of sight, vitreous liquefaction, vitreous opacity, strabismus, frequent blinking, frequent eye rubbing, anisometropia, blurred vision for distant objects, necessitating squinting or partially closing the eyelids to see distant objects clearly, headache caused by eye strain, inattention caused by myopia, difficulty in vision while driving (especially at night, i.e., night myopia), retinal atrophy and degeneration (hemorrhage and hiatus), subretinal neovascularization, or eyeball atrophy.

In some embodiments, the myopia-related symptom includes or does not include myopia or a myopia-related symptom induced by hyperglycemia, such as choroidal neovascularization, retinopathy, or macular degeneration in patients with hyperglycemia.

In some embodiments, the drug, the formulation, the composition, or the device further includes another drug, another compound, or another ophthalmic formulation, which includes but is not limited to myopia treatment drugs (such as pirenzepine, muscarinic antagonist, robaxin, indoramine, timolol maleate, adrenaline, pyrazine, perlapine, methylamine, chlorisondamine, acetylcholinesterase inhibitor, dopamine agonist, γ-aminobutyric acid, naloxone, glucagon, homatropine methylbromide, retinoic acid, salidroside, and formononetin), prazosin, vinpocetine, bendazac lysine, benzydamine, bendazac, M-receptor blockers (for example, blockers, antagonists, or inhibitors targeting M2 or M3 receptors), atropine or atropine sulfate, dibazol, polyunsaturated fatty acids (such as DHA and EPA), homatropine, anisodamine (racemic), tropicamide, 7-methylxanthine, nicotinic acid, piracetam, Salvia miltiorrhiza extract, Carthamus tinctorius extract, fish oil, bear bile extract, vitamins, adenosine triphosphate (ATP), smooth muscle relaxants, drugs for preventing vasospasm, non-selective adenylate antagonists, vasodilators, mydriatic components, decongestant components, accommodative components for eye muscle (for example, ciliary muscle), anti-inflammatory (antiphlogistic) components, astringent components, antihistaminic components, anti-allergic components, components for inhibiting collagen degradation, liver-protecting components (to avoid or reduce hepatotoxicity), components for enhancing blood-retinal barrier (to make it more difficult for compounds to permeate through the physiological barrier), amino acids, antibacterial components, antioxidant components, sugars, polymers or derivatives thereof, cellulose or derivatives thereof, local anesthetic components, amblyopia treatment components, glaucoma treatment components, cataract treatment components, phosphodiesterase inhibitors (specific or non-specific), miRNA and modifications thereof, therapeutic components for ophthalmic diseases, ophthalmically compatible excipients, liver extract, propolis, blueberry (bilberry) fruit extract, anthocyanin, nano-selenium, vitamin E, vitamin C, vitamin B, lutein, astaxanthin, mexidol, pyridoxol (for example, pyridoxol hydrochloride), lipoic acid, and the like. It may be apparent that the drug, the formulation, the composition, or the device herein may also not contain any of the substances described in this paragraph.

In some embodiments, the lipoic acid or the lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing, and one or more drugs for delaying myopia progression and/or treating myopia are formulated or designed for continuous administration, or simultaneous administration, or sequential administration, or alternating administration, or administration at intervals, or separate administration.

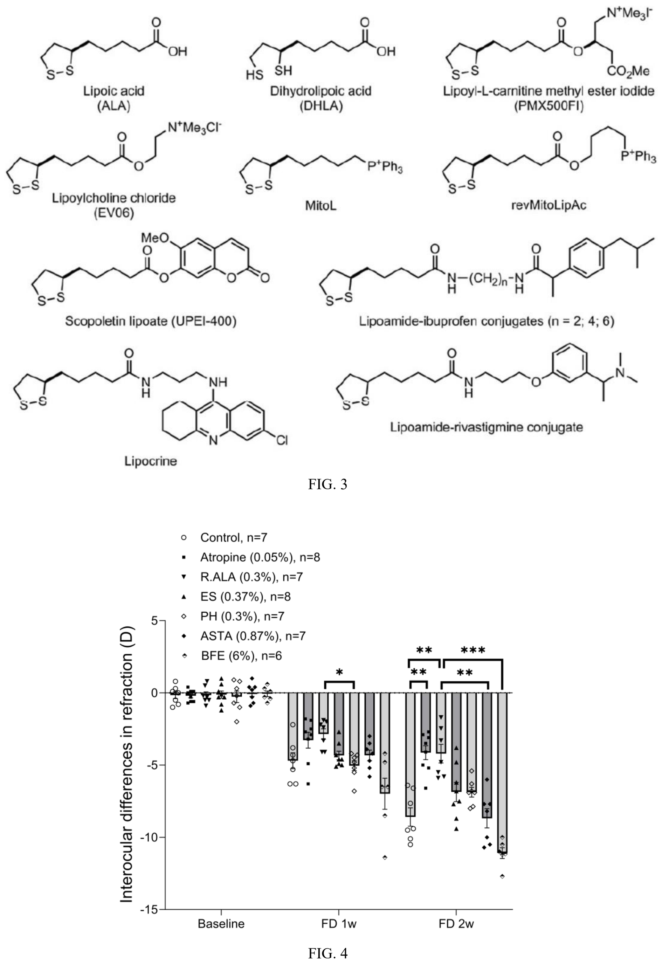

In some embodiments, the compound related to lipoic acid includes but is not limited to: salts further formed from lipoic acid ester, for example, all salt forms of lipoic acid choline ester listed in CN115279745A, including but not limited to lipoic acid choline ester tosylate, lipoic acid choline ester besylate, lipoic acid choline ester chloride, or lipoic acid choline ester iodide; or conjugates of such substances are compounds in FIG. 3 (Walter H. Moos et al., Epigenetic Treatment of Neurodegenerative Ophthalmic Disorders: An Eye Toward the Future., Biores Open Access., 2017 Dec 1; 6(1):169-181.).

In some embodiments, the lipoic acid and the related compound thereof include but are not limited to R-(dextro)-lipoic acid; S-(levo)-lipoic acid (preferably, R-alpha lipoic acid), beta-lipoic acid, dihydrolipoic acid, halogenated or deuterated derivatives of lipoic acid and dihydrolipoic acid, structural analogs of lipoic acid such as CMX-2043, ester forms, derivatives, or structural analogs of lipoic acid (such as PMX500FI), various salts (for example, sodium salt, lysine salt) or esters (especially lipoic acid choline ester, for example, EV06, or alpha-LACE, or UNR844) or amides (for example, Lipoamide) of lipoic acid, lipoic acid choline ester tosylate, lipoic acid choline ester 3,4-dihydroxybenzoate, lipoic acid choline ester besylate, lipoic acid choline ester iodide or chloride, derivatives of lipoic acid choline ester, other nanomaterials, biomacromolecules, or chemical drugs modified with lipoic acid, prodrugs of lipoic acid (such as lipoic acid choline ester or lipoic acid choline ester chloride), metabolites of lipoic acid (such as 6,8-bis(methylthio)octanoic acid (BMOA), 4,6-bis(methylthio)hexanoic acid (BMHA), 2,4-bis(methylthio)butanoic acid (BMBA), bisnorlipoic acid (BNLA), tetranorlipoic acid (TNLA), and beta-hydroxybisnorlipoic acid), and the like.

In some embodiments, the derivative of lipoic acid choline ester may be understood as any compound or a mixture of compounds, other than lipoic acid and choline, formed by reaction of lipoic acid choline ester with a non-aqueous pharmaceutical excipient. In certain embodiments, the derivative is a product formed by reaction of lipoic acid choline ester with propylene glycol. In certain embodiments, the derivative is a product formed by reaction of lipoic acid choline ester with glycerol.

In some embodiments, the metabolite of lipoic acid or the pharmaceutically acceptable salt or ester thereof is 6,8-bis(methylthio)octanoic acid (BMOA), 4,6-bis(methylthio)hexanoic acid (BMHA), 2,4-bis(methylthio)butanoic acid (BMBA), bisnorlipoic acid (BNLA), tetranorlipoic acid (TNLA), beta-hydroxybisnorlipoic acid, tetranorlipoic acid, and dimethylbisnorlipoic acid.

In one embodiment, the pharmaceutically acceptable salt or ester of lipoic acid herein in a compound described in CN115279745A or CN113387923A, especially the compounds described in claims 1 to 21 of CN113387923A. These compounds are considered as retaining the basic functions of lipoic acid. For example, the present application provides a lipoic acid choline ester tosylate with the following structure:

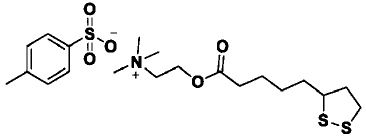

In some embodiments, the present application provides an (R)-lipoic acid choline ester tosylate with the following structure:

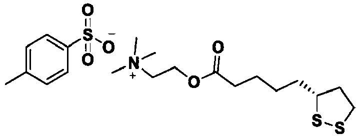

It has an R-isomer with an enantiomeric excess of at least about 90%, at least about 95%, at least about 96%, at least about 97%, at least about 98%, or at least about 99%.

In one embodiment, the present application provides a lipoic acid choline ester besylate with the following structure:

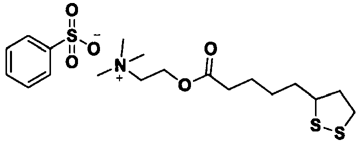

In some embodiments, the present application provides an (R)-lipoic acid choline ester besylate with the following structure:

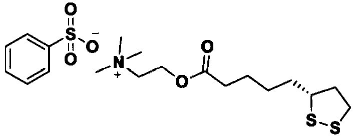

It has an R-isomer with an enantiomeric excess of at least about 90%, at least about 95%, at least about 96%, at least about 97%, at least about 98%, or at least about 99%.

In one embodiment, the present application provides a lipoic acid choline ester 3,4-dihydroxybenzoate with the following structure:

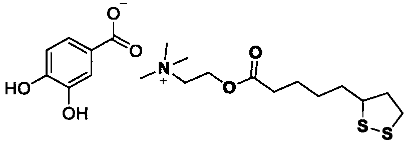

In one embodiment, the present application provides an (R)-lipoic acid choline ester 3,4-dihydroxybenzoate with the following structure:

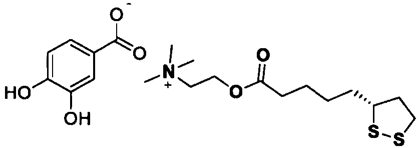

It has an R-isomer with an enantiomeric excess of at least about 90%, at least about 95%, at least about 96%, at least about 97%, at least about 98%, or at least about 99%.

In one embodiment, the related compound of lipoic acid herein is a compound described in CN111315369A, especially the compounds described in claims 1 to 3 thereof. These compounds are considered as retaining the basic functions of lipoic acid. For example, such compounds include a compound of Formula I, or a pharmaceutically acceptable hydrate, solvate, crystal, cocrystal, enantiomer, stereoisomer, polymorph, or prodrug thereof: 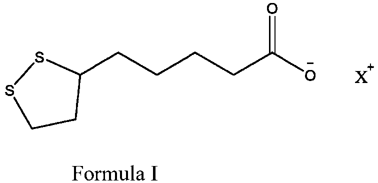 where X⁺ represents: 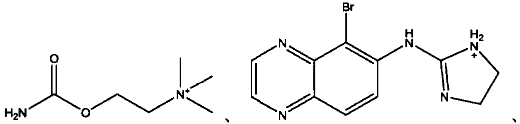 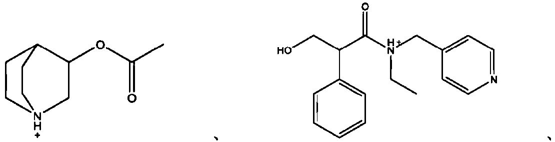 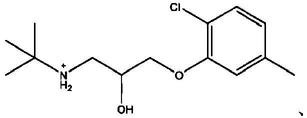 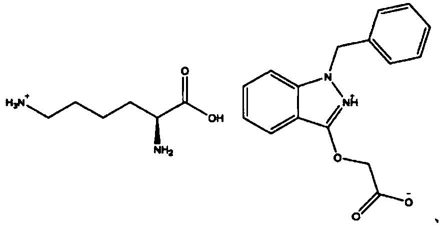 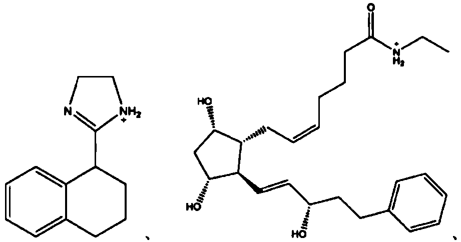 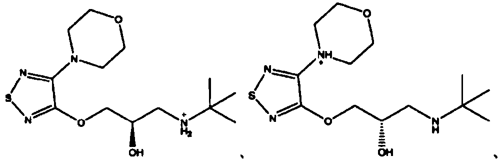 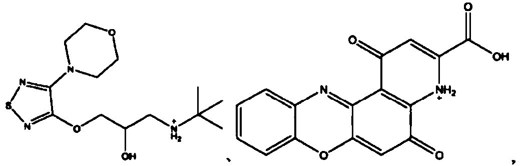 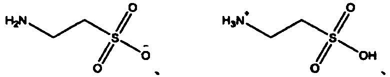 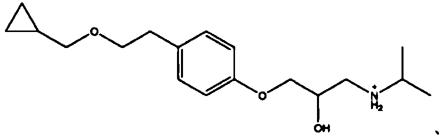 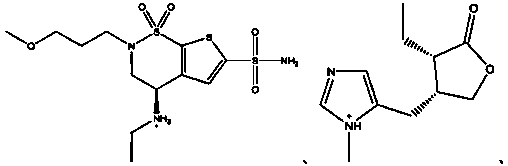 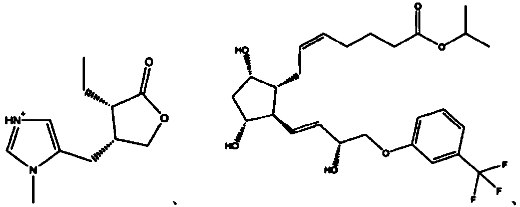 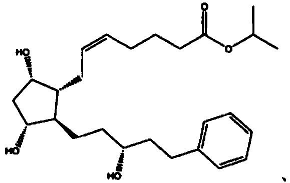 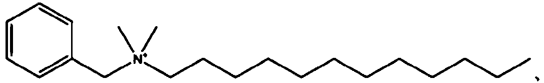 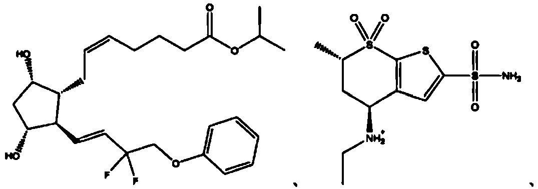 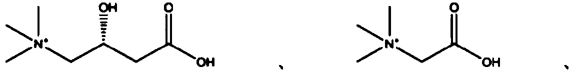 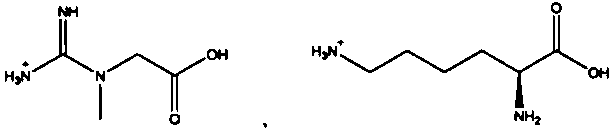 OR

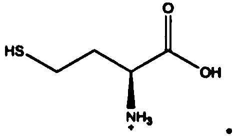

In some embodiments, such compounds include a compound of Formula Ia, or a pharmaceutically acceptable hydrate, solvate, crystal, cocrystal, enantiomer, stereoisomer, polymorph, or prodrug thereof: 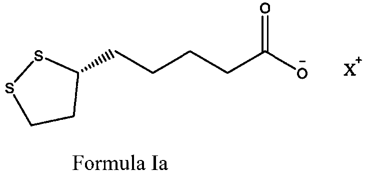 where X⁺ is as defined above.

In some embodiments, such compounds include a compound of Formula Ib, or a pharmaceutically acceptable hydrate, solvate, crystal, cocrystal, enantiomer, stereoisomer, polymorph, or prodrug thereof: 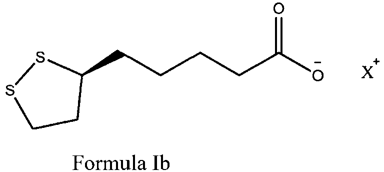 where X⁺ is as defined above.

In some embodiments, in a drug, a formulation, or a composition containing lipoic acid choline ester, the lipoic acid choline ester has a counter ion, including but not limited to chloride ion, bromide ion, iodide ion, sulfate radical, methanesulfonate radical, nitrate radical, maleate radical, acetate radical, citrate radical, fumarate radical, hydrogen fumarate ion, tartrate ion (optical isomers or a mixture thereof), succinate radical, benzoate radical, and glutamate anion. For example, a pharmaceutical composition of LACE chloride or LACE iodide is described in WO 2018/055572.

In some embodiments, the lipoic acid choline ester is the prodrug of lipoic acid, and is hydrolyzed into lipoic acid after being applied to eyes. In certain embodiments, the lipoic acid choline ester may alternatively be considered as a derivative of lipoic acid.

In some embodiments, dihydrolipoic acid is a metabolite of lipoic acid. On eyes, lipoic acid may be further reduced to produce the dihydrolipoic acid.

Lipoic acid and related compounds thereof may be alternatively produced by organisms. Therefore, ingredients or media administrated in myopia treatment may also include microorganisms or mammalian cells capable of producing or secreting lipoic acid. Preferably, the microorganisms or mammalian cells are gene-edited or genetically engineered.

In fact, all of the foregoing related compounds of lipoic acid exist in the prior art, and have been proven to exhibit the same basic effects as lipoic acid with differences in terms of solubility, corneal penetration ability, bioavailability, stability, and the like. A person skilled in the art is also able to prepare more other related compounds of lipoic acid having the functions of lipoic acid.

In some embodiments, the formulation is an oral product such as a health product, food, a dietary supplement, a nutraceutical, or a beverage, or a cosmetic. The cosmetic may be one of regular solutions, aqueous solutions, unsaturated solutions, oil-water mixtures, suspensions (agents), liniments, lotions, sprays, creams, drops, granules, ointments, pastes, pills, suppositories, emulsions, and patch, or a combination thereof.

In some embodiments, the device may be an instrument, an apparatus, a consumable, a system, medical equipment, a health product or a product for changing eye appearance that is capable of releasing drugs or has a drug delivery function or has a potential drug delivery capability, such as corneal contact lenses, eyeglasses, intraocular lenses, sutures, orthokeratology (OK) lens cleaning (maintenance) systems, eye patches, visual acuity-improving patches, cosmetic contact lenses, microneedles, ophthalmic spray systems, eye massagers, eye fumigators, ocular surface drug delivery devices, intraocular drug delivery devices, fundus drug delivery devices, implantable pumps, wearable devices, acupoint massagers, eye relaxation devices, myopia treatment instruments, or drug-device combinations for myopia prevention and control.

In some embodiments, the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing serves as a sole active ingredient or a main active ingredient or a direct active ingredient; or a content or efficacy of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing accounts for 1% and below (for example, a content of at least 0.15%), or more than 1%, more than 10%, more than 20%, more than 30%, more than 40%, more than 50%, more than 60%, more than 70%, more than 80%, more than 90%, or 100% of that of all active ingredients for the use; and/or the content or efficacy of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing accounts for more than 20% (exclusive of 20%) of that of all starting material ingredients for the use, where the percentage (%) may be a mass ratio, or a molar ratio, or a mass-volume ratio, or an efficacy contribution rate to the use.

In some embodiments, a concentration of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing in the drug, the formulation, the composition, or the device is 0.001 µM to 10 mM, preferably 0.01 µM to 1000 µM, preferably 0.05 µM to 100 µM, more preferably 0.1 µM to 25 µM, and more preferably 0.1 µM to 15 µM; 0.001 µM to 100 M, preferably 0.005 µM to 50 M, preferably 1 µM to 1 M, preferably 300 µM to 300 mM, more preferably 1 mM to 200 mM, and more preferably 10 mM to 100 mM (for example, about 46 mM, i.e., 0.94%); and/or, the concentration or proportion of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing in the drug, the formulation, the composition, or device is lower than 25%, preferably lower than 5%, preferably lower than 1%, more preferably lower than 0.01%, and more preferably lower than 0.001%; or higher than 0.0005%, preferably higher than 0.025%, preferably higher than 0.05%, more preferably higher than 0.1%, and more preferably higher than 1%; or not lower than 0.0001%, preferably not lower than 0.001%, preferably not lower than 0.01%, more preferably not lower than 0.1%, and more preferably not less than 0.8%, where the percentage (%) is a mass/volume concentration (g/100 ml) or a mass percentage or a molar (mole number) ratio.

The present application provides a drug, a formulation, a composition, or a device applied to eyes.

The present application further provides a pharmaceutical composition or a compound formulation containing at least two active ingredients, comprising lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing, and other active ingredients for treating myopia. Preferably, an addition amount, concentration, or efficacy of the lipoic acid or lipoic acid choline ester, or the optical isomer or racemate thereof, or solvate thereof, or the pharmaceutically acceptable salt or ester thereof, or the prodrug thereof, or the metabolite thereof, or the related compound or extract thereof, or the crystalline compound thereof, or the combination of the foregoing is not lower than that of any of the other active ingredients for treating myopia, where the efficacy is directed to treating myopia and/or controlling myopia progression.

The present application further provides a pharmaceutical composition or a compound formulation, comprising lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing, and other active ingredients for treating myopia. The other active ingredients for treating myopia include or do not include one or more active ingredients such as propolis, blueberry fruit extract, anthocyanin, beta-carotene, selenium or nano-selenium, vitamin E, vitamin C, lutein, taurine, astaxanthin, DHEA, grape seed extract, mexidol, and pyridoxol (for example, pyridoxol hydrochloride). In some embodiments, the proportion or concentration of lipoic acid or lipoic acid choline ester in the pharmaceutical composition or the compound formulation is at least 0.5%, 0.15%, 0.05%, 0.005%, or 0.00001%.

The present application further provides an ophthalmic formulation or a drug. Lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing serves as a direct, sole, or main active ingredient in the formulation or the drug.

In some embodiments, the formulation or the drug includes but is not limited to eye drops, eye ointments, ophthalmic sprays, implant tablets, ophthalmic gels, eye patches, ophthalmic microspheres, ophthalmic sustained-release formulations, periocular injections, or intraocular injections.

In some embodiments, the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or a combination of two or more in the combination of the foregoing may be administrated in the same period, for example, particularly administrated simultaneously or successively in one administration (treatment) process, administrated on the same day, administrated in the same week, administrated in the same month, or administrated in the same year; or alternately administrated at an interval, for example, alternately administrated at an interval of 4 hours, alternately administrated at an interval of 12 hours, alternately administrated every other day, alternately administrated every other week, alternately administrated every other month, or alternately administrated every other year; or
the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing and one or more other drugs are administrated in the same period, for example, particularly administrated simultaneously or successively in one administration (treatment) process, administrated on the same day, administrated in the same week, administrated in the same month, or administrated in the same year; or alternately administrated at an interval, for example, alternately administrated at an interval of 4 hours, alternately administrated at an interval of 12 hours, alternately administrated every other day, alternately administrated every other week, alternately administrated every other month, or alternately administrated every other year.

The lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing are used in combination with a device (such as corneal contact lenses) and/or a surgery (such as a refractive correction surgery, a laser corneal surgery for myopia, and a lens surgery).

In some embodiments, the concentration or administration frequency of the ophthalmic dosage form using lipoic acid as the main active ingredient or the sole active ingredient is higher than that of the ophthalmic dosage form using lipoic acid choline ester as the main active ingredient or the sole active ingredient.

In some embodiments, the one or more other drugs are drugs for myopia prevention and control and/or myopia treatment, such as atropine sulfate, dibazole, polyunsaturated fatty acids, DHA, fish oil, M-receptor blockers, nicotinic acid, pirenzepine, muscarinic antagonists, 7-methylxanthine (7MX), robaxin, indoramine, timolol maleate, adrenaline, pyrazine, perlapine, pirenzepine, methylamine, chlorisondamine, acetylcholinesterase inhibitors, dopamine agonists, γ-aminobutyric acid, naloxone, glucagon, retinoic acid, salidroside, and formononetin, vasodilators, smooth muscle relaxants, drugs for preventing vasospasm, drugs for regulating collagen metabolism, piracetam, anti-allergic drugs, liver-protecting drugs, drugs for reducing gastrointestinal irritation, as well as propolis, blueberry fruit extract, anthocyanin, beta-carotene, selenium or nano-selenium, vitamin E, vitamin C, lutein, taurine, astaxanthin, DHEA, grape seed extract, mexidol, and pyridoxol (for example, pyridoxol hydrochloride), and the like. It may be apparent that the drug, the formulation, the composition, or the device herein may also not contain any one or more of the substances described in this paragraph.

In some embodiments, the device refers to an instrument, an apparatus, a consumable, a medical device, or a health care product that has visual acuity protection functions or myopia treatment (correction) effects, such as various glasses for delaying myopia progression, OK lenses, framed glasses, eye patches, (myopia) acupoint massagers, eye relaxation devices, and myopia treatment instruments.

In some embodiments, in the process of achieving the foregoing uses, the dosage form for systemic administration (for example, oral tablets) and the dosage form for topical administration (for example, eye drops) may be used at the same time, or in combination, or alternately, or at an interval, or alone.

In some embodiments, abnormal eye development is mainly caused by genetic factors, including DNA-encoded information and/or epigenetic characteristics.

In some embodiments, abnormal eye development refers to a refractive development abnormality mainly induced by environmental factors or mainly caused by human factors (such as prolonged close-up reading, frequent use of electronic screens, persistent close-up work with a lack of distance viewing opportunities, improper use of refractive correction glasses, drug side effects, obesity, trauma, poor lighting in learning environments, and lack of outdoor activities), which, however, is not related to genetic factors, or genetic factors play a secondary, accompanying, or synergistic role therein. The abnormal development includes, for example, abnormal development of eyeball size in children and adolescents (for example, aged 2-30).

In some embodiments, the abnormal development or development abnormality includes excessive axial length or mismatch between the axial elongation rate and the refractive system, resulting in the imaging focus of parallel light rays falling in front of the retina after passing through the normal or abnormal ocular refractive system.

### Brief Description of the Drawings

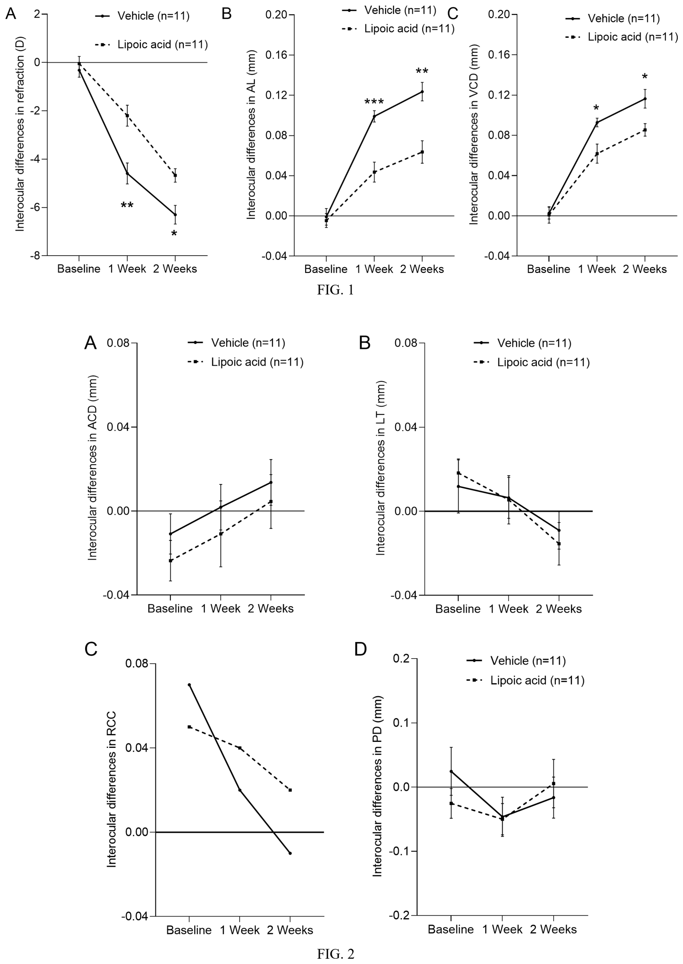

FIG. 1 shows myopia progression under the control of lipoic acid. Interocular differences in refraction, vitreous chamber depth, and axial length in subjects were compared between a lipoic acid treatment group and a negative control group before the experiment, in week 1 of the experiment, and in week 2 of the experiment. A shows differences in refraction between an experimental eye and the contralateral eye; B shows differences in axial length between the experimental eye and the contralateral eye; C shows differences in vitreous chamber depth between the experimental eye and the contralateral eye, where * represents p<0.05, ** represents p<0.01, and *** represents p<0.001; VCD represents the vitreous chamber depth, AL represents the axial length, and n represents the number of samples.
FIG. 2 shows influence of lipoic acid on anterior chamber depth (A), lens thickness (B), radius of corneal curvature (C), and pupil diameter (D), where ACD represents the anterior chamber depth, LT represents the lens thickness, RCC represents the radius of corneal curvature, PD represents the pupil diameter, and n represents the number of samples.
FIG. 3 shows examples of lipoic acid and related compounds thereof.
FIG. 4 shows intervention effects of lipoic acid, mexidol, pyridoxol, astaxanthin, and blueberry fruit extract on refraction in individuals with myopia. Interocular differences changes in refraction in the same subject were compared between different administration groups before the experiment, 1 week after administration, and 2 weeks after administration, where n represents the number of samples, w represents week, * represents P<0.05, ** represents P<0.01, and *** represents P<0.001.

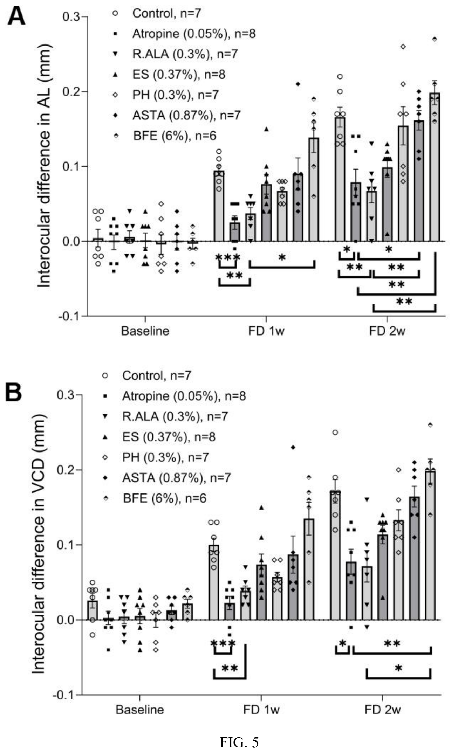

FIG. 5 shows intervention effects of lipoic acid, mexidol, pyridoxol, astaxanthin, and blueberry fruit extract on vitreous chamber depth and axial length in individuals with myopia. Interocular differences changes in axial length (A) and vitreous chamber depth (B) in the same subject were compared between different administration groups before the experiment, 1 week after administration, and 2 weeks after administration. VCD represents the vitreous chamber depth, AL represents the axial length, n represents the number of samples, w represents week, * represents P<0.05, ** represents P<0.01, and *** represents P<0.001.

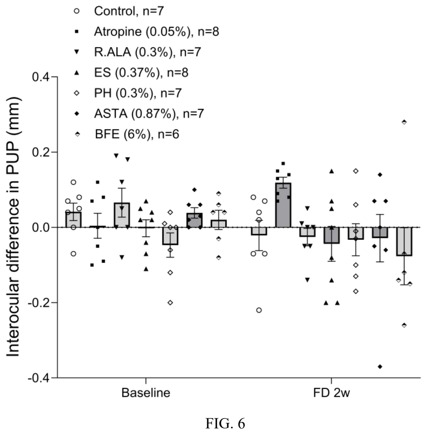

FIG. 6 shows influence of atropine, lipoic acid, mexidol, pyridoxol, astaxanthin, and blueberry fruit extract on pupil size in animal subjects.

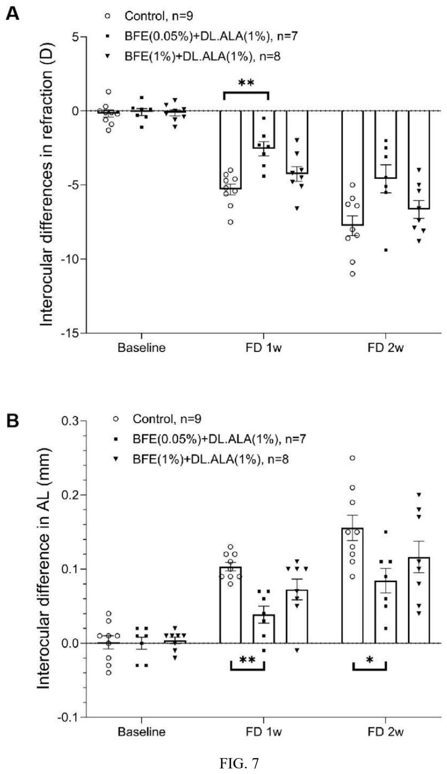

FIG. 7 shows intervention effects of lipoic acid as a main active ingredient or a direct active ingredient on the progression of negative refraction and axial elongation in individuals with myopia. Interocular differences changes in refraction (A) and axial length (B) in the same subject were compared between pharmaceutical compositions of different prescription proportions before the experiment, 1 week after administration, and 2 weeks after administration. At FD 2w, refraction Control VS. BFE (0.05%) + DL.ALA (1%) statistical P value = 0.0569. n represents the number of samples, w represents week, * represents P<0.05, and ** represents P<0.01.

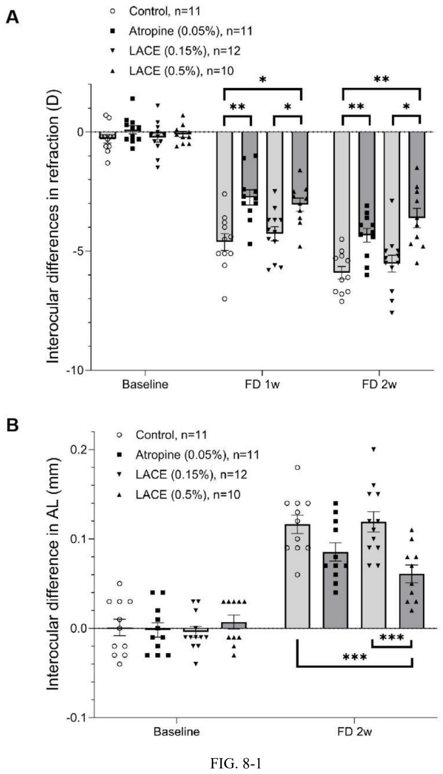

FIG. 8-1 shows significant treatment of myopia with eye drops of lipoic acid choline ester. Interocular differences changes in refraction (A) in the same subject were compared between different concentrations of eye drops of lipoic acid choline ester before the experiment, 1 week after administration, and 2 weeks after administration; and interocular differences changes in axial length (B) in the same subject were compared between different concentrations of eye drops of lipoic acid choline ester before the experiment and 2 weeks after administration. n represents the number of samples, w represents week, * represents P<0.05, ** represents P<0.01, and *** represents P<0.001.

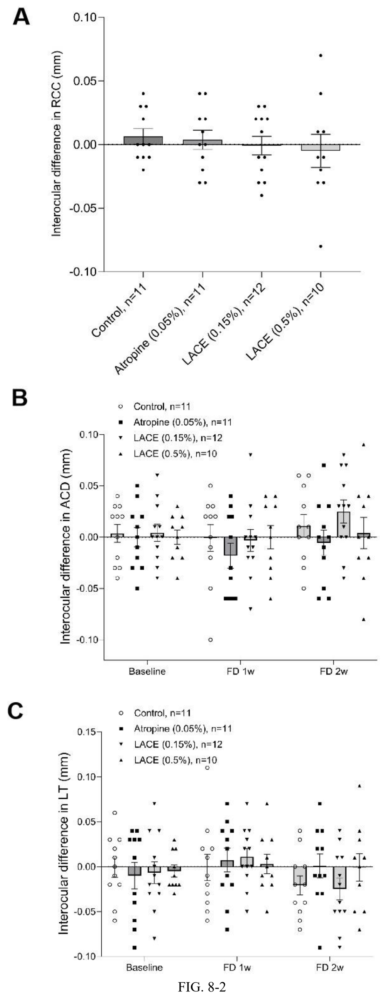

FIG. 8-2 shows influence of different concentrations of lipoic acid choline ester on radius of corneal curvature (A), anterior chamber depth (B), and lens thickness (C), where ACD represents the anterior chamber depth, LT represents the lens thickness, RCC represents the radius of corneal curvature, and n represents the number of samples.

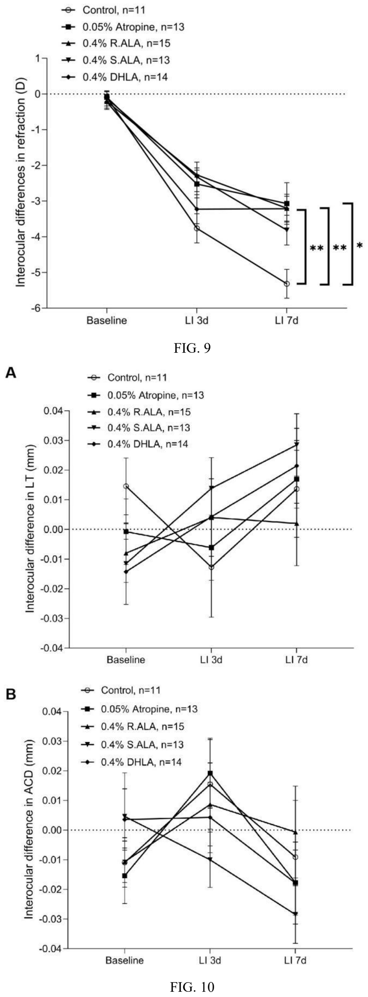

FIG. 9 shows that both an optical isomer of lipoic acid and dihydrolipoic acid can effectively treat negative lens-induced animal model myopia. Interocular differences changes in refraction in the same subject were compared between different chiralities of lipoic acid or metabolites thereof before the experiment, 3 days after administration, and 7 days after administration. n represents the number of samples, d represents day, * represents P<0.05, and ** represents P<0.01.
FIG. 10 shows influence of an optical isomer of lipoic acid and dihydrolipoic acid on lens thickness (A) and anterior chamber depth (B) in an LIM model.

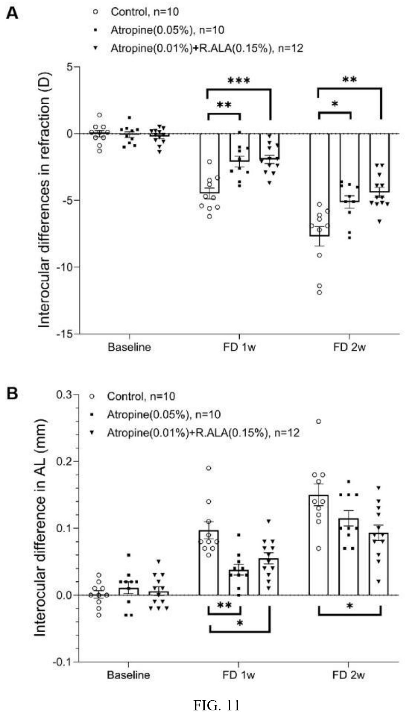

FIG. 11 shows intervention effects of a pharmaceutical composition containing lipoic acid on the progression of negative refraction and axial elongation in individuals with myopia. Interocular differences changes in refraction (A) and axial length (B) in the same subject were evaluated with a compound formulation of atropine and lipoic acid at a low concentration before the experiment, 1 week after administration, and 2 weeks after administration. n represents the number of samples, w represents week, * represents P<0.05, ** represents P<0.01, and *** represents P<0.001.

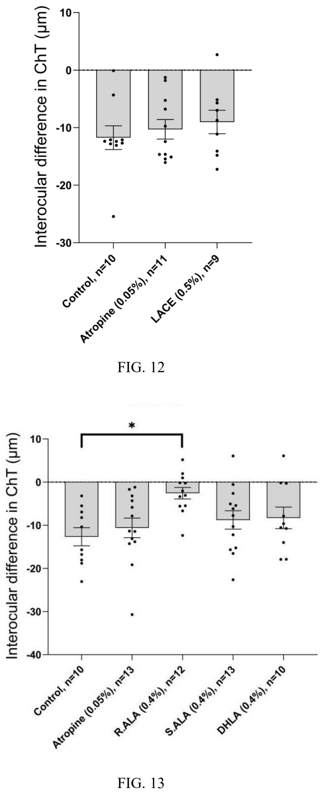

FIG. 12 shows that the choroidal thickness in individuals with myopia may be effectively increased by solely applying eye drops of lipoic acid choline ester, where ChT represents the choroidal thickness, and n represents the number of samples.
FIG. 13 shows that both an optical isomer of lipoic acid and dihydrolipoic acid may effectively increase the choroidal thickness in individuals with myopia, where ChT represents the choroidal thickness, n represents the number of samples, and * represents P<0.05.

### Detailed Description of the Invention

The embodiments of the present application are presented below by way of example. Although the present application has been described in conjunction with these specific embodiments, it should be understood that it is not intended to limit the present application to such specific embodiments. Rather, the present application is intended to cover replacements, modifications, substitutions, variations, and equivalents that may be included within the spirit and scope of the present application as defined in the claims.

The present application provides a method for preventing and treating myopia, which involves administering lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing.

The present application further provides a drug for treating myopia and controlling myopia progression, which is administrated topically to eyes and contains lipoic acid or lipoic acid choline ester as the sole, main, or direct active ingredient. In the prior art, lipoic acid is mainly in the form of tablets, capsules, and injections, and is administrated systemically. However, when lipoic acid or lipoic acid choline ester is used for treating myopia, if the drug is administrated systemically, it is inevitably be affected by factors such as the first-pass effect of drug and the blood-retinal barrier (BRB). As such, it is necessary to administer a large dose of the drug to ensure that lipoic acid or metabolites thereof that enters eyes reach a therapeutic concentration threshold. Such a treatment strategy has a potential risk of systemic adverse reactions, especially when the subjects are young individuals who require long-term continuous administration. Meanwhile, systemic administration is affected by more individual differences, which increases the uncertainty in the efficacy of lipoic acid or lipoic acid choline ester in preventing and controlling myopia. Therefore, the present application has developed a lipoic acid drug for topical ocular administration with better effectiveness and safety. This drug development strategy is aimed at the use of lipoic acid and related compounds thereof (such as UNR844 Chloride) in myopia prevention and control in children and adolescents. It has advantages including convenient administration for patients, high compliance, suitability for long-term administration (for example, for one month, half a year, one year, three years, five years, or ten years), minimal impact on growth and development of young individuals, and safe and reliable ophthalmic dosage forms.

The present application also provides a method, which includes administering to a suitable subject an effective dose of lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing.

The method or pharmaceutical composition of the present application may significantly inhibit onset and development of myopia, markedly inhibit and retard axial elongation and/or an increase in vitreous chamber length in individuals with myopia (including those not yet myopic but predisposed to develop myopia), and increase choroidal thickness in such individuals. Preferably, the method or pharmaceutical composition of the present application are applicable to the population that includes individuals suffering from childhood and/or adolescent myopia (preferably aged 2-30, and more preferably aged 6-18), or early-stage or intermediate-stage myopia, or mild or moderate myopia, or non-pathological myopia, or axial simple myopia, or childhood and/or adolescent axial myopia (preferably aged 2-30, and more preferably aged 6-18), or childhood and/or adolescent progressive myopia (preferably aged 2-30, and more preferably aged 6-18), or primary myopia, or non-age-related myopia, or juvenile myopia, or progressive myopia, or non-refractive myopia. The axial elongation and/or the increase in vitreous chamber length in these individuals with myopia (including those not yet myopic but predisposed to develop myopia) is inhibited or even halted by the method, formulation, or pharmaceutical composition of the present application.

The present application provides a method for treating, preventing, or retarding myopia and related symptoms thereof in a subject, including administrating to the subject a therapeutically effective amount of lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing. Preferably, the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing is administrated separately. Preferably, the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing is administrated in the form of a pharmaceutical composition. Preferably, the pharmaceutical composition is prepared into an ocular formulation. Preferably, the ocular formulation further contains a pharmaceutically acceptable carrier. Preferably, the carrier is an ophthalmically acceptable carrier.

The present application further provides a pharmaceutical composition for treating, preventing, or retarding myopia and related symptoms thereof. The pharmaceutical composition contains lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing. The present application further provides use of lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing in preparing a pharmaceutical composition for treating, preventing, or retarding myopia and related symptoms thereof. Preferably, the pharmaceutical composition is prepared into an ocular formulation. Preferably, the ocular formulation further contains a pharmaceutically acceptable carrier. Preferably, the carrier is an ophthalmically acceptable carrier. Preferably, the carrier is an ophthalmically compatible carrier.

The present application further provides a myopia therapeutic regimen (inhibition of myopia progression) that uses lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing, and other prevention and control drugs (for example, atropine) or optical prevention and control means (for example, OK lenses, or various forms of optical treatment instruments) or refractive surgery for myopia in combination, or at intervals, or alternately for preventing, retarding, and treating myopia.

The present application further provides a myopia therapeutic regimen (inhibition of myopia progression and/or axial elongation) that uses a dosage form for systemic administration (for example, oral tablets) and a dosage form for topical administration (for example, eye drops) of lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing in combination or alternately for preventing, retarding, and treating myopia.

### Terms and Definitions

"Myopia" refers to a condition where in relaxed accommodation, parallel light rays are focused in front of the retina after passing through the eyeball's refractive system of the eyeball. Myopia is commonly manifested with lower distance visual acuity than the normal level and normal near visual acuity. Clinically, individuals are considered myopic if static refraction is ≥ -0.25 diopters (D). The clinical symptoms of myopia include blurred vision for distant objects and clear near vision. In the early stages of myopia, distance vision often fluctuates. Since little or no accommodation is required for near vision, the convergence function is correspondingly weakened, which may easily lead to exophoria or exotropia. In certain paragraphs of the present description, the terms "myopia" and "myopic eye" represent identical concepts and are interchangeable. Those skilled in the art should correctly understand the meaning of "myopia" or "myopic eye" herein based on the context.

In a first aspect, myopia has the following four common classifications: (1) classification by diopters: mild myopia (≤ -3 diopters), moderate myopia (-3 diopters to -6 diopters), and high myopia (≥ 6 diopters); (2) classification by whether refractive components are abnormal: refractive myopia and axial myopia; (3) classification by whether pathological changes occur: pathological myopia and simple myopia; and (4) classification by cause: primary myopia and complicated/secondary myopia.

In some embodiments, myopia or myopic complications may include or may not include any one or more of the types of myopia or myopic complications mentioned in the present application, for example:
"Mild or moderate myopia": Patients have no other symptoms except blurred vision for distant objects. That is, the main symptom is a gradual decline in distance visual acuity, leading to blurred vision for distant objects, while near visual acuity remains normal. Typically, there are no special changes in eyes except for the axial length.
"High myopia" (for example, -6 D): Patients have a deeper anterior chamber and a larger pupil, and the eyeball appears slightly prominent due to an excessively long anteroposterior axis. A white or grayish-white crescent-shaped spot (known as a myopic semilunar spot) may be seen on the temporal side of the optic disc. This is caused by the backward elongation of the sclera, which separates the retinal pigment epithelium and choroid from the temporal edge of the optic disc, exposing the sclera or part of the choroid and sclera. As the sclera at the posterior pole continues to expand backward, knee crack-like streaks and subretinal neovascularization may appear in the macula. Spot-like atrophy and degeneration may occur in the adjacent retina and choroid, leading to posterior scleral staphyloma. Pigment hyperplasia (and even hemorrhage) often occurs in the macula, forming an atrophic spot (Forster-Fuchs spot). Such patients are often accompanied by vitreous liquefaction and opacity. Besides, a few patients may also develop retinal detachment and complicated cataract. Furthermore, high myopia is often associated with opacity of the refractive media and degeneration of the retina and choroid, resulting in poor distance visual acuity and poor near visual acuity, and sometimes floating spots in front of the eyes. If such lesions occur, this type of myopia is no longer simple myopia.
"Refractive myopia": This type of myopia is mainly caused by an excessively large curvature of the cornea or lens, resulting in the refractive power exceeding the normal range, while the axial length is within the normal range.
"Axial myopia": The axial length exceeds the normal range, while the curvature of the cornea and lens is basically within the normal range. Axial myopia is the main type of myopia in children and adolescents.
"Pathological myopia": This type of myopia, also referred to as degenerative myopia or malignant myopia, is a degenerative change of the fundus. Patients usually have high myopic refractive diopters (for example, lower than -6 diopters) and suffer significant impairments of the visual function, with worse distance visual acuity. Moreover, abnormalities are often found in visual field, light perception, contrast sensation, etc., and are often accompanied by poor night vision (nyctalopia), spotted vision, floaters, photopsia, and the like. Thinning of the retinal pigment epithelium and choroid may be seen in the fundus of patients, which is often accompanied by symptoms such as atrophy of the retinal pigment epithelium, choroidal neovascularization, retinal detachment, and macular degeneration. Pathological changes in the fundus continue to progress even after the development stops. This type of myopia may lead to blindness in severe cases.
"Simple myopia": It refers to myopia that develops during the development period of the eyeball. Once the development stops, the myopic refraction tends to stabilize. It often occurs during school age, with a myopic degree of higher than -6 diopters and generally without obvious pathological changes in the fundus. It is also called acquired myopia. This type of myopia develops progressively, and the axial length also increases progressively. Visual acuity can be corrected to the normal level with appropriate lenses, and other visual function indicators are mostly at the normal level. In contrast, this type of myopia is non-simple myopia, and examples of the non-simple myopia include pathological myopia, high myopia, myopia in the elderly, complex myopia, and the like.
"Primary myopia": It is a main type of myopia. The "primary" myopia refers to a type of myopia of unknown cause where the etiology and pathogenesis cannot be determined using existing diagnostic techniques. During onset and development, it exhibits myopia-specific pathological or physiological functional-structural changes, including congenital myopia and acquired simple myopia.
"Complicated/secondary myopia": This type of myopia refers to transient or concomitant myopia caused by eye accommodation dysfunctions or abnormal refractive index under the influence of internal and external factors. Examples of this type of myopia include toxic myopia, drug-induced myopia, traumatic myopia, diabetic myopia, myopia due to incipient cataract, myopia induced by diabetes, myopia induced by oxidative degeneration of the lens, myopia induced by corneal damage or oxidative degeneration, progressive complex myopia accompanied by disordered lipid peroxide (POL) oxidation, and the like. This type of myopia is characterized by clear inducing factors in most cases. When medical interventions are used to address the inducing factors, the degree of myopia can be alleviated or reversed, thus leading to fluctuating and recurrent visual acuity in patients with this type of myopia. This type of myopia also has a high incidence in the elderly population.
"Axial simple myopia": This type of myopia, sometimes also referred to as simple axial myopia, is a simple myopia, which is characterized in that the imaging focus is in front of the retina due to axial elongation and/or an increase in vitreous chamber depth. This type of myopia features substantially normal refractive tissue (for example, the lens) of the eye, and is the most common type of myopia in children and adolescents. It mostly occurs in individuals aged 2 to 30, especially 6 to 18 (Paul N Baird, Nat Rev Dis Primers. 2020 Dec 17; 6(1):99., A J Adams, Am J Optom Physiol Opt. 1987 Feb; 64(2):150-2, and Seang-Mei Saw, Ophthalmic Physiol Opt. 2005 Sep; 25(5):381-91.).
"Curvature myopia": It is myopia caused solely by an increased curvature of the cornea or lens.
"Index myopia": It is myopia caused mainly by an increase in refractive power resulting from an increased refractive index of the aqueous humor or lens, and is a type of refractive myopia.
"Progressive myopia": It is a type of myopia where the refraction continues to decrease over time or as an individual grows older. Without intervention or with the eyes stopping growing and developing before a patient reaches the specific age, this type of myopia generally eventually develops into high myopia.
"Myopia in children and adolescents": It is a type of simple myopia caused by prolonged close-up work. The vast majority of patients with this type of myopia do not develop pathological myopia. The eyeballs of children or adolescents are in the growth and development stage, with strong accommodative capacity and high scleral extensibility. In close-up work such as reading and writing, the eyeballs are in a defocused state, which results in elongation of anteroposterior axis of the eyeball over time, making it easy to develop axial myopia in children and adolescents. Without changes of eye use habits or medical intervention against myopia, the degree of myopia in children and adolescents gradually increases, leading to even longer axial length correspondingly. Therefore, as children and adolescents grow and develop and face intense academic pressure, it may be seen dynamic change of axial length continues to change, and continuous progression of negative refraction. This type of myopia is thus progressive, rather than being mostly static like myopia in adults or the elderly. Accordingly, the main indicators for clinically evaluating the efficacies of drugs for treating this type of myopia are the control of myopia progression and the inhibition of axial elongation.
"Myopia in the elderly": It is usually complex, easily affected by organ aging and underlying diseases, and often accompanied by symptoms such as blood glucose change, vascular sclerosis, insufficient blood supply, ocular microcirculation disorder, and oxidative stress. Most clinically treated patients with this type of myopia have high myopia. A notable feature of adult or elderly patients with myopia is that the degree of myopia generally does not increase with age. The main reason is that the growth and development of eyes in such individuals has basically stopped, and the axial length no longer changes due to prolonged close-up work. Therefore, myopia in the elderly (including adults) and myopia in children and adolescents (minors) are two distinct diseases, and the two indications need to be clearly distinguished in myopia treatment methods. Although the drugs or methods of the present application are suitable for treating various types of myopia, they are preferably used for simple myopia, axial myopia, and myopia in children and adolescents, rather than non-simple myopia such as myopia in the elderly, malignant myopia, and pathological myopia. This is because the clinical intervention strategy for patients with non-simple myopia is no longer to inhibit axial elongation, but rather to prevent blindness caused by severe ocular lesions, for example, blindness induced by retinal detachment.
"Myopia-related symptoms": These symptoms include complications induced by myopia, for example, complications of high myopia, such as the following complications induced by myopia: spotted vision, glaucoma, posterior staphyloma, retinal detachment, retinal tear, amblyopia, macular hemorrhage, choroidal neovascularization, choroidal atrophy, macular degeneration or maculopathy, visual field defect, progressive or sudden diminution of visual acuity (especially near visual acuity), sore and swelling eye and/or eye pain, poor night vision (nyctalopia), astigmatism, anisometropia, loss of sight, vitreous liquefaction, vitreous opacity, strabismus, frequent blinking, photopsia, frequent eye rubbing, blurred vision for distant objects, necessitating squinting or partially closing the eyelids to see distant objects clearly, headache caused by eye strain, difficulty in vision while driving, especially at night (night myopia), retinal atrophy and degeneration (hemorrhage and hiatus), subretinal neovascularization, and eyeball atrophy. It may also be accompanied by: fundus changes of varying degrees, such as myopic arc, macular hemorrhage or subretinal neovascular hemangioma formation; irregular white atrophic spots, pigmented round black spots (Fuchs spots); lattice degeneration and cystoid degeneration in the periphery of the retina; and vitreous liquefaction, opacity, and posterior vitreous detachment at a relatively young age. The risk of retinal hiatus and detachment is higher than that in normal individuals. Usually, due to the elongated anteroposterior diameter of the eyeball, the eyeball becomes more prominent and the posterior pole of the eyeball is expanded, leading to scleral staphyloma. The complications further include choroidal atrophy, retinopathy, macular hemorrhage, and the like caused by myopia, especially high myopia.
"Complex myopia": Compared with simple myopia, the main clinical manifestation of complex myopia is that complications caused by myopia, especially high myopia, endanger visual acuity (whether near visual acuity or distance visual acuity) or induce severe visual impairments (such as retinal detachment and blindness), rather than merely a simple decrease in refraction in myopia. Complex myopia is common in progressive complex myopia accompanied by disordered lipid peroxide (POL) oxidation, myopia in the elderly, myopia caused by hyperglycemia (for example, diabetic myopia), high myopia, or the like. In contrast, other types of myopia are non-complex myopia. Examples of non-complex myopia include myopia in children and adolescents, axial myopia, simple myopia, progressive myopia, mild myopia, moderate myopia, and the like.
"Accommodative spastic myopia": This type of myopia is caused by accommodative tension or spasm resulting from excessive close-up work load on the eyeballs and excessive accommodation of the ciliary muscle and the like.
"Astigmatic myopia": It is a type of myopia where incoming light is scattered in all directions and cannot form a focal point on the retina due to corneal damage or different corneal curvatures.
"Pseudomyopia": it refers to a condition where the myopic degree disappears and emmetropia or hyperopia is present after refraction examination with a cycloplegic agent.
"True myopia": It is usual myopia, referring to a condition where the myopic refraction does not decrease, or decreases by less than 0.5 D, after the use of a cycloplegic agent.
"Mixed myopia": It refers to a condition where the myopic refraction decreases significantly but does not return to emmetropia after the use of a cycloplegic agent.

Other types of myopia include traumatic myopia, toxic myopia, drug-induced myopia, diabetic myopia, instrument-induced myopia, empty field myopia, night myopia, myopia in premature infants, diving-induced myopia, hysterical myopia, and transient myopia occurring during menstruation, pregnancy, and various eye diseases and systemic diseases. For "traumatic myopia", myopia can be induced mainly by blunt trauma among eye traumas, with a refraction mostly lower than -6.00 D.

"Toxic myopia": A myopic response caused by acute or chronic poisoning from toxic substances such as organophosphorus pesticides.

"Drug-induced myopia": It is myopia induced by various drugs, such as sulfonamides, diuretics, tetracyclines, adrenocorticotropic hormone, and contraceptives.

"Diabetic myopia": It is transient myopia developed in diabetic patients.

"Instrument-induced myopia": It is myopia induced by prolonged operation of instruments or equipment at close range.

"Empty field myopia": It refers to a myopic phenomenon caused by the lack of visual stimulation in the normal environment when a person is at a high altitude, gazing at the empty field of vision around the person.

"Night myopia": It refers to a myopic state of the human eyes in a dark environment with light dimmed.

In a second aspect, intervention means for myopia may be classified into myopia correction and myopia treatment, where the major characteristic and purpose of myopia treatment are inhibiting (controlling) myopia progression.

"Myopia correction" refers to the optical correction or reduction of the degree of myopia. Common correction means include wearing framed glasses, clear lens phacoemulsification, intraocular lens implantation, corneal laser surgery, and the like. However, these means merely correct the refraction of the individual and cannot delay myopia progression or reverse excessive axial elongation. That is to say, such myopia intervention methods generally do not serve to halt or retard the progress of continuously progression of negative refraction, and cannot inhibit myopia progression. Consequently, myopia correction methods, such as corneal laser surgery, compensate for refractive errors of the patients and restore the distance visual acuity of the patients. These methods are suitable for improving visual acuity in individuals with pathological myopia, high myopia, or myopia in the elderly, but are not suitable for treating axial myopia or simple myopia, such as myopia in children or adolescents, primary myopia, or mild and moderate myopia. In fact, although distance visual acuity is temporarily restored in individuals with axial myopia after wearing framed glasses, this corrective approach does not inhibit further axial elongation or control the rate of myopia development. Without other pharmacological treatments, the result is a further increase in the degree of myopia. Consequently, patients need to replace their framed glasses with higher prescription degrees at an interval to continue correcting visual acuity.

"Inhibiting (controlling) myopia progression" refers to using various methods or drugs to delay (retard) the continuous increase in degree of myopia, reduce the rate of progression of negative refraction, control the development of myopia, and inhibit (control) continuous axial elongation. This constitutes an etiological treatment, and the targeted patients are predominantly minors whose eyes are still in the developmental stage. For the treatment of myopia in children or adolescents, the primary goal in developing myopia treatment drugs is to retard, control, or even halt the continuous progression of negative refraction and to inhibit the corresponding increase in axial length. That is, for myopia types such as myopia in children or adolescents, axial myopia, and simple myopia, treating myopia should achieve the effect of controlling or inhibiting the development of myopia, rather than simply reversing or correcting this refractive error state.

Unless otherwise specified, "treating (myopia)" in the present application refers to inhibiting (controlling) myopia progression, rather than correcting myopia. As used herein, the terms "treat", "retard", "delay", "inhibit", or "control" refer to treatment measures aimed at retarding (alleviating) or halting the targeted diseases or disorders. For example, after receiving a therapeutic amount of a lipoic acid compound or a pharmaceutical composition containing the same according to the method described herein, a subject exhibits observable and/or measurable inhibition, delaying, reduction, and disappearance of one or more symptoms and signs of an ophthalmic disease, or retarding and delaying in disease progression, whereby the ophthalmic disease of the subject is successfully treated.

It should also be understood that drugs capable of treating myopia typically also possess a "preventive" effect, i.e., can prevent onset and development of myopia. Therefore, the phrase "prevention and control" is also frequently used herein to convey the meanings of both treatment and prevention.

The various modalities of treating or preventing medical diseases described herein are intended to be "significant", encompassing both complete treatment or prevention and less than complete treatment or prevention, where a biologically relevant or medically relevant outcome is achieved. In some embodiments, "treatment" does not require 100% elimination of myopia or symptoms thereof. In some embodiments, "treating" myopia or myopia-related symptoms according to the method of the present application results in prevention, reduction, suppression, or inhibition of myopia progression by, for example, at least about 5%, at least about 10%, or at least about 20%, compared to the levels observed in the absence of the composition or method of the present application (for example, in biologically matched control subjects or samples not exposed to the composition of the present application or the compounds of the method of the present application). In some embodiments, myopia or myopia-related symptoms are treated by at least about 30%, at least about 40%, at least about 50%, at least about 60%, at least about 70%, at least about 80%, at least about 90%, or more (about 100%), compared to myopia or myopia-related symptoms in the absence of the compounds of the method of the present application. Consequently, a person skilled in the art may understand that "treat", "retard", "delay", "inhibit", "control", or "prevent and control" refer to direct actions rather than means that beneficially impact myopia treatment indirectly like ocular health care effects or adjunctive therapeutic effects, such as enhancing ocular blood circulation, or improving comfort during ocular administration.

In a third aspect, a method for evaluating therapeutic effect (efficacy) of myopia treatment involves multiple factors, such as whether the sample population is scientifically selected, whether an objective control is set, and whether an effective dose. In particular, if most of the sample population is the elderly (with ceased eye development and potential underlying diseases such as arteriosclerosis), it is difficult to prove that a drug has therapeutic effect on progressive myopia. If the efficacy is evaluated only through a visual acuity test without designing any parallel control (for example, a placebo group or the contralateral eye of the same subject), it is also impossible to prove that the drug (for example, lipoic acid) has therapeutic effect on myopia. The present application uses two classic myopia disease models-form-deprivation myopia and negative lens-induced myopia-in young animals with eyes still in the growth and development stage, which are commonly used in the preclinical development stage of practical myopia drugs, as well as scientific test standards in the field to evaluate efficacy of compounds in preventing and controlling myopia, especially in preventing and controlling myopia in children and adolescents and/or simple myopia (D A Goss, Am J Optom Physiol Opt. 1981 Oct; 58(10):859-69., and Hao Wu, Proc Natl Acad Sci U S A. 2018 Jul 24; 115(30):E7091-E7100., and Sen Zhang, Invest Ophthalmol Vis Sci. 2019 Jul 1; 60(8):3074-3083.).

"Efficacy" refers to evaluating therapeutic effect of a particular pharmaceutical composition or formulation on myopia or comparing efficacies in accordance with the requirements for drug registration applications and relevant content in the Technical Guidelines for Clinical Research of Drugs for Controlling Myopia Progression issued by the Center for Drug Evaluation of China, using refraction or a combination of refraction and eye axis parameter (length) as the determination indicator. Moreover, variations from baselines in corneal curvature, vitreous chamber depth, choroidal thickness, and axial length may also serve as secondary efficacy indicators for evaluating myopia treatment. Methods for efficacy evaluation or methods for calculating an efficacy contribution rate may utilize a variation value from a baseline in refraction, a variation value from a baseline in axial length, a variation value from a baseline in vitreous chamber depth, and the like. Positive efficacy determination criteria may include a 50% reduction in progression of refractive error compared to the control, or an intergroup difference in refraction exceeding 0.75 D after treatment for three years. For a myopia treatment drug containing two or more active ingredients, a scientific and reasonable design involves separately evaluating the efficacies of monotherapies of all the active ingredients and a compound formulation against myopia. This allows for obtaining the efficacy contribution rate of each active ingredient and the role thereof in the compound formulation (for example, pharmacodynamic synergistic effect, stabilizer effect, or absorption-promoting effect). Regarding the efficacies in preventing (preventing and controlling) myopia, inhibiting myopia progression, or correcting myopia, the foregoing evaluation indicators and determination criteria for myopia treatment and methods for evaluating effects of active ingredients may also be referenced to determine or derive the efficacy and clinical significance of a specific compound (for example, lipoic acid or lipoic acid choline ester).

In a fourth aspect, other conventional terms are explanatorily elaborated.

"Distance visual acuity", also referred to as uncorrected distance visual acuity, medically refers to the visual acuity measured at a standard horizontal distance of 5 meters from the visual acuity chart, with the eyes open and gazing straight ahead, without wearing glasses or any visual acuity-improving auxiliary devices (such as framed glasses, contact lenses, cosmetic contact lenses, and stenopaic spectacles).

"Topical administration" refers to direct administration of a drug to a body part of interest or an administration route that allows for taking effect topically, including but not limited to epidermal administration, inhalation administration, administration as an enema, ocular administration, and nasal administration.

"Systemic administration" includes administration routes such as intravenous administration, oral administration, intramuscular injection, and subcutaneous injection, whereby a drug may be distributed throughout the body via bloodstream.

"Improved systemic administration" refers to a safe, efficient, and convenient form of systemic administration that utilizes specific drug targeting technologies to effectively reduce drug toxicity associated with systemic administration while simultaneously enabling enrichment of a lipoic acid drug in the eyes.

"Direct active ingredient", also referred to as "active (pharmaceutical) ingredient", denotes a substance that has direct therapeutic effect on a disease (for example, myopia). It is a prerequisite for treating a disease, and parameters such as administration dosage thereof and administration route thereof directly impact treatment outcome of the disease. A clear causal relationship exists between administration of the ingredient and achievement of the therapeutic effect. For instance, if efficacy of a compound formulation without the ingredient significantly worsens and has a significant difference compared to the original formulation, then the removed ingredient is considered a direct active ingredient.

"Sole active ingredient" refers to the only ingredient in a final product (such as a drug) that exerts efficacy, for example, directly inhibiting, preventing and/or treating myopia.

"Main active ingredient" refers to an active ingredient of which an addition amount, proportion, concentration or exerted efficacy is not lower than those of other active ingredients (such as those with myopia treatment effects) in a final product (such as a drug), or an active ingredient of which a content accounts for more than 50%, more than 60%, more than 70%, more than 80%, or more than 90% of all active ingredients, where the percentage is a mass ratio or a molar ratio.

"The foregoing" refers to the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof.

"Ophthalmically compatible" refers to formulations, polymers and other materials and/or dosage forms that are suitable for contact with ocular tissues of humans and animals without excessive toxicity, irritation, allergic reactions or other problems or complications (commensurate with reasonable benefits/risk ratios).

In the present description and the appended claims, unless the context clearly dictates otherwise, singular forms, including the singular forms "a," "an", and "the", specifically encompass plural referents of the terms they refer to. Additionally, as used herein, unless specifically stated otherwise, the word "or" is used in the inclusive sense of "and/or" rather than the "exclusive sense" of "either/or".

As used herein, references to numerical ranges for variables are intended to convey that the present application may be practiced with variables equal to any values within the ranges. Thus, for inherently discontinuous variables, the variables may be equal to any integer values within the numerical ranges, including the endpoints of the ranges. Similarly, for inherently continuous variables, the variables may be equal to any real values within the numerical ranges, including the endpoints of the ranges. For example, a variable described as having a value between 0 and 2 may be 0, 1, or 2 for inherently discontinuous variables, and 0.0, 0.1, 0.01, 0.001, or any other real value for inherently continuous variables.

As used herein, "about" may be understood by a person of ordinary skill in the art and vary to some extent depending on the context in which it is used. If use of the term is unclear to a person of ordinary skill in the art, then in the context in which the term is used, "about" may mean a value within a range of the enumerated value plus 10% or minus 10%.

As used herein, "administering" a compound, formulation, or drug to a subject includes any route of introducing or delivering the compound to the subject to perform the intended function. "Administration" may be carried out through any suitable route, including oral, intraocular, ocular surface, intranasal, parenteral (intravenous, intramuscular, intraperitoneal, or subcutaneous) or topical administration (for example, topical application to skin in periocular and other regions). "Administration" includes self-administration and administration by another person. For ocular or periocular administration, the administration may be directed to the affected eye, the unaffected eye, or both eyes of the subject.

As used herein, the term "effective amount" refers to an amount sufficient to achieve the desired therapeutic and/or preventive effect, for example, an amount that causes prevention or alleviation of a condition associated with an ophthalmic disease. The amount of a composition administrated to a subject may vary depending on type and severity of the disease and characteristics of the individual, such as general health, age, gender, body weight, and tolerance to drugs. The amount may also depend on degree, severity, and type of the disease. A person skilled in the art may be able to determine an appropriate dose based on these factors and other factors. The composition may alternatively be administrated in combination with one or more other therapeutic compounds. In the method described herein, a lipoic acid compound or a pharmaceutical composition containing the same may be administrated to a subject exhibiting one or more symptoms or signs of an ophthalmic disease. For example, a "therapeutically effective amount" of lipoic acid or lipoic acid choline ester refers to an average level of pharmacological action that alleviates the ophthalmic disease to the minimum extent.

As used herein, the terms "formulation" and "composition" are used interchangeably and refer to a mixture of two or more compounds, elements, or molecules. In some aspects, the terms "formulation" and "composition" may be used to refer to a mixture of one or more active agents with a carrier or other excipients. The composition can take virtually any physical form, including solid, liquid (for example, solution), or gas.

Furthermore, the term "dosage form" may encompass one or more formulations or compositions provided in a form suitable for administration to a subject. For example, an injectable dosage form is a formulation or a composition prepared in a manner suitable for administration by injection.

As used herein, the term "pharmaceutically acceptable" means being approved by a regulatory authority, such as CFDA (China), EMEA (Europe), and/or FDA (US), and/or any other national regulatory authority, for use in animals, preferably in humans, such as a drug carrier, drug concentration, or form of drug presence.

As used herein, the term "simultaneous" therapeutic application refers to administration of at least two active ingredients (compounds) via the same route at the same time or substantially the same time.

As used herein, the term "separate" therapeutic application refers to administration of at least two active ingredients (compounds) via different routes at the same time or substantially the same time.

As used herein, the term "sequential" therapeutic application refers to administration of at least two active ingredients at different time, via the same route or different routes. More specifically, sequential application means that one of the active ingredients is completely administrated before administration of other active ingredients. Thus, one active ingredient may be administrated several minutes, hours, or days before administration of other active ingredients. No simultaneous treatment is performed in this case.

As used herein, the term "prevention" of a disorder or disease refers to a compound, in statistical samples, reducing onset of the disorder or disease in a treated sample relative to an untreated control sample, or delaying onset of one or more signs of the disorder or disease or reducing the severity of one or more signs of the disorder or disease relative to the untreated control sample.

As used herein, the term "related compound" includes any compound having the function of lipoic acid or lipoic acid choline ester, such as derivatives or analogs. A person skilled in the art also know how to prepare various related compounds of lipoic acid or related compounds of lipoic acid choline ester that retain the function of the lipoic acid or the lipoic acid choline ester.

As used herein, the term "derivative" or "analog" of a compound includes any molecule related to the compound in function and/or structure, such as acids, amides, esters, ethers, acetylated variants, hydroxylated variants, or alkylated variants of the compound. The term "derivative" further includes structurally related compounds that have lost one or more of the substituents listed above. Preferred derivatives of a compound are molecules that have a significant degree of similarity to the compound, as determined by known methods. Analogous compounds, along with the similarity indices thereof to the parent molecule, may be found in numerous databases, such as PubChem (http://pubchem.ncbi.nlm.nih.gov/search/) or DrugBank (http://www.drugbank.ca/). In a more preferred embodiment, derivatives should have a Tanimoto similarity index greater than 0.4, preferably greater than 0.5, more preferably greater than 0.6, and even more preferably greater than 0.7 with the parent drug. The Tanimoto similarity index is widely used to measure the degree of structural similarity between two molecules. The Tanimoto similarity index may be calculated by software available online, such as Small Molecule Subgraph Detector (http://www.ebi.ac.uk/thornton-srv/software/SMSD/). Preferred derivatives should be related to the parent compound both structurally and functionally, i.e., they should also retain at least part of the activity of the parent drug. Moreover, the term "derivative" further includes metabolites of a drug, for example, molecules produced by (biochemical) modification or processing of the drug, usually by specialized catalytic systems, after administration to an organism, and which exhibit or retain the biological activity of the drug. Moreover, the term "derivative" further includes halogenated (for example, fluorinated), protiated, deuterated, tritiated compounds or compositions thereof in the form of lipoic acid or salts thereof.

The "derivatives" of lipoic acid choline ester refer to any compound or a mixture of compounds other than lipoic acid and choline formed by reaction of lipoic acid choline ester with non-aqueous pharmaceutical excipients. In some embodiments, the derivative is a product formed by reaction of lipoic acid choline ester with propylene glycol. In certain embodiments, the derivative is a product formed by reaction of lipoic acid choline ester with glycerol.

Salt forms of lipoic acid choline ester, inclusive of all salt forms of lipoic acid choline ester described in CN115279745A, include but are not limited to lipoic acid choline ester tosylate, lipoic acid choline ester besylate, lipoic acid choline ester chloride, or lipoic acid choline ester iodide.

In a specific embodiment, the term "metabolite" as used herein refers to a modified or processed drug that retains at least part of the activity of the parent drug, preferably exhibiting an inhibitory effect against the activity of phosphodiesterase or having therapeutic, preventive, or retarding effects on myopia and related symptoms. For example, in an organism, lipoic acid may be reduced to dihydrolipoic acid or oxidized to beta-lipoic acid, which are metabolites of lipoic acid.

"Extract" refers to a substance extracted or processed from a raw material derived from microorganisms, plants, or animals (for example, the whole or any part of a plant) by using appropriate chemical or physical methods, such as solvent extraction or squeezing. This substance may be a pure product, may contain minor impurities, or may be a mixture.

"Ophthalmic composition" or "ocular formulation" or "ophthalmic formulation" refers to an ophthalmic composition, or an ophthalmic pharmaceutical composition, or an ophthalmic medicinal product; or a drug, formulation, cosmetic, health care product, drug-device combination, or device for prevention and/or treatment of eye diseases, protection, maintenance, or improvement of visual acuity, and avoidance, retarding, or reversal of visual impairments; or eye drops (eye water), eye ointments, ophthalmic sprays, implant tablets, ophthalmic gels, eye patches, ophthalmic microspheres, ophthalmic sustained-release formulations, periocular injections, intraocular injections, etc.

"Fish oil" refers to lipid substances derived from higher animals, particularly fish (such as cod and salmon), squid, and seals, specifically referring to the polyunsaturated fatty acids contained therein, including but not limited to Omega-3 unsaturated fatty acids, DHA, EPA, DPA, ALA, nisinic acid, stearidonic acid, eicosatetraenoic acid, or a combination thereof.

The active components in the formulations or pharmaceutical compositions of the present application are added to water or other suitable solvents that have been deoxygenated with an inert gas to a level of less than 5 ppm, where the preferred inert gas is nitrogen, and the preferred deoxygenation level is 2 ppm.

The administration routes of lipoic acid and related compounds thereof may be eye drop instillation, injection, infusion, and oral administration.

In terms of specific formulations, the formula of the ocular formulations includes any one or a combination of cyclodextrin (for example, hydroxypropyl beta-cyclodextrin), distearoyl phosphatidylcholine, soybean lecithin, octadecylamine, Poloxamer 188, sodium chloride, mannitol, mercaptoethanol, tromethamine, sodium sulfite, ethylenediamine, benzyl alcohol, and arginine.

Lipoic acid formulations for treating myopia further include other active ingredients and pharmaceutical excipients, such as reducing agents (glutathione and N-acetylcarnosine), glycerol, propylene glycol, ethyl pyruvate, semifluorinated alkanes, amino acids or derivatives thereof (such as alanine, methionine, cysteine, and histidine), sugars or metabolites thereof (for example, glucose-6-phosphate), oxygen scavengers (for example, Oxy-Guard^{™} or StabilOx^{™}), and vitamins (vitamin B1, vitamin B2, vitamin C, and vitamin E).

In a pharmaceutical composition or formulation containing lipoic acid choline ester, the lipoic acid choline ester has a counter ion, including but not limited to chloride ion, bromide ion, iodide ion, sulfate radical, methanesulfonate radical, nitrate radical, maleate radical, acetate radical, citrate radical, fumarate radical, hydrogen fumarate ion, tartrate ion (optical isomers or a mixture thereof), succinate radical, benzoate radical, and glutamate anion, for example, all compositions mentioned in CN109906076A, particularly the compositions mentioned in claims 1 to 9 thereof. In one embodiment, the pharmaceutical composition contains 0.1%-10% of a pharmaceutical salt of lipoic acid choline ester, 1%-30% of cyclodextrin, 0.1% -2% of a tonicity regulator, 0.1%-0.5% of a viscosity enhancer, 0.003 % -0.010% of a preservative, 0.05% to about 1.0% of a biochemical energy source and water for injection.

The present application discloses and identifies a compound for treating, preventing, or retarding myopia and related symptoms thereof, namely lipoic acid, to enhance the effect of reducing or retarding myopia while avoiding or minimizing adverse side effects, such as those observed with atropine therapy.

The present application relates to a pharmaceutical composition or a method for treating, preventing, or retarding myopia and related symptoms thereof in individuals, such as infants, school-age children, adolescents, or younger adults. In some instances, treating, preventing, or retarding myopia and related symptoms thereof may include administering a therapeutically effective amount of a pharmaceutical composition or dosage form to a subject in need thereof.

The pharmaceutical composition or formulation contains a therapeutically effective amount of a lipoic acid or lipoic acid choline ester compound, or a salt thereof, or a related compound thereof.

In another aspect, the present application provides an ophthalmic device containing a pharmaceutical composition. The pharmaceutical composition contains lipoic acid or a therapeutically acceptable salt thereof or a derivative thereof. Preferably, the ophthalmic device delivers the pharmaceutical composition in a sustained release manner; and more preferably, the ophthalmic device delivers the pharmaceutical composition by pulse.

Through experiments, we surprisingly found that the use of lipoic acid or lipoic acid choline ester alone may significantly retard progression of negative refraction in form-deprivation or lens-induced myopia models in guinea pigs, and even completely inhibit (halt) the progression of negative refraction in some individuals, and may significantly inhibit axial elongation. Based on this, it may prove that lipoic acid compounds have the effects of preventing or treating myopia and preventing or controlling progression of myopia in animals, especially in humans, such as infants, school-age children, children and adolescents, minors, individuals aged 2 to 30, or young adults.

In some embodiments, the subjects of the technical solutions of the present application are children or adolescents, aged 2-30, preferably 6-18, or 12-18. In some embodiments, the subjects of the technical solutions of the present application are adults, for example, aged 16-65, and preferably 16-26.

In topical administration experiments, no discomfort or ocular abnormalities were observed in all administrated animals, and no toxic manifestations such as weight loss or decreased food intake were observed. Based on the existing clinical application basis of lipoic acid, a person skilled in the art may expect that lipoic acid will have good drug safety when used in the clinical treatment of treating, preventing, or improving myopia and related symptoms thereof.

The pharmaceutical compositions of the present application have significantly superior technical effects to atropine in treating, preventing, or retarding myopia and related symptoms thereof, and no adverse reactions such as ocular irritation, pupil dilation, inflammation, or allergy were observed in experiments.

In certain embodiments of the pharmaceutical composition, ophthalmic device, or treatment method disclosed herein, patients were treated for a period between about 1 month and 20 years, such as at least 6 months, at least 1 year, at least 2 years, at least 3 years, at least 5 years, at least 7 years, or at least 9 years.

In other embodiments, regarding the pharmaceutical compositions, ophthalmic devices, or treatment methods according to any one of the above embodiments and any one or more of the other embodiments herein, the pharmaceutical compositions are topical formulations, such as topical ophthalmic compositions, ophthalmic gel formulations, ophthalmic emulsions, ophthalmic liposomes, nanodiscs, nanoparticle suspensions, pastes, ointments, or unguents (e.g., ophthalmic ointments such as eye ointments), and oral preparations (such as tablets, capsules, powders, or syrups).

In other embodiments, regarding the drug, formulation, composition, device, or treatment method according to any one of the above embodiments and any one or more of the other embodiments herein, the drug, formulation, or composition further contain one or more other ophthalmologically acceptable excipients and additives, including carriers, stabilizers, osmotic pressure regulators, preservatives, antioxidants, buffers, tonicity regulators, thickeners, or other excipients. In other embodiments, the osmotic pressure regulator is sodium chloride. In other embodiments, the preservative is selected from benzalkonium chloride, cetrimonium, sodium perborate, stabilized oxychloro complex, SofZia, polyquaternium-1, chlorobutanol, ethylenediaminetetraacetic acid disodium, polyhexamethylene biguanide, or a combination thereof. In other embodiments, the buffer is selected from borates, borate-polyol complexes, phosphate buffers, citrate buffers, acetate buffers, carbonate buffers, organic buffers, amino acid buffers, or a combination thereof. In other embodiments, the tonicity regulator is selected from sodium chloride, sodium nitrate, sodium sulfate, sodium bisulfate, potassium chloride, calcium chloride, magnesium chloride, zinc chloride, potassium acetate, sodium acetate, sodium bicarbonate, sodium carbonate, sodium thiosulfate, magnesium sulfate, disodium hydrogen phosphate, sodium dihydrogen phosphate, potassium dihydrogen phosphate, dextrose, mannitol, sorbitol, glucose, sucrose, urea, propylene glycol, glycerol, or a combination thereof. In other embodiments, the carrier is selected from, but not limited to water; a mixture of water and a water-miscible solvent; vegetable or mineral oils containing 0.01% to 5% by weight of hydroxyethylcellulose; ethyl oleate; carboxymethylcellulose; carboxymethyl cellulose; hydroxymethylcellulose; hydroxyethylcellulose; ethyl acrylate; polyacrylamide; pectins; alginates; starch derivatives; polyvinyl alcohol; polyvinylpyrrolidone; polyvinyl methyl ether; polyethylene oxide; cross-linked polyacrylic acid; carbomer; lecithin; polyethylene glycol stearate; polyols; acidic cosolvents; heptadecaethyleneoxyl oxidized hexadecanol; or polyoxyethylene sorbitan monooleate.

In other embodiments, regarding the drug, formulation, composition, device, or treatment method according to any one of the above embodiments and any one or more of the other embodiments herein, the drug, formulation, composition, device, or treatment method contains polyols and/or acidic cosolvents, optionally hydroxypropyl-beta-cyclodextrin and citric acid or tartaric acid.

In other embodiments, concerning the use of the present application, the effect of lipoic acid or lipoic acid choline ester, or an optical isomer or racemate thereof, or a solvate thereof, or a pharmaceutically acceptable salt thereof, or a prodrug thereof, or a metabolite thereof, or a related compound or extract thereof, or a crystalline compound thereof, or a combination of the foregoing, on prevention and control (treatment) of myopia or inhibition of axial length, is independent of the gender, age of the subject, type of myopia, rate of myopia progression, and severity of myopia.

In other embodiments, regarding the pharmaceutical composition, ophthalmic device, or treatment methods according to any one of the above embodiments and any one or more of the other embodiments herein, the pharmaceutical compositions are sustained-release formulations or subconjunctival depots; or the pharmaceutical compositions are contained in the ophthalmic devices, such as eye patches, sutures, and corneal contact lenses; or the pharmaceutical compositions are ophthalmic compositions and/or the ophthalmic compositions are contained in the ophthalmic devices; or the ophthalmic devices are contact lenses, ocular inserts, corneal coverings, corneal inlays, nanodiscs, liposomes, nanoparticles, lacrimal plugs, or hydrogel bases with microfluidic depots; or the ophthalmic devices deliver the pharmaceutical compositions in a sustained release manner; or the pharmaceutical compositions are formulated as ophthalmic compositions for treating ophthalmic diseases or conditions; or the pharmaceutical compositions are formulated as ophthalmic compositions for treating pre-myopia (or at risk of developing myopia), myopia, high myopia, moderate myopia, low myopia, true myopia, pseudomyopia, or myopia development; or the pharmaceutical compositions are substantially uniformly distributed throughout the ophthalmic devices; or the ophthalmic devices are contained in contact lens blister packages, or the pharmaceutical compositions are immersed in the ophthalmic devices within the contact lens blister packages.

Regarding pharmaceutical formulation forms, administration modes and dosages, cells, organs, or tissues may be brought into contact with lipoic acid or related compounds thereof using any method known to those skilled in the art. Appropriate methods include in vitro methods, indirect in vivo methods, or in vivo methods. In vivo methods typically involve administering the lipoic acid compound of the present application or a pharmaceutical composition containing the same to a mammal, preferably to a human. When used in vivo for treatment, the lipoic acid compound or the pharmaceutical composition containing the same may be administrated to a subject in an effective amount (i.e., an amount that produces the desired therapeutic effect). The dosage and administration schedule may depend on severity of an ophthalmic disease in a subject, the subject, and the medical history of the subject.

Unlike completely new compounds, lipoic acid (or lipoic acid choline ester) is a mature drug that has been clinically used for many years. Based on the prior art and content of the description, a person skilled in the art may determine the "boundary" of compounds equivalent to lipoic acid (or lipoic acid choline ester), and is able to achieve the objectives of the present application by using a suitable "optical isomer or racemate thereof, or a solvate thereof, or a pharmaceutically acceptable salt or ester thereof, or a prodrug thereof, or a metabolite thereof, or a related compound or extract thereof, or a crystalline compound thereof". For example, regarding different forms of a compound, salt formation of the compound is a conventional technology in the art, and various salts of the compound are generally considered to have similar functions to the original compound (for example, YE Xuan et al., Case studies of salt selection and optimization in drug development, Chinese Journal of New Drugs, 2019, Vol. 28, No. 19, pp. 2332-2335). Optical isomers or racemates thereof, or crystalline compounds thereof have exactly the same molecular structure and composition as the compound, and in the absence of contrary evidence, it is generally acknowledged that these compounds all have similar functions. As for solvates, it is exemplarily described herein that lipoic acid (or lipoic acid choline ester) dissolved in solvents such as normal saline, DMSO, PEG, and Tween is used for treating myopia (see Example 1).

The compound disclosed herein may also exist as a prodrug. The prodrug of the compound described herein is in modified forms that readily undergo chemical changes under physiological conditions to yield lipoic acid or related compounds thereof. Furthermore, the prodrug may be converted into the compound in an ex vivo environment by chemical or biochemical methods. For example, when placed in a transdermal patch reservoir containing suitable enzymes or chemical reagents, the prodrug may be slowly converted into the compound. The prodrug is generally useful because, in some cases, it may be easier to administer than the compound or parent drug. For instance, the prodrug may exhibit bioavailability when administrated orally, whereas this cannot be applied to the parent drug. The solubility of the prodrug in the pharmaceutical composition may also be higher than that of the parent drug. Many prodrug derivatives are known in the art, such as those that rely on hydrolytic cleavage or oxidative activation of the prodrugs. A non-limiting example of a prodrug is a compound administrated as an ester ("prodrug") but then metabolically hydrolyzed to a carboxylic acid (active entity). For example, lipoic acid choline ester chloride is a prodrug of lipoic acid, where chloride is the salt form, and the choline ester may help the compound penetrate the cornea. When or after the lipoic acid choline ester chloride enters the cornea, the lipoic acid choline ester chloride is hydrolyzed into choline and lipoic acid.

The compounds disclosed herein may exist as therapeutically acceptable salts. The present application includes the above-listed compounds in salt form, including acid addition salts. Suitable salts include those formed with organic acids and inorganic acids. Such acid addition salts are generally pharmaceutically acceptable. However, non-pharmaceutically acceptable salts may be used in preparation and purification of problematic compounds. Base addition salts may also be formed and are pharmaceutically acceptable.

As used herein, the term "therapeutically acceptable salt" refers to a salt or zwitterionic form of a compound disclosed herein that is water-soluble, oil-soluble, or dispersible and therapeutically acceptable, as defined herein. Salts may be prepared during final separation and purification of the compounds, or prepared separately by reacting the compounds in appropriate free base form with suitable acids. Representative acid addition salts include acetates, adipates, alginates, L-ascorbates, aspartates, benzoates, benzenesulfonates (besylates), bisulfates, butyrates, camphorates, camphorsulfonates, citrates, digluconates, formates, fumarates, gentisates, glutarates, glycerophosphates, glycolates, hemisulfates, heptanoates, hexanoates, hippurates, hydrochlorides, hydrobromides, hydriodates, 2-hydroxyethanesulfonates (isethionates), lactates, maleates, malonates, DL-mandelates, mesitylenesulfonates, methanesulfonates, naphthalenesulfonates, nicotinates, 2-naphthalenesulfonates, oxalates, pamoates, pectinates, persulfates, 3-phenylpropionates, phosphonates, citrates, picrates, pivalates, propionates, pyroglutamates, succinates, sulfonates, tartrates, L-tartrates, trichloroacetates, trifluoroacetates, phosphates, glutamates, bicarbonates, para-toluenesulfonates (p-tosylates), and undecanoates. Furthermore, basic groups in the compounds disclosed herein may be quaternized with: methyl, ethyl, propyl, and butyl chlorides, bromides, and iodides; dimethyl, diethyl, dibutyl, and dipentyl sulfates; decyl, lauryl, myristyl, and cholesteryl chlorides, bromides, and iodides; and benzyl and phenethyl bromides. Examples of acids that may be used to form therapeutically acceptable addition salts include inorganic acids (such as hydrochloric acid, hydrobromic acid, sulfuric acid, and phosphoric acid) and organic acids (such as oxalic acid, maleic acid, succinic acid, and citric acid). Salts may alternatively be formed by coordination of compounds with alkali metal or alkaline earth metal ions. Thus, the present application encompasses sodium, potassium, magnesium, and calcium salts, among others, of the compounds disclosed herein.

The crystalline forms of lipoic acid encompass all known types, for example, the crystalline form described by Samuel Golob in the document (Samuel Golob, Improving Biopharmaceutical Properties of Vinpocetine Through Cocrystallization, J Pharm Sci. 2016 Dec; 105(12):3626-3633.).

Base addition salts may be prepared during final separation and purification of compounds by reacting carboxyl with suitable bases (such as hydroxides, carbonates, or bicarbonates of metal cations) or with ammonia or organic primary, secondary, or tertiary amines. Cations of therapeutically acceptable salts include lithium, sodium, potassium, calcium, magnesium, and aluminum, as well as non-toxic quaternary ammonium cations, such as ammonium, tetramethylammonium, tetraethylammonium, methylamine, dimethylamine, trimethylamine, triethylamine, diethylamine, ethylamine, tributylamine, pyridine, N,N-dimethylaniline, N-methylpiperidine, N-methylmorpholine, dicyclohexylamine, procaine, dibenzylamine, N,N-dibenzylphenethylamine, 1-diphenylhydroxymethylamine, and N,N'-dibenzylethylenediamine. Other representative organic amines suitable for forming base addition salts include ethylenediamine, ethanolamine, diethanolamine, piperidine, and piperazine.

While it is possible to administer the compounds of the present application in the form of crude chemicals, it is also possible to provide them as pharmaceutical formulations. Accordingly, provided herein are pharmaceutical formulations containing one or more of compounds disclosed herein, or one or more pharmaceutically acceptable salts, esters, prodrugs, amides, or solvates thereof, together with one or more pharmaceutically acceptable carriers and optionally one or more other therapeutic ingredients. A carrier is "acceptable" in the sense of being compatible with other ingredients in a formulation and not deleterious to recipients thereof. Appropriate formulations depend on the chosen administration route. Any known techniques, carriers, and excipients that are suitable and understood in the art may be used, for example, as described in Remington's Pharmaceutical Sciences. The pharmaceutical compositions disclosed herein may be produced in any manner known in the art, for example, by means of conventional mixing, dissolving, granulating, sugar-coating, fine grinding, emulsifying, capsule encapsulating, entrapping, or compression processes, and may also be formulated as drug-device combinations.

Formulations include those suitable for oral administration, parenteral (including subcutaneous, intradermal, intramuscular, intravenous, intra-articular, and intra-medullary) administration, intraperitoneal administration, transmucosal administration, transdermal administration, rectal administration, and topical (including dermal, buccal, sublingual, ocular, intranasal, and intraocular) administration. The most suitable route may depend, for example, on condition and disease of the recipient. The formulations may be conveniently presented in unit dosage form and may be prepared by any methods well known in the field of medicine. Typically, such method includes a step of combining the compounds of the present application, or pharmaceutically acceptable salts, esters, amides, prodrugs, or solvate thereof ("active ingredients"), with carriers which constitute one or more auxiliary ingredients. In general, the formulations are prepared by uniformly and closely combining the active ingredients with liquid carriers or finely-divided solid carriers, or both, and then, if necessary, shaping products into the desired formulations.

The formulations of the compounds disclosed herein suitable for oral administration may be presented: as discrete units, such as capsules, cachets, or tablets, each containing a predetermined amount of active ingredients; as powders or granules; as solutions or suspensions in aqueous or non-aqueous liquids; or as oil-in-water or water-in-oil emulsions. The active ingredients may alternatively be presented as boluses, electuaries, or pastes.

Pharmaceutical formulations available for oral use include tablets, push-fit capsules made of gelatin, and sealed soft capsules made of gelatin and plasticizers (for example, glycerol or sorbitol). Tablets may be prepared by compression or molding optionally with one or more auxiliary ingredients. Compressed tablets may be prepared by compressing active ingredients in a free-flowing form (for example, powders or granules) optionally mixed with a binder, inert diluent, lubricant, surfactant, or dispersing agent in a suitable machine. Molded tablets may be prepared by molding a mixture of powdered compounds moistened with an inert liquid diluent in a suitable machine. Tablets may be optionally coated or scored and may be formulated to provide sustained or controlled release of the active ingredients therein. The dosages of all formulations for oral administration should be suited to the intended purpose of myopia prevention or treatment achievable via this route. Push-fit capsules may contain active ingredients mixed with a filler (for example, lactose), a binder (for example, starch) and/or a lubricant (for example, talc or magnesium stearate), and optionally a stabilizer. In soft capsules, active compounds may be dissolved or suspended in a suitable liquid (for example, fatty oil, liquid paraffin, or liquid polyethylene glycol). Furthermore, stabilizers may be added. Sugar-coated pill cores with suitable coatings may be provided. To this end, concentrated sugar solutions may be used, which may optionally contain acacia, talc, polyvinylpyrrolidone, carbomer gel, polyethylene glycol, and/or titanium dioxide, lacquer solutions, and suitable organic solvents or solvent mixtures. Dyes or pigments may be added to tablets or sugar-coatings for identification or to characterize different combinations of active compound dosages.

Examples of fillers or diluents for oral pharmaceutical formulations (such as capsules and tablets) include, but are not limited to, lactose, mannitol, xylitol, dextrose, sucrose, sorbitol, compressible sugars, microcrystalline cellulose (MCC), powdered cellulose, corn starch, pregelatinized starch, dextrates, dextran, dextrin, dextrose, maltodextrin, calcium carbonate, calcium hydrogen phosphate, tricalcium phosphate, calcium sulfate, magnesium carbonate, magnesium oxide, poloxamers (for example, polyethylene oxide), methylcellulose, and hydroxypropyl methylcellulose. The fillers may have complexed solvent molecules, as in the case where the lactose used is lactose monohydrate.

Examples of disintegrants for oral pharmaceutical formulations (such as capsules and tablets) include, but are not limited to, sodium starch glycollate, sodium carboxymethylcellulose, calcium carboxymethyl cellulose, cross-linked sodium carboxymethylcellulose, povidone, crospovidone (polyvinylpolypyrrolidone), methylcellulose, microcrystalline cellulose, powdered cellulose, low-substituted hydroxypropyl cellulose, starch, pregelatinized starch, and sodium alginate.

Furthermore, glidants and lubricants may be used in the oral pharmaceutical formulations to ensure uniform blending of excipients during mixing. Examples of lubricants include, but are not limited to, calcium stearate, glyceryl monostearate, glyceryl palmitostearate, hydrogenated vegetable oil, light mineral oil, magnesium stearate, mineral oil, polyethylene glycol, sodium benzoate, sodium lauryl sulfate, sodium stearyl fumarate, stearic acid, talc, and zinc stearate. Examples of glidants include, but are not limited to, silica (SiO₂), talc, corn starch, and poloxamers. Poloxamers (or those available from BASF Corporation) are A-B-A block copolymers, where the block A is a hydrophilic polyethylene glycol homopolymer and the block B is a hydrophobic polypropylene glycol homopolymer.

Examples of tablet binders include, but are not limited to, acacia, alginic acid, carbomer, sodium carboxymethylcellulose, dextrin, ethylcellulose, gelatin, guar gum, hydrogenated vegetable oil, hydroxyethyl cellulose, hydroxypropyl cellulose, hydroxypropyl methylcellulose, copolyvidone, methylcellulose, liquid glucose, maltodextrin, polymethacrylates, povidone, pregelatinized starch, sodium alginate, starch, sucrose, tragacanth gum, and zein.

Compounds may be formulated for parenteral administration by injection, for example, by bolus injection or continuous infusion. Formulations for injection may be provided in unit dosage form, for example, in ampoules or in multi-dose containers to which preservatives are added. Compositions may take such forms as suspensions, solutions, or emulsions in oily or aqueous vehicles, and may contain formulating agents such as suspending agents, stabilizers, and/or dispersing agents. Formulations may be provided in unit-dose or multi-dose containers, such as sealed ampoules and vials, and may be stored in a powder form or in a freeze-dried (lyophilized) condition requiring only addition of sterile liquid carriers, for example, normal saline or sterile pyrogen-free water, immediately prior to use. Ready-to-use injectable solutions and suspensions may be prepared from sterile powders, granules, and tablets of the types previously described. In a preferred embodiment, the pharmaceutical compositions of the present application are in the form of an injection, particularly a syringe. Preferably, the pharmaceutical compositions are administrated by intraocular injection, and more preferably by intravitreal injection into the vitreous body.

Formulations for parenteral administration include, but are not limited to: aqueous and non-aqueous (oily) sterile injectable solutions of active compounds, which may contain antioxidants, buffers, bacteriostats, and solutes that render the formulations isoosmotic with the blood of the intended recipient; and aqueous and non-aqueous sterile suspensions, which may include suspending agents and thickeners. Suitable lipophilic solvents or vehicles include fatty oils (for example, sesame oil) or synthetic fatty acid esters (such as ethyl oleate or triglycerides) or liposomes. Aqueous injectable suspensions may contain substances that increase the viscosities of the suspensions, such as methylcellulose, sodium carboxymethylcellulose, sorbitol, or dextran. Optionally, these substances are, for example, ethyl oleate, or triglycerides, or liposomes. Aqueous injectable suspensions may contain substances that increase the viscosities of the suspensions, such as sodium carboxymethylcellulose, sorbitol, or dextran. Optionally, the suspensions may also contain suitable stabilizers or reagents that increase solubility of a compound to allow for preparation of solutions at high concentrations.

In addition to the aforementioned formulations, the compounds mentioned herein, such as lipoic acid, may also be formulated as stored formulations. Such long-acting formulations may be administrated by implantation (for example, subcutaneous or intramuscular) or by intramuscular injection. Thus, for example, compounds may be formulated with suitable polymeric or hydrophobic materials (for example, as emulsions in acceptable oils) or ion-exchange resins, or formulated as slightly soluble derivatives, for example, slightly soluble salts.

For buccal or sublingual administration, compositions may take the form of tablets, lozenges, troches, or gels formulated conventionally. Such compositions may contain active ingredients, such as the compounds mentioned herein like lipoic acid, in flavored bases, for example, sucrose and acacia or tragacanth gum.

The compounds may also be formulated into rectal compositions, such as suppositories or retention enemas, containing, for example, conventional suppository bases such as cocoa butter, polyethylene glycol, or other glycerides.

In addition to the aforementioned formulations, the compounds mentioned herein, such as lipoic acid, may also be formulated as single-use formulations, devices, articles for altering ocular appearance, or medical consumables, for topical administration, such as single-use eye drops (each package contains only one therapeutically effective dose), individually packaged eye patches, cosmetic contact lenses, and daily disposable contact lenses.

The compounds disclosed herein, such as lipoic acid, may be administrated topically, i.e., non-systemically. This includes applying the compounds disclosed herein externally to the eyes, epidermis, or oral cavity, and instilling such compounds into ears, eyes, and nose, such that the compounds do not significantly enter the bloodstream. In contrast, systemic administration refers to oral, intravenous, intraperitoneal, and intramuscular administration.

Formulations suitable for topical administration include liquid or semi-liquid formulations (such as gels, liniments, lotions, creams, ointments, or pastes) that are suitable for penetrating the skin to reach the site of action, and formulations suitable for administration to eyes, ears, or nose, such as drops and sprays. An active ingredient for myopia treatment may account for, for example, 0.0000001% to 99% w/w (by weight) of the total content of a formulation. In certain embodiments, the active ingredient may account for up to more than 50% w/w. In other embodiments, it may account for lower than 1% w/w. In certain embodiments, the active ingredient may account for 0.01% w/w to 1% w/w. In other embodiments, it may account for 0.00001% to 0.001% w/w of the formulation; alternatively, the active ingredient for topical administration may account for, for example, 0.0000001% to 100% w/v (by weight to volume) of the formulation; or in certain embodiments, the active ingredient may account for up to more than 50% w/v. In other embodiments, it may account for lower than 1% w/v. In certain embodiments, the active ingredient may account for 0.01% w/v to 1% w/v. In other embodiments, it may account for 0.00001% to 0.001% w/v of the formulation.

The gel formulation form includes formulations formed by linking lipoic acid or prodrugs thereof or related compound thereof with a hydrogel. Preferably, the hydrogel in the hydrogel-linked lipoic acid or prodrug thereof is a biodegradable hydrogel.

The hydrogel contains at least one polymer, and preferably consists of at least one polymer. The polymer is preferably selected from poly(acrylic acid), poly(acrylates), poly(acrylamides), poly(alkoxy) polymers, poly(amides), poly(amidoamines), poly(amino acids), poly(anhydrides), poly(asparagine), poly(butyric acid), poly(caprolactone), poly(carbonates), poly(cyanoacrylates), poly(dimethylacrylamides), poly(esters), poly(ethylene), poly(ethylene glycol), poly(ethylene oxide), poly(ethyloxazoline), poly(glycolic acid), poly(hydroxyethyl acrylate), poly(hydroxyethyloxazoline), poly(hydroxypropylmethacrylamide), poly(hydroxypropyl methacrylate), poly(hydroxypropyloxazoline), poly(iminocarbonates), poly(N-isopropylacrylamide), poly(lactic acid), poly(lactic-co-glycolic acid), poly(methacrylamides), poly(methacrylates), poly(methyloxazoline), poly(propylene fumarate), poly(organophosphorus cyanogen), poly(ortho-esters), poly(oxazolines), poly(propylene glycol), poly(siloxanes), poly(carbamates), poly(vinyl alcohol), poly(vinylamine), poly(vinyl methyl ether), poly(vinylpyrrolidone), siloxanes, ribonucleic acids, deoxyribonucleic acids, albumins, antibodies and fragments thereof, plasma proteins, collagen, elastin, fascin, fibrin, keratin, polyaspartic acid, polyglutamic acid, prolamin, transferrin, cytochromes, flavoprotein, glycoproteins, hemoproteins, lipoproteins, metalloproteins, phytochromes, phosphoproteins, opsins, agar, agarose, alginates, arabinan, arabinogalactan, carrageenan, cellulose, carboxymethyl cellulose, hydroxypropyl methylcellulose and other carbohydrate-based polymers, chitosan, dextran, dextrin, gelatin, hyaluronic acid and derivatives thereof, mannan, pectins, rhamnogalacturonan, starch, hydroxyalkylated starch, xylan, and copolymers and functionalized derivatives thereof.

Preferably, the hydrogel is a hydrogel based on biodegradable polyethylene glycol (PEG).

Preferably, the polyethylene glycol (PEG) is of a single molecular weight or is a mixture of different molecular weights in a specific ratio.

The hydrogel is a shaped article, preferably in the shape of microparticles. More preferably, the hydrogel is in the shape of microparticle bead. Even more preferably, the microparticle bead has a diameter of 1µm - 1000 µm, more preferably 5 µm - 500 µm, still more preferably 10 µm - 100 µm, and even more preferably 20 µm - 80 µm. The diameter of the bead is determined when the microparticle bead is suspended in an isoosmotic aqueous buffer. In a preferred embodiment, the hydrogel-linked lipoic acid or prodrug thereof is bead-shaped. More preferably, the hydrogel-linked lipoic acid or prodrug thereof is in the shape of microparticle bead. Even more preferably, the microparticle bead has a diameter of 1µm - 1000 µm, more preferably 5µm - 500 µm, still more preferably 10 µm - 100 µm, and even more preferably 20µm - 80 µm. The diameter of the bead is determined when the microparticle bead is suspended in an isoosmotic aqueous buffer. Such hydrogel may be polymerized in different ways, for example, by free-radical polymerization, ionic polymerization, or complex formation reactions.

If a hydrogel is processed via free-radical polymerization or ionic polymerization, at least two starting materials are a crosslinking macromonomer or crosslinking monomer (referred to as a crosslinking reagent) and a multifunctional macromonomer (referred to as a backbone reagent). The crosslinking reagent carries at least two interconnectable functional groups, while the backbone reagent carries at least one interconnectable functional group and at least one chemical functional group not involved in the polymerization step. Additional diluent monomers may or may not be present. Useful interconnectable functional groups include, but are not limited to, groups capable of free-radical polymerization, such as vinyl, vinyl-benzene, acrylates, acrylamides, methacrylates, and methacrylamides, and ionically polymerizable groups such as oxetane, azetidine, and ethylene oxide. In an alternative preparation method, the hydrogel is generated via a chemical complex formation reaction. In such reactions, the starting material includes at least one macromolecular starting material possessing complementary functionality for reactions such as condensation or addition reaction. In one embodiment, only one macromolecular starting material is used, which is a heteromultifunctional backbone reagent containing a lot of polymerizable functional groups that may be identical or different.

In addition to active ingredients such as lipoic acid, the topical ocular, otic, and nasal formulations of the present application may further contain excipients. Excipients commonly used in such formulations include, but are not limited to, isoosmotic agents, preservatives, chelating agents, buffers, and surfactants. Other excipients include solubilizers, stabilizers, comfort enhancers, polymers, demulcents, pH regulators, and/or lubricants. Any one of various excipients can be used in the formulations of the present application, including water, mixtures of water and water-miscible solvents (for example, C1-C7 alkanols), vegetable oils or mineral oils containing 0.5% to 5% of non-toxic water-soluble polymers, natural products (for example, alginates, pectins, tragacanth gum, karaya gum, guar gum, xanthan gum, carrageenan, agar, and acacia), starch derivatives (such as starch acetate and hydroxypropyl starch), and other synthetic products (such as polyvinyl alcohol, polyvinylpyrrolidone, polyvinyl methyl ether, polyethylene oxide, preferably cross-linked polyacrylic acid, and mixtures of these products). The concentrations of the excipients are typically 1 to 100,000 times the concentrations of the active ingredients. In a preferred embodiment, the excipients to be included in formulations are generally selected based on inertness thereof with respect to the active ingredient composition of the formulations.

For ocular, otic, and nasal formulations, suitable isoosmotic adjusting agents include, but are not limited to, mannitol, dextrose, sodium chloride, glycerol, sorbitol, and the like. Suitable buffers include, but are not limited to, phosphates, citrates, borates, acetates, and the like. Suitable surfactants include, but are not limited to, ionic and non-ionic surfactants (although non-ionic surfactants are preferred), such as polysorbate 80, RLM 100, POE 20 cetylstearyl ethers (for example, CS20), and poloxamers (for example, F68). Formulations may contain substances that increase viscosity of a solution or suspension, such as sodium carboxymethylcellulose, hydroxypropyl methylcellulose, microcrystalline cellulose, sorbitol, or dextran. Optionally, the formulations may further contain suitable stabilizers or reagents that increase solubility of a compound to enable preparation of solutions at high concentrations. These stabilizers or reagents include, but are not limited to, ethanol, benzyl alcohol, polyethylene glycol, phenethyl alcohol, and glycerol.

The formulation provided herein may contain one or more preservatives. Examples of such preservatives include benzalkonium chloride, parabens, sodium perborate, sodium chlorite, alcohols (for example, chlorobutanol, benzyl alcohol, or phenethyl alcohol), guanidine derivatives (for example, polyhexamethylene biguanide), sodium perborate, polyquaternium-1, amino alcohols (for example, AMP-95), or sorbic acid. In certain embodiments, the formulation may also be a preservative, thereby obviating the need for a preservative. In certain embodiments, although the formulation is not a preservative, a packaging material or a packaging design may be employed to prevent drug degradation (for example, changes in physicochemical properties and/or biological effects), thereby obviating the need for a preservative.

For ocular, otic, or nasal administration, the formulations may be solutions, suspensions, or gels. In a preferred aspect, formulations in aqueous solutions or suspensions for topical administration to the eyes or ears are in the form of drops. Formulations in aqueous solutions or suspensions for topical administration to the nose are in the form of drops, spray, or aerosol. The term "aqueous" generally denotes an aqueous formulation containing >20%, >50%, more preferably >75%, and particularly >90% water by weight. Such drops may be delivered from single-dose eye drop bottles, which are preferably sterile, thereby obviating the need for a bacteriostatic component in the formulation. Alternatively, drops may be delivered from a multi-dose eye drop bottle, which preferably includes a device for withdrawing any preservative from a formulation upon delivery. Such a device is known in the art. A solution formulation and a suspension formulation may be administrated nasally using a nebulizer. Intranasal delivery of solutions, suspensions, or dry powders may also be facilitated by propellant-based aerosol systems, where the propellant includes, but is not limited to, a hydrofluoroalkane-based propellant. Alternatively, an active pharmaceutical ingredient may be delivered in the form of a dry powder.

For ocular diseases, the components of the present application may be delivered to the eyes as concentrated gels or similar vehicles, or as soluble inserts placed under the eyelids.

In a specific embodiment, the formulation of the present application is administrated once a day. However, the formulation may also be prepared for administration at any frequency, including once a week, once every 5 days, once every 3 days, once every 2 days, twice a day, three times a day, four times a day, five times a day, six times a day, eight times a day, hourly, or at any higher frequency. Depending on the therapeutic regimen, the administration frequency is also maintained for varying durations. The duration of a specific therapeutic regimen may range from a single administration to regimens that extend over several months or years. The formulation is administrated in different doses, but a typical dose is one to two drops per administration, or an equivalent amount of gel or other formulations (such as tablets and eye ointment). A person of ordinary skill in the art is familiar with determining a therapeutic regimen for a specific indication.

Gels for topical or transdermal administration may generally include mixtures of volatile solvents, non-volatile solvents, and water. In certain embodiments, a volatile solvent component of a buffered solvent system may include lower (C1-C6) alkyl alcohols, lower alkyl diols, and lower diol polymers. In a further embodiment, the volatile solvent is ethanol. The volatile solvent component is considered to act as a penetration enhancer while also producing a cooling effect on the skin as it evaporates. The non-volatile solvent portion of the buffered solvent system is selected from lower alkylene diols and lower diol polymers. In certain embodiments, propylene glycol is used. The non-volatile solvent retards evaporation of the volatile solvent and reduces vapor pressure of the buffered solvent system. The amount of the non-volatile solvent component, like that of the volatile solvent, is determined by the pharmaceutical compound or the drug used. When there is too little non-volatile solvent in the system, the pharmaceutical compound may crystallize due to evaporation of the volatile solvent, whereas an excess may lead to low bioavailability due to inadequate release of the drug from the solvent mixture. The buffer component of the buffered solvent system may be selected from any buffers commonly used in the art, and in certain embodiments, water is used. A common ingredient ratio is about 20% of the non-volatile solvent, about 40% of the volatile solvent, and about 40% of water. Several optional ingredients may be added to a topical composition. These optional ingredients include, but are not limited to, chelating agents and gelling agents. Suitable gelling agents may include, but are not limited to, semi-synthetic cellulose derivatives (for example, hydroxypropyl methylcellulose), synthetic polymers, galactomannan polymers (such as guar gum and derivatives thereof), and cosmetic agents.

Lotions include those suitable for application to the skin or the eyes. An ocular lotion may comprise a sterile aqueous solution that optionally contains bactericides, and may be prepared by a method similar to that used for preparing drops. Lotions or liniments for application to the skin may also include reagents to accelerate drying and cooling the skin (such as alcohols or acetone) and/or humectants (for example, glycerol) or oils (for example, castor oil or peanut oil).

The pharmaceutical compositions of the present application may be creams, ointments (e.g., 3% unguent, or ointment), or pastes, which are semi-solid formulations of active ingredients intended for external application. They may be prepared by mixing active ingredients in a finely divided or powdered form, either alone or in aqueous or non-aqueous fluid solutions or suspensions, with a greasy or non-greasy bases using a suitable machine. The base may include: hydrocarbons, such as vaseline, hard, soft, or liquid paraffin, glycerol, beeswax, and metallic soaps; mucilage; naturally derived oils, such as fish oil, almond oil, corn oil, peanut oil, castor oil, or olive oil; lanolin or derivatives thereof; or fatty acids (for example, stearic acid or oleic acid), along with alcohols (for example, propylene glycol), or macrogel, or lanosterol, or dihydrolanosterol. The formulations may be blended with any suitable surfactant, such as anionic, cationic, or non-ionic surfactants like sorbitan esters or polyoxyethylene derivatives thereof. Suspending agents may also be included, such as natural gums, cellulose derivatives, or inorganic materials like silicaceous silica, and other ingredients, such as lanolin or lanosterol.

Drops or sprays may include sterile aqueous or oily solutions or suspensions and can be prepared by dissolving active ingredients in aqueous solutions containing suitable bactericides and/or fungicides and/or any other suitable preservatives, and in certain embodiments, surfactants. The resulting solutions may then be clarified by filtration, transferred to suitable containers, which are then sealed and sterilized by autoclaving or by maintaining at 98-100°C for half an hour. Alternatively, the solutions may be sterilized by filtration and transferred into the containers by an aseptic technique. Examples of bactericides and fungicides suitable for inclusion in drops include azole compounds (for example, econazole), phenylmercuric nitrate or phenylmercuric acetate (0.002%), benzalkonium chloride (0.01%), and chlorhexidine acetate (0.01%). Suitable solvents for preparing oily solutions include glycerol, diluted alcohols, and propylene glycol.

Formulations for topical administration in the mouth (for example, buccal or sublingual) include lozenges containing active ingredients in flavored bases (such as sucrose and acacia or tragacanth gum), and troches containing active ingredients in bases such as gelatin and glycerol, or sucrose and acacia.

For administration by inhalation, compounds mentioned herein, such as lipoic acid, may be conveniently delivered by an insufflator, nebulizer, pressurized pack, or other convenient devices for delivering aerosol sprays. The pressurized pack may contain a suitable propellant, such as hydrofluoroalkane, dichlorodifluoromethane, trichlorofluoromethane, dichlorotetrafluoroethane, carbon dioxide, or other suitable gas. In the case of a pressurized aerosol, a dosage unit may be determined by providing a valve to deliver a metered amount. Alternatively, for administration by inhalation or insufflation, the compounds according to the present application may take the form of a dry powder composition, for example, a powder mixture of a compound and a suitable powder base (for example, lactose or starch). The powder composition may be provided in a unit dosage form, for example, in a capsule, cartridge, gelatin, or blister package, from which the powder may be administrated by means of an inhaler or insufflator.

A preferred unit-dose formulation is one containing an effective dose of the active ingredients, as described herein, or an appropriate fraction thereof.

It should be understood that, in addition to the ingredients specifically mentioned above, the formulations described above may include other reagents conventional in the art for the respective types of formulations, such as microneedle formulations, or formulations suitable for oral or intranasal administration (which may include flavoring agents).

The compounds may be administrated orally or via injection at a dose ranging from 0.001 mg/kg per day to 200 mg/kg per day. The dose range for adults and the elderly is generally 5 mg per day to 1200 mg per day, while the dose for children or adolescents should be reduced or maintained. Tablets provided in discrete units or in other presentation forms may conveniently contain a certain amount of one or more compounds that are effective at such a dose or a plurality of such doses, for example, in units containing 200 mg to 1200 mg, typically about 300 mg to 600 mg, and preferably 400 mg.

The compounds mentioned herein, such as lipoic acid, may be administrated in various ways, for example, orally, topically, or by injection, or systemically and topically at the same time, or systemically and topically alternately. An exact amount of a compound administrated to a patient is determined by an attendant physician. A specific dosage level for any particular patient may depend on a variety of factors, including activity of the specific compound used, age, body weight, general health, gender, diet, administration time, administration route, rate of excretion, drug combination, the specific disease being treated, and severity of the indication or condition being treated. Furthermore, the administration route may vary depending on the condition and severity of the condition.

In certain circumstances, it may be appropriate to administer at least one of the compounds described herein (or a pharmaceutically acceptable salt, ester, or prodrug thereof) in combination with another therapeutic agent. By way of example only, if one of the side effects experienced by a patient receiving a compound described herein is hypotension, it may be appropriate to administer an anti-hypotensive agent in combination with an initial therapeutic agent. Alternatively, by way of example only, efficacy of one of the compounds described herein may be enhanced by administering an anti-allergic agent (for example, the anti-allergic agent itself may merely have a minimal therapeutic benefit, but an enhanced therapeutic benefit may be obtained when the anti-allergic agent is used combined with a therapeutic agent mentioned herein). Alternatively, by way of example only, the benefit experienced by a patient may be enhanced by administering one of the compounds described herein with another therapeutic agent (which also includes a therapeutic regimen) that also possesses a therapeutic benefit. By way of example only, in myopia treatment involving administration of one of the compounds described herein, the therapeutic benefit may be enhanced by further providing the patient with another myopia treatment agent. In any case, regardless of the disease, disorder, or condition being treated, the overall benefit experienced by the patient may simply be additive of two therapeutic agents, or the patient may experience a synergistic benefit.

Pharmaceutical compositions, in either dry or liquid form, may be provided as single-dose or multi-dose pharmaceutical compositions.

In one embodiment of the present application, a liquid or dry pharmaceutical composition is provided as a single dose, which means that a container in which it is provided contains one pharmaceutical dose. Alternatively, the liquid or dry pharmaceutical composition is a multi-dose pharmaceutical composition, which means that the container in which it is provided contains more than one therapeutic dose. That is, the multi-dose composition contains at least 2 doses. Such a multi-dose composition may be used for different patients in need thereof, or may be used for a single patient, where the remaining doses are stored after application of the first dose until needed.

In another aspect of the present application, the pharmaceutical composition is contained within a container. Containers for liquid or dry pharmaceutical compositions include, for example, eye drop bottles, syringes, vials, vials with stoppers and seals, ampoules, and cartridges. Particularly, the liquid or dry pharmaceutical compositions are provided in syringes. If a pharmaceutical composition is a dry pharmaceutical composition, the container is preferably a dual-chamber syringe. In this embodiment, the dry pharmaceutical composition is provided in a first chamber of the dual-chamber syringe, and a reconstitution solution is provided in a second chamber of the dual-chamber syringe.

The dry composition is reconstituted before being applied to a patient in need thereof. Reconstitution may be carried out in the container in which the dry composition is provided, such as in eye drop bottles, syringes, dual-chamber syringes, ampoules, and cartridges. Reconstitution is performed by adding a predetermined amount of a reconstitution solution to the dry composition. The reconstitution solution is a sterile liquid, such as water or a buffer, which may contain other additives like preservatives and/or antimicrobial agents, such as benzyl alcohol and cresol. Preferably, the reconstitution solution is sterile water. When the dry composition is reconstituted, it is referred to as a "reconstituted pharmaceutical composition", or a "reconstituted pharmaceutical composition ", or a "reconstituted composition".

Ocular formulations: The pharmaceutical compositions of the present application may be administrated in the form of ocular formulations. The ocular formulations of the present application include ophthalmically acceptable carriers.

The amount of an active ingredient that may be combined with a carrier material to produce a single dosage form may vary depending on the host to be treated and the specific administration route.

As used herein, an "ophthalmically acceptable carrier" refers to an ophthalmically acceptable solvent, suspending agent, or vehicle for delivering a pharmaceutical composition to the eyes of a subject. The carrier may be solid or liquid. The carrier is "ophthalmically acceptable" in the sense that it is suitable for administration to the eyes without causing any significant adverse reactions.

Typically, an ophthalmically acceptable carrier is or includes water. Typically, the ocular formulation is in the form of eye drops or a gel for application to the eyes. Typically, the majority of the formulation is water. Typically, the formulation includes greater than 50% by weight (for example, greater than 60% by weight, 65% by weight, 70% by weight, 75% by weight, 80% by weight, 85% by weight, or 90% by weight), more typically greater than 95% by weight (for example, 96% by weight, 97% by weight, 98% by weight, or 99% by weight) of water.

In some embodiments, the ophthalmically acceptable carrier is an oil-in-water emulsion or an oil. In such embodiments, the ocular formulation may be in the form of a cream for application to the eyes. In such embodiments, the formulation may contain greater than 10% by weight, more typically greater than 20% by weight, of an oily ingredient.

In other embodiments, the carrier may be a biodegradable polymer, for example, biodegradable polymeric ocular implants for sustained release of the compounds of the present application and optionally other compounds. For instance, the carrier is a biodegradable, biocompatible polymer base. In one embodiment, a compound may be embedded within a polymer base while maintaining structural integrity. The polymer may be natural, such as a polypeptide, protein, or polysaccharide, or may be synthetic, such as a poly(alpha-hydroxy acid). Examples include carriers made from, for example, collagen, fibronectin, elastin, cellulose acetate, cellulose nitrate, methyl cellulose, polysaccharides, fibrin, gelatin, and a combination thereof. In one embodiment, the polymer is polylactic acid (PLA) or poly(lactic-co-glycolic acid) (PLGA). The polymer base may be prepared and separated in various forms and sizes, including microspheres and nanospheres.

In some embodiments, a therapeutic compound is prepared with a carrier that protects the therapeutic compound against rapid elimination from the body, where the carrier is, for example, a release control agent, including implants and microencapsulated delivery systems. Biodegradable, biocompatible polymers may be used, such as ethylene vinyl acetate, polyanhydrides, polyglycolic acid, collagen, polyorthoesters, and polylactic acid. Such formulations may be prepared using a known technique.

Excipients: Excipients suitable for the ocular formulations of the present application include, for example, demulcents, softeners, hypertonic agents, preservatives, buffers, or pH regulators. Examples of suitable excipients include:
A. Demulcents: synthetic cross-linked acrylic polymers with high molecular weights (such as Carbomer 974 and Carbomer 980); cellulose derivatives (such as hydroxypropyl methylcellulose ("HPMC"), hydroxyethyl cellulose, methyl cellulose, carboxymethyl cellulose, or sodium carboxymethylcellulose (carmethose); dextran (for example, Dextran 70); gelatin; polyols (such as glycerin, polyethylene glycol 300, polyethylene glycol 400, polysorbate 80, and propylene glycol); polyvinyl alcohol; povidone (polyvinylpyrrolidone); poloxamers; and hyaluronic acid (a disaccharide polymer) or a sodium or potassium salt thereof.
B. Softeners: lanolin (for example, anhydrous lanolin); oily ingredients (such as light mineral oil, mineral oil, paraffin, vaseline, liquid paraffin, white ointment, white vaseline, white wax, and yellow wax); and fish oil and castor oil.
C. Preservatives: benzalkonium chloride; sodium perborate; Oxyd (0.05% sodium chlorite, and 0.01% hydrogen peroxide); polyquaternium-1 (polymer of ethanol, 2,2',2"-nitrilotris-, 1,4-dichloro-2-butene, and N,N,N',N'-tetramethyl-2-butene-1,4-diamine); sodium silver chloride; polyhexamethylene biguanide; oxyborates; and (0.005% m/v sodium chlorite).
D. Ocular hypertonic agent: sodium chloride.
E. The ocular formulations of the present application may further contain preservatives that inhibit microbial growth and extend the shelf life of the formulations. Preservatives that may be used in the ocular formulations of the present application include, for example, benzalkonium chloride, sodium perborate, Oxyd (0.05% sodium chlorite, and 0.01% hydrogen peroxide), polyquaternium-1 (polymer of ethanol, 2,2',2"-nitrilotris-, 1,4-dichloro-2-butene, and N,N,N',N'-tetramethyl-2-butene-1,4-diamine), sodium silver chloride, polyhexamethylene biguanide, and oxyborates. Sodium chlorite (0.005% m/v) is a microbicide with broad-spectrum antimicrobial activity and very low toxicity to mammalian cells, which protects the formulations during storage but ultimately dissociates into water, sodium ions, chloride ions, and oxygen after exposure. Because such preservatives are also found in natural tears, the risk of preservative-induced ocular irritation and corneal injury are minimized. Sodium chlorite is safe and effective for use for the long run. This preservative has no side effects on epithelial cells in vitro or in vivo and is less disruptive to cellular integrity than many other preservatives in use.

The ocular formulations of the present application may be prepared by any suitable method for preparing ocular formulations. Ocular formulations are typically sterile, and therefore the methods may include a step of sterilizing the ocular formulations. Preferably, the ocular formulations are transparent and exhibit a reflectivity similar to that of tears, with a suitable pH (typically buffered to a pH of about 7.5) to avoid serious corneal irritation and prevent microorganisms. The formulations of the present application suitable for topical administration to the eyes are preferably isoosmotic or slightly hypotonic to counteract any tear hypertonicity caused by evaporation and/or diseases. This may require a tonicity agent to adjust the osmolality of the formulation to a level at or near 210 milliosmoles per kilogram (mOsm/kg) to 320 mOsm/kg. The formulations of the present application generally have an osmolality in the range of 220 mOsm/kg to 320 mOsm/kg, preferably in the range of 235 mOsm/kg to 300 mOsm/kg. Surface tension values near or below the observed surface tension value of the tear film are generally preferred. The ocular formulations are generally formulated as sterile aqueous solutions.

In certain embodiments involving ocular use, the compositions of the present application are formulated together with one or more tear substitutes. Various tear substitutes are known in the art, including but not limited to: monomeric polyols, such as glycerol, propylene glycol, and ethylene glycol; polymeric polyols, such as polyethylene glycol; cellulose esters, such as hydroxypropyl methylcellulose, sodium carboxymethylcellulose, and hydroxypropyl cellulose; dextrans, such as dextran 70; vinyl polymers, such as polyvinyl alcohol; and carbomers, such as Carbomer 934P, Carbomer 941, Carbomer 940, and Carbomer 974P. The formulations or pharmaceutical compositions of the present application may be used in conjunction with contact lenses, corneal contact lenses, or other ophthalmic products (such as framed glasses and myopia treatment instruments).

Preferred formulations are prepared using a buffer system that maintains the pH of the formulations between about 4.5 and about 8. The most preferred formulations have a pH of 5.5 to 7.5. Optional buffers are physiologically tolerant buffers that maintain the pH within the desired range, such as sodium phosphate, bicarbonates, succinates, histidine, citrates, acetates, sulfates, nitrates, chlorides, and pyruvates. Antacids, for example, Mg(OH)₂ or ZnCO₃, may also be used. The buffer capacity may be adjusted to match conditions most sensitive to the pH stability.

The ocular formulations of the present application may further include algefacients, such as menthol, camphor, borneol, geraniol, eucalyptol, and linalool. When an algefacient is mixed, a concentration of the algefacient is preferably 0.0001W/V% to 0.1W/V%.

The ocular formulations of the present application may further contain other therapeutic components commonly used ophthalmically, such as decongestant ingredients (such as adrenaline, adrenaline hydrochloride, ephedrine hydrochloride, tetrahydrozoline hydrochloride, naphazoline hydrochloride, naphazoline nitrate, phenylephrine hydrochloride, and dl-methylephedrine hydrochloride), anti-inflammatory/astringent agents (such as neostigmine methylsulfate, ε-aminocaproic acid, allantoin, berberine hydrochloride hydrate, berberine sulfate hydrate, sodium azulenesulfonate, dipotassium glycyrrhizinate, zinc sulfate, zinc lactate, and lysozyme hydrochloride), antihistamines (such as diphenhydramine hydrochloride and chlorpheniramine maleate), water-soluble vitamins (sodium flavin adenine dinucleotide, cyanocobalamin, pyridoxol hydrochloride, panthenol, calcium pantothenate, and sodium pantothenate), amino acids (such as potassium L-aspartate, magnesium L-aspartate, potassium magnesium L-aspartate·(equimolar mixture), and aminoethanesulfonic acid), ingredients for relieving visual fatigue and/or dry eyes (such as taurine, vitamin A, hydroxypropyl methylcellulose, and vitamin E), polyunsaturated fatty acids (such as fish oil, omega-3 unsaturated fatty acid, DHA, and EPA), Piracetam, sulfonamide drugs, nicotinic acid, retinoic acid, as well as lutein, salidroside, formononetin, atropine, dibazol, and M-receptor blockers (for example, blockers or antagonists or inhibitors targeting M1 receptors).

Contents of active substances in the pharmaceutical compositions of the present application: During drug mixing, the concentration of a therapeutically active compound may be selected as an effective and suitable amount for each substance. Considering factors such as ocular irritation and formulation stability, the therapeutically active compound (for example, lipoic acid) in the pharmaceutical composition may be present at a high, medium, or low concentration. For example, it may account for about 0.0000001-100%, about 0.00001-10%, about 0.001-20%, about 0.1-30%, about 1-40%, about 10-50%, about 20-60%, about 30-70%, about 40-80%, or about 50-90% (W/V) of the total amount of the pharmaceutical composition, such as about 1 µM, about 0.1 µM, about 0.01 µM, about 5 µM, about 10 µM, about 15 µM, about 25 µM, and about 50 µM, and/or it may account for at least about 0.0000001%, about 0.000001%, about 0.00001%, about 0.0001%, about 0.001%, about 0.01%, about 0.05%, about 0.1%, about 0.15%, about 1%, or about 10% (W/V) of the total amount of the pharmaceutical composition.

Delivery of the ocular formulations: the ocular formulations of the present application may be delivered to patients in the form of eye drops (in the form of single-dose or multi-dose droppers), ointments, gels, creams, or biodegradable polymeric ocular implants (designed for sustained release), or via eye moisturization (for example, multi-dose sprays).

Packaging of the ocular formulations should be related to the preservative-free or preserved nature of solutions. Packaging methods, such as form-fill-seal technology which integrates blow molding, sterile filling, and sealing into a single process, are particularly suitable for packaging preservative-free formulations in unit-dose containers. Typically, these single-dose containers are composed of low-density polyethylene or polypropylene and include a twist-off cap.

The formulations and methods of the present application are intended for use in any subject that may benefit from the formulations and methods of the present application. The subject is typically a mammal, and more typically a human. However, the present application is not limited to treating a human and may be applied to veterinary uses. Therefore, in accordance with the present application, the terms "subject", "patient" or "subject in need thereof" include humans as well as non-human animals, including, for example, farm animals such as sheep, pigs, cattle, and horses; companion animals such as dogs and cats; and laboratory animals such as mice, rats, and rabbits. In a preferred embodiment, the mammal is a human.

In another aspect, lipoic acid or lipoic acid choline ester, or optical isomers or racemates thereof, or solvates thereof, or pharmaceutically acceptable salts or esters thereof, or prodrugs thereof, or metabolites thereof, or related compounds or extracts thereof, or crystalline compounds thereof, or combinations of the foregoing in the present application are used for treatment of myopia and related symptoms thereof, or myopia correction, or for delaying myopia progression. These are mainly achieved by inhibiting axial elongation and increase in vitreous chamber depth and/or inhibiting decrease in choroidal thickness in individuals with myopia or a tendency to develop myopia.

In another aspect, the present application provides an ophthalmic device containing a pharmaceutical composition. The pharmaceutical composition contains lipoic acid or lipoic acid choline ester, or a therapeutically acceptable salt or derivative thereof, or a combination thereof. Preferably, the ophthalmic device delivers the pharmaceutical composition in a sustained release manner.

In certain embodiments of the pharmaceutical compositions, ophthalmic devices, or treatment methods disclosed herein, the pharmaceutical compositions are formulated as ophthalmic compositions, for example, formulated as ophthalmic compositions for treating ophthalmic diseases or conditions.

In certain embodiments of the pharmaceutical compositions, ophthalmic devices, or treatment methods disclosed herein, the pharmaceutical compositions are substantially uniformly distributed throughout the ophthalmic devices.

In certain embodiments of the pharmaceutical compositions, ophthalmic devices, or treatment methods disclosed herein, the pharmaceutical compositions are administrated to eyes of the patient; or the pharmaceutical compositions are administrated topically; or the pharmaceutical compositions are administrated to eyes in the form of eye drop formulations, eye spray formulations, or eye gel formulations; or the pharmaceutical compositions are administrated to eyes in the form of ophthalmic emulsions, ophthalmic liposomes, nanodiscs, nanoparticle suspensions, or ophthalmic ointments. The pharmaceutical compositions are administrated ophthalmically to eyes of the patient.

In certain embodiments of the pharmaceutical compositions, ophthalmic devices, or treatment methods disclosed herein, the pharmaceutical compositions are administrated once, twice, 3 times, 4 times, or 5 times per day.

In certain embodiments, the pharmaceutical compositions as disclosed herein may be ophthalmic formulations or aqueous compositions, such as ophthalmic aqueous formulations, for example, in the form of eye drops. For example, the ophthalmic aqueous formulations as described herein may be packaged in eye drop bottles and administrated as drops. In certain embodiments, the ophthalmic aqueous formulations may be administrated once (i.e., as a single dose), which may include one, two, three, or more drops instilled into eyes of the patient. In certain embodiments, one dose of the ophthalmic aqueous formulation described herein is one drop of the aqueous composition from the eye drop bottle.

In certain embodiments, the pharmaceutical compositions as disclosed herein may be ophthalmic gel formulations. For example, the ophthalmic gel formulations may be packaged in eye drop bottles and administrated as drops. In certain embodiments, the ophthalmic gel formulations may be administrated as a single application (i.e., a single dose), which may include one, two, three, or more drops instilled into eyes of the patient. In certain embodiments, one dose of the ophthalmic gel described herein is one drop of the gel composition from the eye drop bottle.

In certain embodiments, the pharmaceutical composition as disclosed herein may be an ophthalmic ointment formulation. For example, the ophthalmic ointment formulation may be packaged in a tube or another squeezable container that has a dispensing orifice through which a strip of ointment is delivered. In certain embodiments, the ophthalmic ointment formulation may be administrated once (i.e., as a single dose), which may include one or more strips applied into eyes of the patient. In certain embodiments, one dose of the ophthalmic ointment is one strip of ointment composition dispensed through the orifice of the dispensing tube.

Treating a subject with a therapeutically effective amount of a therapeutic composition or formulation described herein may include a single treatment or a series of treatments.

In certain embodiments of the pharmaceutical compositions, ophthalmic devices, or treatment methods disclosed herein, the methods are intended to prevent, control, alleviate, retard, or reduce development of myopia in the treated patient.

In certain embodiments of the pharmaceutical compositions, ophthalmic devices, or treatment methods disclosed herein, the methods are intended to control, retard, reduce, delay, and/or alleviate development of myopia in a treated patient within the following ranges relative to an untreated patient: between about 5% and 95%, between about 5% and 90%, between about 5% and 80%, between about 5% and 70%, between about 5% and 60%, between about 5% and 50%, between about 5% and 40%, between about 5% and 30%, between about 5% and 20%, between about 10% and 100%, between about 20% and 90%, between about 30% and 90%, between about 40% and 90%, between about 50% and 90%, or between about 75% and 90%.

In certain embodiments of the pharmaceutical compositions, formulations, ophthalmic devices, or treatment methods disclosed herein, use of the pharmaceutical compositions, formulations, ophthalmic devices, or treatment methods limits magnitude of negative refraction in eyes of the subject to about 1.0 D - 6.0 D, 1.0 D - 5.0 D, 1.0 D - 4.0 D, 1.0 D - 3.0 D, 1.0 D - 2.0 D, lower than 6.0 D, lower than 5.0 D, lower than 4.0 D, lower than 3.0 D, lower than 2.0 D, and lower than 1.0 D.

In certain embodiments of the pharmaceutical compositions, ophthalmic devices, or treatment methods disclosed herein, the methods are intended to reverse development of myopia in the treated patient.

In certain embodiments of the pharmaceutical compositions, ophthalmic devices, or treatment methods disclosed herein, the methods are intended to prevent, control, retard, reduce, alleviate, delay, and/or reverse axial (or longitudinal) growth of eyes of the treated patient, i.e., axial elongation and/or increase in vitreous chamber length.

In certain embodiments of the pharmaceutical compositions, ophthalmic devices, or treatment methods disclosed herein, the pharmaceutical compositions, ophthalmic devices, or treatment methods are intended to control, retard, alleviate, reduce, delay, and/or reverse axial elongation and/or increase in vitreous chamber length in a treated patient (for example, with mild myopia, or myopia in children or adolescents) within the following ranges relative to an untreated patient: between about 5% and 95%, between about 5% and 90%, between about 5% and 80%, between about 5% and 70%, between about 5% and 60%, between about 5% and 50%, between about 5% and 40%, between about 5% and 30%, between about 5% and 20%, between about 10% and 100%, between about 20% and 90%, between about 30% and 90%, between about 40% and 90%, between about 50% and 90%, or between about 75% and 90%.

In certain embodiments of the drugs, formulations, compositions, ophthalmic devices, or treatment methods disclosed herein, the methods are intended to control, halt, retard, reduce, delay, and/or alleviate onset and development of myopia in patients diagnosed with myopia or at risk of developing myopia. The methods allow for increasing the choroidal thickness of the eyes of the patient (for example, a myopic eye, a pre-myopic eye, or an eye at risk of developing myopia) and/or reducing the axial (or longitudinal) growth rate of the eyes of the patient (for example, the myopic eye, the pre-myopic eye, or the eye at risk of developing myopia). That is, the axial elongation and/or the increase in vitreous chamber length may be inhibited.

In certain embodiments of the pharmaceutical compositions, ophthalmic devices, or treatment methods disclosed herein, the methods are intended to control, retard, reduce, delay, and/or alleviate axial (or longitudinal) growth of eyes of a treated patient by about 5% - 95%, about 5% - 90%, about 5% - 80%, about 5% - 70%, about 5% - 60%, about 5% - 50%, about 5% - 40%, about 5% - 30%, about 5% - 20%, about 10% - 100%, about 20% - 90%, about 30% - 90%, about 40% - 90%, about 50% - 90%, or about 75% - 90% relative to an untreated patient.

In certain embodiments of the pharmaceutical compositions, ophthalmic devices, or treatment methods disclosed herein, the pharmaceutical compositions, ophthalmic devices, or treatment methods are intended to prevent, control, retard, reduce, alleviate, change, delay, and/or reverse myopic refraction of eyes of the treated patient.

In certain embodiments of the pharmaceutical compositions, ophthalmic devices, or treatment methods disclosed herein, the pharmaceutical compositions, ophthalmic devices, or treatment methods result in lower severity in adverse reactions compared to atropine monotherapy.

In certain embodiments of the pharmaceutical compositions, ophthalmic devices, or treatment methods disclosed herein, the pharmaceutical compositions, ophthalmic devices, or treatment methods result in a smaller increase in pupil size or no effect on pupil size compared to atropine monotherapy.

In certain embodiments of the pharmaceutical compositions, ophthalmic devices, or treatment methods disclosed herein, the pharmaceutical compositions, ophthalmic devices, or treatment methods inhibit the axial elongation or the degree of refraction developing toward myopia compared to atropine monotherapy.

### Example 1: Preparation of Main Formulation or Pharmaceutical Composition

Form-deprivation and lens-induced myopia models in guinea pigs are classic and recognized myopic animal models in the art, and may be used for evaluating efficacy and safety of myopia treatment drugs, and methods for establishing such models are well-known to those skilled in the art. For animal modeling and administration routes in the present application, reference is made to previously published scientific documents by the research group (Sen Zhang, Invest Ophthalmol Vis Sci. 2019 Jul 1; 60(8):3074-3083.; Miaozhen Pan, Exp Eye Res. 2021 Jan; 202:108332.).

A lipoic acid preparation is a pharmaceutical composition formulated by the inventors using only lipoic acid as an effective pharmaceutical ingredient. Alpha-lipoic acid compound was purchased from MedChemExpress. Without adding other pharmaceutical excipients or other active ingredients, a ratio of components for preparing a 0.94% lipoic acid eye drop solution was as follows: DMSO (for dissolving a required mass of lipoic acid compound):PEG300:normal saline = 1:49:50 (by volume), where each 100 mL of the lipoic acid eye drop solution contained 0.94 g of alpha-lipoic acid. At room temperature, the lipoic acid formulation exhibited a clear, transparent, and homogeneous overall appearance, with no visible suspended substances to naked eyes.

R-alpha lipoic acid (R.ALA) was purchased from Suzhou Fushilai Pharmaceutical Co., Ltd. The inventors dissolved the R.ALA powder in DMSO to prepare a 100× mother solution. The mother solution was a yellow, clear, transparent, homogeneous, and stable liquid at normal temperature. Then, the mother solution was diluted according to the following ratio to prepare a working solution: specifically, mother solution:PEG300:Tween80:0.9% normal saline = 1:45:5:49. For example, if the final concentration of the R.ALA formulation used in the experiment was 0.4%, the mother solution was prepared to have a concentration of 40%.

S-alpha lipoic acid (S.ALA) was purchased from Suzhou Fushilai Pharmaceutical Co., Ltd. The S.ALA powder was dissolved in DMSO to prepare a 40% mother solution, and then a working solution was prepared by diluting the mother solution according to the ratio of mother solution:PEG300:Tween80:0.9% normal saline = 1:45:5:49. The final concentration of the S.ALA formulation used was 0.4%.

Dihydrolipoic acid (DHLA) was purchased from MedChemExpress. First, the DHLA powder was dissolved in DMSO to prepare a 40% mother solution, and then a working solution was prepared according to the ratio of mother solution:PEG300:Tween80:0.9% normal saline = 1:45:5:49. The final concentration of the DHLA formulation used in the experiment was 0.4%.

R-alpha-Lipoic acid choline ester (LACE) was purchased from Suzhou Fushilai Pharmaceutical Co., Ltd. The LACE powder was directly and completely dissolved in 0.9% normal saline to prepare lipoic acid choline ester solution eye drops with concentrations required by the experiment, for example, 0.5%.

Emoxypine succinate (ES) was purchased from MedChemExpress. It was completely dissolved in 0.9% normal saline to prepare a formulation with a concentration of 0.37%.

Pyridoxol hydrochloride (PH) was purchased from Selleck. It was completely dissolved in 0.9% normal saline to prepare a formulation with a concentration of 0.3%.

Astaxanthin (ASTA) was purchased from Selleck. The ASTA powder was dissolved in a mixed solvent, and the final ratio of solvents in the formulation was as follows: DMSO:PEG300:Tween80:0.9% normal saline = 1:45:5:49. The final concentration of the ASTA formulation used in the experiment was 0.87%.

Blueberry fruit extract (BFE) was purchased from Selleck. The BFE powder was dissolved in a mixed solvent, and the final ratio of solvents in the formulation was as follows: DMSO:PEG300:Tween80:0.9% normal saline = 1:45:5:49. The final concentration of the BFE formulation used in the experiment was 6%.

Atropine sulfate (Atropine) was purchased from MedChemExpress. It was completely dissolved in 0.9% normal saline to prepare a 2 mg/ml stock solution, which was then diluted to the concentration required by the experiment, for example, 0.05%.

All working solutions used in the examples (for example, formulations containing lipoic acid, or prodrugs thereof, or metabolites thereof) were aliquoted and stored in a refrigerator at -80°C. One tube was freshly taken out of the refrigerator for each experimental administration to avoid repeated freeze-thaw cycles. All preparation operations were carried out in a dark room.

Other formulations or pharmaceutical compositions described or used in the present application but not mentioned in this example were prepared and stored in accordance with common laboratory methods or standards well-known to those skilled in the art. During the preparation processes of all formulations and pharmaceutical compositions, conventional physical and chemical solubilization means such as heating, stirring, and pH regulation may be used as appropriate. No compound precipitation occurred during the administration of all formulations.

In the examples of the present application, healthy guinea pigs (i.e., without underlying diseases, such as hypertension, hyperglycemia, eye diseases, or abnormalities), and both male and female guinea pigs were used. The animal experiments in the present application have been reviewed and approved by the Experimental Animal Ethics Committee of Wenzhou Medical University.

### Example 2: Effectively Control of Progression of Myopia in a Form-Deprivation Model (FDM) in a Guinea Pig via Lipoic Acid

After individuals with significant eye diseases or abnormalities were eliminated from three-week-old tricolor guinea pigs, by detecting refraction (using an infrared eccentric photorefraction device) and the eye axis (using A-scan ultrasonography), animals with refractive power between 3 and 8 diopters (D), an interocular difference in refraction not exceeding 2 D, and interocular differences in vitreous chamber depth and axial length not exceeding 0.05 mm were selected. These animals were randomly divided into two groups: an FD + vehicle control group (1% DMSO + 49% PEG300 + 50% normal saline, by volume) and an FD + 0.94% lipoic acid drug treatment group. At 8:00 AM on day 1 of the experiment, form deprivation was induced in the guinea pigs using a mask method, where the right eye (experimental eye) was occluded and the left eye was left unoccluded (contralateral eye). The FD induction was maintained throughout the entire period of the lipoic acid efficacy experiment, with the hoods only briefly removed for drug administration or ocular examination (for example, refraction detection). Starting on the first day of the efficacy experiment, position of the hood was checked daily at 8:00 AM, 12:00 PM, and 7:00 PM, as well as prior to administration. Individuals with hoods fell off more than three times during the administration period were excluded. Beginning on the day of modeling, the corresponding vehicle or drug (lipoic acid formulation) was administrated to the experimental eye daily between 9:00 AM and 10:00 AM via periocular subconjunctival injection at an injection volume of 100 µL. The vehicle or drug was administrated once daily for two consecutive weeks. Interocular refraction and eye axis parameters of all subjects were measured at the start of the efficacy experiment, 1 week after administration, and after the final administration. All data collection and processing methods were the same as those previously published by the laboratory of the inventors (Pan M, Zhao F, Xie B, et al. Dietary omega-3 polyunsaturated fatty acids are protective for myopia. Proc Natl Acad Sci U S A 2021 Oct 26; 118(43):e2104689118). Statistical basis was the difference between the experimental eye and the contralateral eye in the same subject. This efficacy experiment was replicated in two separate batches of animal subjects, and all experimental results were substantially consistent, leading to the same conclusions.

The experimental results showed that variation trends in refraction and eye axis parameters in animals of the vehicle negative control group met the expectations for the form-deprivation myopia model, confirming the successful modeling of the myopia model in this experiment, which was suitable for evaluating the efficacy of lipoic acid in myopia intervention. Compared to the vehicle control group, the myopia inhibition rates in the lipoic acid drug group at week 1 and week 2 of the treatment were 42.4% and 21.1%, respectively, and both showed statistic differences from the vehicle negative control group. This demonstrated that lipoic acid could significantly inhibit and retard progression of negative refraction in individuals with myopia, i.e., it could effectively delay and control onset and development of myopia (Table 1). Meanwhile, compared to the vehicle group, after administration of lipoic acid, axial elongation in individuals with myopia could be significantly inhibited and the increase in vitreous chamber depth in them could be retarded, with all these indicators showing statistic differences from the negative control group (see FIG. 1). Specific eye axis parameters (mean + SD) were as follows: At 1 week after administration, the mean of the interocular difference (form-deprivation myopia induced eye vs. contralateral eye) in axial length in the same individual was 0.04364 + 0.03295 mm for the lipoic acid treatment group versus 0.09909 + 0.01868 mm for the vehicle negative control group, respectively, and the corresponding means of the interocular differences in vitreous chamber depth were 0.0009091 + 0.027 mm vs. 0.1164 + 0.03075 mm. At 2 weeks after administration, the mean of the interocular difference in axial length was 0.06364 ± 0.03722 mm for the lipoic acid treatment group versus 0.1236 + 0.03042 mm for the vehicle negative control group, respectively, and the corresponding means of the interocular differences in vitreous chamber depth were 0.08545 + 0.02067 mm vs. 0.06182 ± 0.03157 mm (Table 2). Additionally, no any toxic reactions, ocular irritations, or significant ocular abnormalities were observed in the animals after the administration of lipoic acid. Related indicators such as anterior chamber depth, lens thickness, and corneal curvature in the subjects were also unaffected by the drug (see FIGS. 2A, B, and C). No mydriatic effect was observed in all the animals of the lipoic acid drug treatment group during the administration (FIG. 2D).

The above results indicated that lipoic acid could treat myopia and delay myopia progression by inhibiting the axial elongation and/or the increase in vitreous chamber depth in individuals with myopia or individuals with a tendency to develop myopia.

**Table 1. Effective Control of Progression of Negative Refraction in Myopia Model via Lipoic Acid**

| Difference in Refraction (Refraction, D) | | | |
|---|---|---|---|
| | | Mean | SD |
| Baseline | Vehicle | -0.3173 | 0.9536 |
| | Lipoic acid | -0.04545 | 0.9822 |
| 1 Week | Vehicle | -4.591 | 1.438 |
| | Lipoic acid | -2.2 | 1.441 |
| 2 Weeks | Vehicle | -6.3 | 1.276 |
| | Lipoic acid | -4.673 | 0.9253 |

**Table 2. Capability of Lipoic Acid to Effectively Inhibit Decline in Eye Axis Parameters of Myopia Model**

| | | Difference in Axial Length (AL, mm) | | Difference in Vitreous Chamber Depth (VCD, mm) | |
|---|---|---|---|---|---|
| | | Mean | SD | Mean | SD |
| Baseline | Vehicle | -0.0009091 | 0.02737 | 0.002727 | 0.01954 |
| | Lipoic acid | -0.004545 | 0.02296 | 0.09273 | 0.01421 |
| 1 Week | Vehicle | 0.09909 | 0.01868 | 0.1164 | 0.03075 |
| | Lipoic acid | 0.04364 | 0.03295 | 0.0009091 | 0.027 |
| 2 Weeks | Vehicle | 0.1236 | 0.03042 | 0.06182 | 0.03157 |
| | Lipoic acid | 0.06364 | 0.03722 | 0.08545 | 0.02067 |

### Example 3: Effectively Inhibiting Decrease in Choroidal Thickness in Myopic Eye of FDM Guinea Pig Model via Lipoic Acid

Choroidal thickness of all animal subjects in Example 2 were detected using Spectralis HRA + OCT (Heidelberg Engineering, Heidelberg, Germany) for 30 minutes to 60 minutes after the final administration (of either the vehicle or lipoic acid). Data collection and processing methods were the same as those previously published by the laboratory of the inventors (Pan M, Zhao F, Xie B, et al. Dietary omega-3 polyunsaturated fatty acids are protective for myopia. Proc Natl Acad Sci U S A 2021;118). Statistical basis was the difference between the experimental eye and the contralateral eye in the same subject.

In the vehicle negative control group, the choroidal thickness of the experimental eye was significantly reduced compared to the contralateral eye in the same individual. The results showed that the use of lipoic acid alone (administrated as eye drops) could significantly inhibit the decrease in choroidal thickness in myopic eyes. In some animals of the lipoic acid treatment group, the choroidal thickness of the experimental eye was even completely maintained at the level of the normal (contralateral) eye under pharmacological intervention. The experimental data indicated that the choroidal thickness of the experimental eye approached the choroidal thickness level of the normal eye in the same individual after administration of the drug (lipoic acid), resulting in a decreased interocular difference, and the statistical result showed a significant difference between the lipoic acid treatment group and the vehicle group. In conclusion, lipoic acid could significantly inhibit the decrease in choroidal thickness and retard the thinning trend of the choroid in individuals with myopia or individuals with a tendency to develop myopia. Meanwhile, these experimental results demonstrated that lipoic acid could treat and correct myopia by inhibiting the thinning of the choroidal in individuals with myopia or individuals with a tendency to develop myopia.

In individuals with myopia, parallel light rays passing through the refractive system of an eye with relaxed accommodation are focused in front of the retina. When lipoic acid increased the choroidal thickness in such individuals with myopia, it caused the retina to move towards the lens, ultimately leading to a reduction in distance or even a precise match between the imaging focus and the retina in the myopic eye. In individuals with myopia, the distance between the imaging focus and the retina was the degree of myopia. By reducing the distance between the imaging focus and the retina and inhibiting the increase of this distance in individuals with myopia, lipoic acid inherently reduced or controlled the degree of myopia. Administration of lipoic acid to individuals with myopia resulted in the treated eye achieving improved distance visual acuity and effective improvements of the myopic refractive state (i.e., corrected myopia, and reduced degree of myopia), including an enhancement of the distance visual acuity by lipoic acid. Therefore, the myopia intervention and treatment (prevention and control) effects demonstrated by lipoic acid in the present application were not limited to axial myopia, refractive myopia, pathological myopia, simple myopia, pseudomyopia, or true myopia, and were independent of factors such as age or gender of the subject, degree of myopia, rate of myopia progression, ethnicity, genetic background, or age of myopia onset. That is, lipoic acid exhibited the treatment and intervention effects on all types of myopia.

### Example 4: Comparison on the Effect of Treating Myopia among Lipoic Acid, Mexidol, Pyridoxol, Astaxanthin, and Blueberry Fruit Extract (Anthocyanin)

Utilizing the same efficacy evaluation system for myopia treatment in the guinea pig form-deprivation model as described in Example 2, the prevention and control effects of mexidol, pyridoxol, astaxanthin, and blueberry fruit extract on myopia were compared against lipoic acid. The administration route was periocular subconjunctival injection. The experimental groups were set as follows: 0.05% atropine sulfate group (positive control, Atropine), 0.37% emoxypine succinate group (ES), 0.3% pyridoxol hydrochloride group (PH), 0.87% astaxanthin group (ASTA), 6% blueberry fruit extract group (BFE), and 0.3% R-alpha lipoic acid group (R-LA), and 0.9% normal saline was set as a negative control group (Control).

The experimental results showed that the changes in refraction and eye axis parameters of animals in the negative control group met the expectations for this myopic animal model, and the positive control drug atropine exhibited the intended efficacy in the experiment. These results demonstrated that the myopia model in this experiment was successfully established and could be used for efficacy evaluation of test drugs. As could be seen from FIG. 4, during the administration period (at the week 1 detection), lipoic acid showed the optimal myopia treatment effect; at the end of the administration period, only atropine sulfate and R-alpha lipoic acid exhibited significant myopia treatment effects, while other test drugs showed no statistical differences from the vehicle group. Moreover, lipoic acid was significantly superior to other comparative drugs under the same experimental conditions, such as pyridoxol, astaxanthin, and blueberry fruit extract in inhibiting progression of negative refraction in individuals with myopia. Analyzed from the refraction indicator alone, no myopia treatment effect was observed after topical administration of the blueberry fruit extract to the eyes; instead, it showed a tendency to induce myopia or aggravate severity of myopia. The eye axis parameter results of each experimental group also supported the above conclusion (see FIG. 5), indicating that lipoic acid could effectively inhibit the axial elongation and the increase in vitreous chamber depth in individuals with myopia or individuals with a tendency to develop myopia. In terms of both efficacy evaluation indicators, i.e., the axial length and the vitreous chamber depth, the myopia treatment effect of lipoic acid was significantly superior to that of other comparative drugs at two detection time points after administration. Atropine could also effectively control myopia progression after being administrated, but obvious mydriasis occurred after administration (FIG. 6), and this adverse reaction limited clinical application thereof. In conclusion, the myopia treatment effect of lipoic acid was significantly superior to that of other comparative drugs. Therefore, compared with the prior art, lipoic acid was significantly superior to the prior art in treating myopia and inhibiting the axial elongation in individuals with myopia, which was unpredictable by those skilled in the art in advance.

### Example 5: Use of Pharmaceutical Composition Containing Lipoic Acid for Myopia Treatment

The same efficacy evaluation system for myopia treatment in the guinea pig form-deprivation model as described in Example 2 was utilized to evaluate the myopia treatment effects of blueberry fruit extract + lipoic acid compound formulations at different prescription ratios. The administration route was periocular subconjunctival injection. Three experimental groups were established specifically as follows: group of blueberry fruit extract (0.05%) + DL-alpha lipoic acid (1%) (0.05% BFE + 1% DL-ALA), group of blueberry fruit extract (1%) + DL-alpha lipoic acid (1%) (1% BFE + 1% DL-ALA), and a vehicle negative control group (Control) (the vehicle for all test compositions in this experiment consisted of 46 parts of PEG300, 5 parts of Tween 80, and 49 parts of 0.9% normal saline). DL-alpha lipoic acid (DL-ALA) was purchased from Suzhou Fushilai Pharmaceutical Co., Ltd.

The experimental results showed that the changes in refraction and eye axis parameters of animals in the negative control group met the expectations for this myopic animal model, demonstrating that the myopia model in this experiment was successfully established and could be used for the efficacy evaluation of test pharmaceutical compositions. As can be seen from FIG. 7, when the contents of lipoic acid and blueberry fruit extract in a pharmaceutical composition were equal (ratio of lipoic acid to blueberry fruit extract = 1:1), the pharmaceutical composition only exhibited a tendency to treat myopia. However, there were no statistical differences in either the refraction indicator or the axial length indicator compared with the vehicle control group. Nevertheless, while the concentration of lipoic acid was kept unchanged, after the proportion of the blueberry fruit extract in the compound formulation was reduced (i.e., lipoic acid was used as the main active ingredient or direct active ingredient of the pharmaceutical composition (ratio of lipoic acid to blueberry fruit extract = 20:1), the pharmaceutical composition significantly inhibited the progression of negative refraction and the axial elongation in the myopia model. Compared with the case where the ratio of the two ingredients in the pharmaceutical composition was 1:1, when lipoic acid was used as the main active ingredient or direct active ingredient of the pharmaceutical composition (ratio of lipoic acid to blueberry fruit extract > 1:1), the myopia treatment effect of this formulation was significantly enhanced, and there were statistical differences in the efficacy evaluation indicators compared with the vehicle control group.

The foregoing results indicated that the pharmaceutical composition with lipoic acid as the main active ingredient exhibited better myopia prevention and control effects than the pharmaceutical composition with lipoic acid as a non-main active ingredient. This was also an unexpected effect: by reducing the contents of other main active ingredients in the pharmaceutical composition such that lipoic acid served as the main active ingredient of the formulation, the prevention and treatment effects on myopia were not reduced, but significantly improved instead. Previously, it was not known that lipoic acid could be used to treat myopia and produce such a significant therapeutic effect. Therefore, there was no motivation to increase the content of lipoic acid in the pharmaceutical composition to exceed that of other active ingredients, and thus there was no attempt or consideration to improve myopia treating pharmaceutical compositions or enhance the effect of combined drug therapy for myopia by this method.

### Example 6: Administration of Lipoic Acid Choline Ester Eye Drops (in Chloride Form) for Delaying Myopia Progression in Young Individuals

The same efficacy evaluation system for myopia treatment in the guinea pig form-deprivation model as described in Example 2 was utilized to systematically study influence of R-alpha lipoic acid choline ester eye drops (in the chloride form, LACE) at different concentrations on myopia prevention and control effects. Animal subjects in all groups were administered by direct eye drop instillation, twice a day (once antemeridiem and once post meridiem), with an amount of 25 µL administrated each time. The experimental groups included two LACE test concentrations of 0.5% and 0.15%. Meanwhile, a 0.05% atropine sulfate group (positive control, Atropine) and a 0.9% normal saline vehicle group (negative control, Control) were set as controls.

The experimental results showed that the changes in refraction and eye axis parameters of animals in the negative control group met the expectations for this myopic animal model, and the positive control drug atropine exhibited the intended efficacy in the experiment. These results demonstrated that the myopia model in this experiment was successfully established and could be used for the efficacy evaluation of test drugs. As can be seen from FIG. 8-1, lipoic acid choline ester showed a positively related dose-effect relationship in the treatment of the form-deprivation myopia model: with increase in the administrated dose, it may be seen more satisfactory myopia treatment effect, and stronger inhibitory effect on the axial elongation in individuals with myopia. Compared with the vehicle control group, 0.5% lipoic acid choline ester could significantly inhibit progression of negative refraction in form-deprivation myopia and significantly inhibit the axial elongation in form-deprivation myopia throughout the administration period. Meanwhile, under the condition of administration by eye drop instillation, 0.5% lipoic acid choline ester, as the prodrug of lipoic acid, also showed better comprehensive performance in the treatment of myopia in young individuals than the positive control atropine, especially in terms of inhibiting progression of negative refraction and the axial elongation of myopic eyes after long-term administration. Throughout the administration period, the inventors did not observe drug-induced mydriasis in animals in the lipoic acid choline ester group, and there were no statistical differences in anterior chamber depth, lens thickness, and corneal curvature from the negative control group (FIG. 8-2).

Non-invasive administration of lipoic acid choline ester could effectively treat myopia and significantly inhibit the axial elongation in individuals with myopia or individuals with a tendency to develop myopia, with the efficacy enhanced as the concentration increased. It is well-known that excessive axial length in individuals with myopia is a direct cause of various fundus lesions (such as complications of high myopia) and even complete loss of visual acuity in this population. Therefore, the medical focus of myopia treatment is also to avoid excessive axial length of myopic eyes. The strong biological effect of lipoic acid choline ester in inhibiting axial elongation, as demonstrated in the pharmacological treatment of myopia, proved the superiority and uniqueness of lipoic acid choline ester as a drug for effectively treating myopia in children and adolescents, simple myopia, primary myopia, progressive myopia, and axial myopia. It also suggested that individuals with myopia or individuals with a tendency to develop myopia should use lipoic acid or lipoic acid choline ester as early as possible to prevent and control onset and development of myopia, so as to avoid irreversible damage to the visual acuity of the patients. In addition, lipoic acid choline ester was once formulated into eye drops at a concentration of 1.5% as an active ingredient for the treatment of presbyopia, and clinical studies showed that it had good bioavailability and high safety. During the administration to young individuals in this experiment, no ocular abnormalities or mydriasis were observed in all the animal subjects in the lipoic acid choline ester group. This result further demonstrated that lipoic acid or choline ester-type prodrugs thereof were safe and reliable for children or adolescents. Meanwhile, this study explored a minimum effective dose of lipoic acid choline ester eye drops administrated to treat myopia, which could provide necessary reference data for subsequent development of formulation prescriptions and clinical application in young individuals.

In conclusion, as evaluated from the perspectives of efficacy and safety, the risk-benefit ratio of lipoic acid or lipoic acid choline ester administrated to treat individuals with myopia was superior to that of atropine sulfate (for example, when lipoic acid or lipoic acid choline ester was used during the daytime, it did not cause photophobia associated with mydriasis (a side effect observed with atropine) while treating myopia. The lipoic acid or lipoic acid choline ester was particularly suitable for delaying onset and development of myopia in children and adolescents, as well as for prevention and control of myopia in school-age individuals.

### Example 7: Significantly Inhibiting and Retarding Myopia Progression in Negative Lens-Induced Myopia (LIM) Model in Guinea Pig via Optical Isomers of Lipoic Acid and Metabolites Thereof

After individuals with significant ocular diseases or abnormalities were eliminated from healthy 3-week-old tricolor guinea pigs (both males and females), by detecting refraction (using an infrared eccentric photorefraction device) and the eye axis (using A-scan ultrasonography), animals with refractive power between 3 diopters and 8.5 diopters (D), an interocular difference in refraction not exceeding 2 D, and both interocular differences in vitreous chamber depth and axial length not exceeding 0.05 mm were selected. These animals were randomly divided into the following 5 groups.
1. Lens induction + vehicle control group (Control): monocular wear of a -4 D lens, with 100 µL of vehicle (DMSO:PEG300:Tween80:0.9% normal saline = 1:45:5:49) administrated to the lens-wearing eye daily for 1 week, and the number of samples = 11;
2. Lens induction + R-alpha lipoic acid experimental group (R.ALA): monocular wear of a -4 D lens, with 100 µL of 0.4% R-alpha lipoic acid administrated to the lens-wearing eye daily for 1 week, and the number of samples = 15;
3. Lens induction + S-alpha lipoic acid experimental group (S.ALA): monocular wear of a -4 D lens, with 100 µL of 0.4% S-alpha lipoic acid administrated to the lens-wearing eye daily for 1 week, and the number of samples = 13;
4. Lens induction + dihydrolipoic acid experimental group (DHLA): monocular wear of a -4 D lens, with 100 µL of 0.4% dihydrolipoic acid administrated to the lens-wearing eye daily for 1 week, and the number of samples = 14;
5. Lens induction + positive control group (Atropine): monocular wear of a -4 D lens, with 100 µL of 0.05% atropine sulfate administrated to the lens-wearing eye daily for 1 week, and the number of samples = 13.

Among them, the negative control group (vehicle control group) differed from other experimental groups in that it contained no active ingredient. Beginning on the day of modeling, the corresponding vehicle or drug was administrated to the experimental eye (lens-wearing eye) daily between 9:00 AM and 10:00 AM via periocular subconjunctival injection at an injection volume of 100 µL, once daily for 1 week. All procedures for negative lens-induced myopia modeling, peribulbar injection administration, detection of parameters such as refraction, and data processing for all the subjects were consistent with the schemes previously used by the research group (CN114796205B). At the beginning, on day 3, and at the end of the LIM model efficacy experiment, the refraction was detected in animal subjects. The statistical basis was the difference between the experimental eye (lens-wearing eye) and the contralateral eye of the same subject.

The experimental results showed that the changes in refraction of animals in the negative control group met the expectations for the LIM myopia model, and the positive control drug atropine exhibited the intended efficacy in the experiment. These results demonstrated that the myopia model in this experiment was successfully established and could be used for the efficacy evaluation of test drugs. As can be seen from FIG. 9, all test compounds showed significant myopia treatment effects throughout the entire administration period. That is, they all exerted a delaying effect on the decrease in refraction of individuals with myopia, except that mydriasis occurred in the atropine group. After the end of the administration period, the refraction indicators of the R-alpha lipoic acid group and the dihydrolipoic acid group showed extremely significant differences compared with the vehicle group. On day 3 of administration, the efficacy of the R-alpha lipoic acid group was substantially the same as that of the S-alpha lipoic acid group, and both were superior to that of the dihydrolipoic acid group, and their average values were even better than that of the positive control atropine group. Meanwhile, it could also be clearly seen from FIG. 9 that R-alpha lipoic acid could exert a high inhibitory effect in the early stage of myopia onset and maintain this inhibitory effect consistently. That is, the excellent myopia prevention and control efficacy exhibited by lipoic acid was independent of the severity of myopia or the rate of progression of negative refraction. During the administration process, neither the optical isomers of lipoic acid nor dihydrolipoic acid caused ocular irritations, and they did not affect the anterior chamber depth and lens thickness (FIG. 10).

It was generally believed that the main biological activity of lipoic acid was exhibited by the R type; meanwhile, it was known that dihydrolipoic acid had a stronger reducing capacity than lipoic acid. However, our experimental results demonstrated that both chiral forms of lipoic acid could effectively treat myopia, and their therapeutic efficacies for the onset of myopia were basically consistent. In general, the efficacy of dihydrolipoic acid was inferior to that of R-alpha lipoic acid, indicating that the treatment of myopia with lipoic acid or metabolites thereof relied on a novel and previously unknown mechanism of action.

### Example 8: Use of Formulations Containing Lipoic Acid for Myopia Treatment

The same efficacy evaluation system for myopia treatment in the guinea pig form-deprivation model as described in Example 2 was utilized to evaluate the therapeutic effect of a compound formulation of low-concentration atropine and lipoic acid on myopia. The administration route was periocular subconjunctival injection, and three experimental groups were set specifically as follows: 0.05% atropine sulfate group (Atropine, positive control), 0.01% atropine sulfate + 0.15% R-alpha lipoic acid group (0.01% Atropine + 0.15% R.ALA), and vehicle negative control group (Control, 0.9% normal saline). The preparation method of the above compound formulation was as follows: An R-alpha lipoic acid mother solution (dissolved in DMSO) at a concentration of 15% and an atropine sulfate mother solution (dissolved in normal saline) at a concentration of 0.2% were added to a mixed solvent in the following ratio: 15% R-alpha lipoic acid mother solution : 0.2% atropine sulfate mother solution : PEG300 : Tween80 : 0.9% normal saline = 1:5:45:5:44, resulting in a final concentration of the formulation of 0.01% atropine sulfate + 0.15% R-alpha lipoic acid.

The experimental results showed that the changes in refraction and eye axis parameters of animals in the negative control group met the expectations for this myopic animal model, demonstrating that the myopia model in this experiment was successfully established and could be used for the efficacy evaluation of test pharmaceutical compositions. As can be seen from FIG. 11 (A), when the animal subjects were administrated a relatively low concentration of lipoic acid (0.15%) at the same time, the efficacy of 0.01% atropine during the myopia treatment process was comparable to that of 0.05% atropine (both groups showed significant differences in refraction compared with the vehicle group, and the 0.01% Atropine + 0.15% R.ALA group showed extremely significant statistical differences). Even at the end of administration, the refraction indicator of the compound formulation was superior to that of the 0.05% atropine sulfate group. The myopia treatment efficacy benefited from the effective inhibition of axial elongation in individuals with myopia by the compound formulation. As can be clearly observed from FIG. 11 (B), the axial length of myopic eyes of the animal subjects was significantly inhibited by the compound formulation of atropine and lipoic acid throughout the entire administration period.

Atropine sulfate is limited by its adverse reactions such as mydriasis, photophobia, and post-withdrawal "rebound". Thus, only low concentrations (such as 0.01% and 0.02%) can be used in clinical myopia treatment. However, due to the small dosage, the drug response rate at such concentrations is low, and the results of myopia prevention and control are mostly not very satisfactory. It is a goal pursued by those skilled in the art to effectively improve the therapeutic effect of myopia prevention and control drugs such as atropine, while significantly avoiding the occurrence of their adverse reactions, thereby generally reducing the medication risk for young or juvenile individuals. The combination of 0.01% Atropine + 0.15% R.ALA (atropine + lipoic acid) can not only reduce the adverse reactions of atropine but also enhance its efficacy in treating myopia. The prior art has never suggested or implied the existence of such a combined medication effect. We unexpectedly found that lipoic acid may improve the myopia therapeutic efficacy of M-receptor inhibitors such as atropine by means of a synergistic effect of drugs. It is speculated that this may be due to different intervention targets thereof in myopia treatment, altered pharmacokinetic properties of active ingredients in the eyes, or other unknown factors. In addition, with extremely complex pathogenesis of myopia, the above phenomena require further in-depth research. Nevertheless, the potential synergistic effect of lipoic acid when combined with other myopia treatment drugs in the process of myopia prevention and control is worthy of focused attention and utilization in the development of drugs for myopia.

In conclusion, based on the myopia prevention and control effect of lipoic acid when co-administrated with atropine in this experiment, and the fact that lipoic acid can effectively treat myopia through a novel and unknown mechanism, we believe that lipoic acid can also be combined with other myopia treatment drugs or prepared into pharmaceutical compositions for treatment and prevention and control of myopia, especially for children or adolescent individuals. In the above-mentioned pharmaceutical compositions, the concentration, proportion, or efficacy contribution rate of lipoic acid may be higher than, lower than, or consistent with those of other active ingredients.

### Example 9: Effective Increase in Choroidal Thickness in Myopic Eyes via Administration of Lipoic Acid Choline Ester Eye Drops

After completion of the experiment, the choroidal thickness was detected in animals in the two control groups and the 0.5% lipoic acid choline ester eye drop administration group in Example 6 (some animals died during the measurement due to reasons unrelated to the test drugs, so the corresponding samples were eliminated). For the detection and analysis methods, reference was made to the previously published literature by the research group (Sen Zhang, Invest Ophthalmol Vis Sci. 2019 Jul 1; 60(8):3074-3083).

As shown in FIG. 12, compared with the negative control group, lipoic acid choline ester could effectively inhibit thinning of the choroid in the form-deprivation model eyes, and this therapeutic effect was superior to that of the positive control atropine group. Therefore, lipoic acid choline ester had significant inhibitory and reversing effects on the choroidal thinning trend in individuals with myopia. In conclusion, the results of the experiment indicated that lipoic acid choline ester could treat and correct myopia by inhibiting the decrease in choroidal thickness in individuals with myopia or individuals with a tendency to develop myopia.

### Example 10: Significantly Inhibiting Choroidal Thinning in Individuals with Myopia via Optical Isomers of Lipoic Acid and Dihydrolipoic Acid

Using the animal subjects in Example 7, choroidal parameters were detected after completion of the experiment (a few animals died during the measurement due to reasons unrelated to the test drugs, so these samples were eliminated). Specifically, choroidal thickness was measured in the LIM myopia model animals after completion of the 7-day administration experiment. Commercially available OCTA (Spectralis HRA + OCT, OCTA, Heidelberg, Germany) modified to be more suitable for animal eyeballs was used to detect the choroidal thickness. Before detection, the guinea pigs were fully anesthetized. After obtaining the OCTA images of the guinea pigs, the measurement results were quantified using a self-developed program (MatLab R2017a, MathWorks) to realize real-time quantitative detection of choroidal thickness in the guinea pigs (Sen Zhang, Invest Ophthalmol Vis Sci. 2019 Jul 1; 60(8):3074-3083).

The results are shown in FIG. 13. Compared with the vehicle control group, both the optical isomers of lipoic acid and dihydrolipoic acid could effectively inhibit the decrease in choroidal thickness in individuals with myopia, as did the positive control atropine (after pharmacological intervention, the choroidal thickness of the experimental eye approached the choroidal thickness level of the normal eye in the same individual, i.e., the difference between the two decreased). It should be noted that both the optical isomers of lipoic acid and dihydrolipoic acid had better inhibitory efficiency on choroidal thinning in individuals with myopia than the positive control atropine. In particular, R-alpha lipoic acid could more significantly increase the choroidal thickness of myopic eyes.

In both the form-deprivation model and the negative lens-induced myopia model, the present application reports for the first time that lipoic acid, lipoic acid choline ester, and dihydrolipoic acid can all increase the choroidal thickness of myopic eyes. In individuals with myopia, parallel light rays passing through the refractive system of an eye with relaxed accommodation are focused in front of the retina. When lipoic acid or structural analogs thereof increased the choroidal thickness in such individuals with myopia, the retina moved toward the lens, ultimately leading to a reduction in distance between the imaging focus and the retina in the myopic eye. The distance between the imaging focus and the retina corresponds to the degree of myopia. Lipoic acid or structural analogs thereof could shorten this distance or inhibit the increase in this distance in individuals with myopia, thus inherently reducing the degree of myopia and treating myopia. Administration of lipoic acid or structural analogs thereof to individuals with myopia resulted in the treated eye achieving improved distance visual acuity and effective reduction in the myopic refractive state, including an enhancement of the distance visual acuity. Therefore, the myopia treatment and intervention effects demonstrated by lipoic acid or structural analogs thereof in the present application were not limited to axial myopia, refractive myopia, pathological myopia, simple myopia, pseudomyopia, or true myopia, and were independent of factors such as age or gender of the subject, degree of myopia, rate of myopia progression, ethnicity, age of myopia onset, and cause of myopia. That is, the optical isomers of lipoic acid, or prodrugs thereof (for example, lipoic acid choline ester), or metabolites thereof (for example, dihydrolipoic acid), exhibited treatment and intervention effects on all types of myopia.

Considering the long-term and continuous nature of myopia drug therapy, coupled with the fact that the majority of the subjects were individuals in the growth and development stage, lipoic acid and lipoic acid choline ester demonstrated superiority over other compounds in terms of both efficacy and safety. Our results demonstrated that: lipoic acid and any of the corresponding compounds thereof in salt or ester forms (for example, lipoic acid choline ester) could effectively treat myopia and control the progression of negative refraction in individuals with myopia or individuals with a tendency to develop myopia by inhibiting axial elongation and retarding the increase in vitreous chamber depth. Meanwhile, both lipoic acid and its pharmaceutically acceptable salts or esters (for example, lipoic acid choline ester) could significantly increase the choroidal thickness and reduce the degree of myopia in individuals with myopia. The dosage forms of the aforementioned lipoic acid and the pharmaceutically acceptable salts or esters thereof (for example, lipoic acid choline ester) for use in myopia treatment, intervention, correction, and prevention/control may be eye drops, or eye ointments, ocular sprays, ocular injections, or ocular gels, and even as oral dosage forms. The present application demonstrated that devices, formulations, or pharmaceutical compositions containing such compounds (drugs) can be used to control myopia progression.

## Claims

1. Use of lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing, wherein the use is one or two or more of:
(1) preventing and/or treating myopia and myopia-related symptoms, and/or correcting myopia; or
(2) controlling onset and development of myopia with orthokeratology lenses or another myopia treatment drug; or
(3) performing intervention treatment on myopic eyes or delaying myopia progression; or
(4) inhibiting and retarding axial elongation and/or an increase in vitreous chamber depth in an individual with myopia or an individual with a tendency to develop myopia, and increasing a choroidal thickness in an individual with myopia or an individual with a tendency to develop myopia; or
(5) preventing, retarding, reducing or treating abnormal eye development related to a refractive error in a child and/or an adolescent; or
(6) enabling an individual to obtain clearer distance visual acuity without wearing lenses or relying on visual acuity correction means than distance visual acuity before using the lenses or the means and/or than distance visual acuity when not using the lenses or the means; or
(7) inhibiting, delaying or retarding progress or rate of negative refraction in an individual with myopia or an individual with a tendency to develop myopia; or
(8) preparing a drug, a formulation, a composition, or a device for achieving at least one of the uses of (1) to (7).

2. The use according to claim 1, wherein an administration route of systemic administration such as oral administration, and/or topical administration (eye drop instillation, intraocular injection, intraocular implantation, periocular application of a skin ointment/emulsion, or application of an eye ointment), and/or parenteral administration (for example, mucosal administration, transdermal administration, microneedle administration), and/or non-invasive administration (for example, administration using an ophthalmic spray) is adopted.

3. The use according to claim 1 or 2, wherein the drug, the formulation, or the composition is an injection, a tablet, a lyophilized powder for injection, a capsule, an effervescent tablet, a chewable tablet, a buccal tablet, a granule, an ointment, a syrup, an oral liquid, an aerosol, nasal drops, an externally applied formulation, an oral formulation, or the like; preferably an ophthalmic dosage form, including but not limited to eye drops (eye water), an eye ointment, an ophthalmic spray, an implant tablet, an ophthalmic gel, an eye patch, an ophthalmic microsphere, an ophthalmic sustained-release formulation, a periocular injection, and an intraocular injection; and alternatively, a regular solution, an aqueous solution, an unsaturated solution, an oil-water mixture, a suspension, a liniment, a lotion, a cream, drops, a granule, a spray, an ointment, a patch, a paste, a pill, a suppository, an emulsion, or a group containing cellulose (for example, methylcellulose), a polyol, cyclodextrin (for example, hydroxypropyl-beta-cyclodextrin), a reducing agent (for example, glutathione, N-acetylcarnosine), glycerol, liquid paraffin, petrolatum, propylene glycol, ethyl pyruvate, semifluorinated alkane, an amino acid or a derivative thereof (for example, alanine, methionine, cysteine, and histidine), a sugar or a metabolite thereof (for example, glucose-6-phosphate), an oxygen scavenger (for example, Oxy-Guard^{™} or StabilOx^{™}), a vitamin (for example, vitamin B1, vitamin B2, vitamin C, or vitamin E), NADPH, a dendrimer, a nanomaterial, a sustained-release material, a liposome, or any combination thereof.

4. The use according to any one of the preceding claims, wherein the individual with myopia or the individual with a tendency to develop myopia is an individual medically diagnosed with myopia; or a child and/or adolescent, preferably aged 2 to 30, and more preferably aged 6 to 18; or a minor, preferably an individual whose eyes are still in a stage of growth and development; or a school-age individual, preferably a student in grades 1 to 12; or an individual with one or both parents having high myopia; or an individual with insufficient hyperopia reserve.

5. The use according to any one of the preceding claims, wherein the myopia is: refractive myopia or axial myopia; congenital myopia (myopia present at birth or before school age), early-onset myopia (onset before 14 years old), delayed-onset myopia (onset at 16-18 years old), late-onset myopia (onset after adulthood); low myopia (mild myopia), moderate myopia, high myopia (severe myopia), malignant myopia; pseudomyopia, true myopia; myopia in children and/or adolescents (preferably aged 2-30, and more preferably aged 6-18), myopia in minors, myopia in adults, myopia in the elderly; simple myopia, pathological myopia, complex myopia; axial simple myopia, simple axial myopia; axial myopia in children and/or adolescents (preferably aged 2-30, and more preferably aged 6-18); axial myopia in school-age and preschool individuals; primary myopia, secondary myopia; primary myopia in children and/or adolescents (preferably aged 2-30, and more preferably aged 6-18); or progressive myopia in children and/or adolescents (preferably aged 2-30, and more preferably aged 6-18); curvature myopia, index myopia, astigmatic myopia, positional myopia, curved myopia; axial myopia with continuously increased negative refraction; myopia caused by prolonged near work, myopia and pseudomyopia induced by eye strain, negative refraction caused by adverse drug reactions, myopic eyes, myopia caused by reading, myopia caused by using electronic products such as mobile phones, myopia caused by mismatched refractive media (components), myopia caused by abnormal refractive development, myopia caused by excessive eyeball growth, myopia caused by unhygienic eye use, myopia **characterized by** imaging focuses of distant objects falling in front of the retina due to various reasons, myopia demonstrating poor or no response to atropine treatment, non-complex myopia, myopia caused by insufficient outdoor activities, accommodative spastic myopia, myopia in children, myopia in infants, hereditary myopia, myopia dominated by environmental factors, mixed myopia, traumatic myopia, toxic myopia, drug-induced myopia, diabetic myopia, instrument-induced myopia, empty field myopia, night myopia, myopia in premature infants, diving-induced myopia, hysterical myopia, and transient myopia occurring during menstruation, pregnancy, and various eye diseases and systemic diseases.

6. The use according to any one of the preceding claims, wherein the myopia-related symptoms refer to complications caused by myopia or excessive axial length, for example, complications of high myopia, such as spotted vision, glaucoma, posterior staphyloma, retinal detachment, retinal tear, retinopathy, amblyopia, macular hemorrhage, choroidal neovascularization, choroidal atrophy, macular degeneration or maculopathy, visual field defect, progressive or sudden diminution of visual acuity (especially near visual acuity), sore and swelling eyes and/or eye pain, nyctalopia, astigmatism, loss of sight, vitreous liquefaction, vitreous opacity, strabismus, frequent blinking, frequent eye rubbing, anisometropia, blurred vision for distant objects, necessitating squinting or partially closing the eyelids to see distant objects clearly, headache caused by eye strain, inattention caused by myopia, difficulty in vision while driving (especially at night, i.e., night myopia), retinal atrophy and degeneration (hemorrhage and hiatus), subretinal neovascularization, or eyeball atrophy.

7. The use according to any one of the preceding claims, wherein the drug, formulation, composition, or device further comprises another drug, compound, or ophthalmic formulation, including but not limited to a myopia treatment drug (such as pirenzepine, muscarinic antagonist, robaxin, indoramine, timolol maleate, adrenaline, pyrazine, perlapine, methylamine, chlorisondamine, acetylcholinesterase inhibitor, dopamine agonist, γ-aminobutyric acid, naloxone, glucagon, homatropine methylbromide, and retinoic acid), an M-receptor blocker (for example, blockers, antagonists, or inhibitors targeting M2 or M3 receptors), atropine or atropine sulfate, dibazol, a polyunsaturated fatty acid (such as DHA and EPA), homatropine, anisodamine (racemic), scopolamine, tropicamide, 7-methylxanthine, nicotinic acid, piracetam, Salvia miltiorrhiza extract, Carthamus tinctorius extract, bear bile extract, fish oil, adenosine triphosphate (ATP), smooth muscle relaxants, a drug for preventing vasospasm, a non-selective adenylate antagonist, a vasodilator, a mydriatic component, a decongestant component, an accommodative component for eye muscle (for example, ciliary muscle), an anti-inflammatory (antiphlogistic) component, an astringent component, an antihistaminic component, an anti-allergic component, a component for inhibiting collagen degradation, a liver-protecting component (to avoid or reduce hepatotoxicity), a component for enhancing blood-retinal barrier (to make it more difficult for a compound permeating through the physiological barrier), an amino acid, an antibacterial component, an antioxidant component (such as vitamin C, tea polyphenol, and glutathione), a sugar, a polymer or a derivative thereof, cellulose or a derivative thereof, a local anesthetic component, an amblyopia treatment component, a glaucoma treatment component, miRNAs and a modification thereof, a therapeutic component for ophthalmic diseases, an excipient, and the like.

8. The use according to any one of the preceding claims, wherein the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing, and one or more drugs for delaying myopia progression and/or myopia treatment are formulated or designed for continuous administration, or simultaneous administration, or sequential administration, or alternating administration, or administration at intervals, or separate administration.

9. The use according to any one of the preceding claims, wherein the related compound comprises but is not limited to: all compounds listed in CN115279745A and CN111315369A, and a salt further formed from lipoic acid or a lipoic acid ester, preferably a salt form of lipoic acid choline ester, including but not limited to lipoic acid choline ester tosylate, lipoic acid choline ester besylate, lipoic acid choline ester chloride, or lipoic acid choline ester iodide; or conjugates of the foregoing are compounds of FIG. 3, or 6-(benzylthio)-8-[(hydroxyphenylmethyl)thio]octanoic acid, lipoamide (1,2-dithiolane-3-pentanamide), 8-(ethyldithio)-6-(phenyldithio)octanoic acid, lipoyl chloride, 5-(1,2-10 dithiolan-3-yl)pentanoic acid, 6,8-dimercaptooctanoic acid (dihydrolipoic acid), dihydrolipoates, 5-(1,2-dithiolan-3-yl)pentanoic acid, 5-(1,2-thiaselenolani-5-yl)pentanoic acid, 5-(1,2-thiaselenolan-3-yl)pentanoic acid, and 6,8-dimercaptooctanoic acid.

10. The use according to any one of the preceding claims, wherein the formulation is an oral product such as a health product, food, a dietary supplement, a nutraceutical, or a beverage, or a cosmetic.

11. The use according to any one of the preceding claims, wherein the device is an instrument, an apparatus, a consumable, a system, a medical instrument, a health product or a product for changing eye appearance that is capable of releasing a drug or has a drug delivery function or has a potential drug delivery capability, such as corneal contact lenses, eyeglasses, intraocular lenses, a suture, an orthokeratology (OK) lens cleaning (maintenance) system, an eye patch, a visual acuity-improving patch, cosmetic contact lenses, a microneedle, an ophthalmic spray system, an eye massager, an eye fumigator, an ocular surface drug delivery device, an intraocular drug delivery device, a fundus drug delivery device, an implantable pump, a wearable device, an acupoint massager, an eye relaxation device, a myopia treatment instrument, or a drug-device combination for myopia prevention and control.

12. The use according to any one of the preceding claims, wherein the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing serves as a sole active ingredient or a main active ingredient or a direct active ingredient;
and/or a content or efficacy of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing accounts for lower than 1%, or more than 1%, more than 10%, more than 20%, more than 30%, more than 40%, more than 50%, more than 60%, more than 70%, more than 80%, more than 90%, or 100% of the content or efficacy of all active ingredients for the use;
and/or a content, concentration, or proportion of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing in the drug, formulation, composition, or device is at least 0.001%, or at least 0.05%, or at least 0.15%.
and/or the efficacy of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing accounts for more than 20%, preferably more than 50%, of the efficacy of all starting material ingredients or all active ingredients for the use;
wherein the percentage (%) is a mass ratio, or a molar ratio, or a mass-volume ratio, or an efficacy contribution rate to the use.

13. The use according to any one of the preceding claims, wherein the concentration of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing in the drug, formulation, composition, or device is 0.001 µM to 100 M, preferably 0.005 µM to 50 M, preferably 1 µM to 1 M, preferably 300 µM to 300 mM, more preferably 1 mM to 200 mM, and more preferably 10 mM to 100 mM (for example, about 46 mM, namely 0.94%); and/or the concentration or proportion of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing in the drug, formulation, composition, or device is higher than 0.0005%, preferably higher than 0.025%, preferably higher than 0.05%, more preferably higher than 0.1%, and more preferably higher than 1%, wherein the percentage (%) is a mass/volume concentration (g/100 ml) or a mass percentage or a molar (mole number) ratio.

14. A topically administrated drug, formulation, composition, or device for treating myopia or controlling myopia progression, wherein a concentration of lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing in the drug, formulation, composition, or device is 0.001 µM to 100 M, preferably 0.005 µM to 50 M, preferably 1 µM to 1 M, preferably 300 µM to 300 mM, more preferably 1 mM to 200 mM, and more preferably 10 mM to 100 mM;
and/or the concentration or proportion of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing in the drug, formulation, composition, or device is not lower than 0.0001%, preferably not less than 0.001%, preferably not lower than 0.01%, more preferably not lower than 0.1%, and more preferably not lower than 0.8%, wherein the percentage (%) is a mass/volume concentration (g/100 ml) or a mass percentage or a molar (mole number) ratio.

15. A pharmaceutical composition or compound formulation containing at least two active ingredients, comprising lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing, and other active ingredients for treating myopia.

16. The pharmaceutical composition or compound formulation according to claim 15, wherein an addition amount, concentration, and/or efficacy of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing in the pharmaceutical composition or compound formulation is not lower than an addition amount, concentration, and/or efficacy of any of the other active ingredients for treating myopia, wherein the efficacy is directed to treating myopia and/or controlling myopia progression.

17. The pharmaceutical composition or compound formulation according to claim 15 or 16, wherein the compound formulation comprises but is not limited to eye drops, an eye ointment, an ophthalmic spray, an implant tablet, an ophthalmic gel, an eye patch, an ophthalmic microsphere, an ophthalmic sustained-release formulation, a periocular injection, or an intraocular injection.

18. An ocular formulation, wherein lipoic acid or lipoic acid choline ester, or an optical isomer or racemate thereof, or a solvate thereof, or a pharmaceutically acceptable salt or ester thereof, or a prodrug thereof, or a metabolite thereof, or a related compound or extract thereof, or a crystalline compound thereof, or a combination of the foregoing serves as a direct, sole, or main active ingredient for myopia treatment in the ophthalmic formulation, wherein a concentration of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing is 0.001 µM to 100 M, preferably 0.005 µM to 50 M, preferably 1 µM to 1 M, preferably 300 µM to 300 mM, more preferably 1 mM to 200 mM, and more preferably 10 mM to 100 mM; and/or the concentration or proportion of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing is not lower than 0.0001%, preferably not lower than 0.001%, preferably not lower than 0.01%, more preferably not lower than 0.1%, and more preferably not lower than 0.8%, wherein the percentage (%) is a mass/volume concentration (g/100 ml) or a mass percentage or a molar (mole number) ratio.

19. The ophthalmic formulation according to claim 18, comprising but not limited to eye drops, an eye ointment, an ophthalmic spray, an implant tablet, an ophthalmic gel, an eye patch, an ophthalmic microsphere, an ophthalmic sustained-release formulation, a periocular injection, or an intraocular injection.

20. According to any one of the preceding claims, wherein a content of the lipoic acid or lipoic acid choline ester, or the optical isomer or racemate thereof, or the solvate thereof, or the pharmaceutically acceptable salt or ester thereof, or the prodrug thereof, or the metabolite thereof, or the related compound or extract thereof, or the crystalline compound thereof, or the combination of the foregoing is a therapeutically effective amount.
